# EUROPEAN PATENT APPLICATION

(11) **EP 4 755 878 A1**
(43) Date of publication of application: **10.06.2026**
(21) Application number: 24849020.3
(22) Date of filing: 24.07.2024
(51) Int. Cl.: C07D 311/94, C07C 217/94, C07D 413/08, C07D 417/08, C07D 497/04, C09K 9/02, G02C 7/10

(54) **PHOTOCHROMIC COMPOUND, PHENOL DERIVATIVE, CURABLE COMPOSITION, OPTICAL ARTICLE, LENS, AND EYEGLASS**

(30) Priority: 02.08.2023 JP 2023126493
(71) Applicant: TOKUYAMA CORPORATION, Yamaguchi 745-8648 (JP)
(72) Inventor: MIYAZAKI, Masayuki, Shunan-shi, Yamaguchi 745-8648 (JP)
(74) Representative: Kraus & Lederer PartGmbB
(86) International application number: PCT/JP2024/026478
(87) International publication number: WO 2025/028365

(57) **Abstract**

The purpose of the present disclosure is to provide: a photochromic compound having excellent temperature dependency and an excellent color fading rate; a phenol derivative that can be an intermediate for the photochromic compound; and a curable composition, an optical article, a lens, and an eyeglass, each containing the photochromic compound. An embodiment of the present invention provides a photochromic compound having a backbone represented by formula (1). In formula (1), M represents C, Si, or Ge. Ring A is a substituted or unsubstituted aromatic ring, a substituted or unsubstituted aromatic heterocyclic ring, or a substituted or unsubstituted condensed polycyclic ring obtained through fusing, to either of said rings, an aromatic ring or an aromatic heterocyclic ring. Z1 represents a group represented by formula (1a) or a group represented by formula (1b).

## Description

### TECHNICAL FIELD

The present invention relates to a photochromic compound, a phenol derivative, a curable composition, an optical article, a lens, and eyeglasses.

### BACKGROUND ART

Photochromic compounds are compounds that can reversibly take two isomeric forms with different absorption spectra upon irradiation with light including ultraviolet light such as sunlight or light from a mercury vapor lamp. In general, compounds in a colorless or decolored state quickly change in color and are isomerized to a colored or color-developed state (chromogenic reaction) by irradiation with ultraviolet light. The photochromic compounds have been studied and developed as materials for photochromic lenses.

In applications of such photochromic lenses, the photochromic compounds may require the following properties:
(I) a degree of coloration in a visible light range before irradiation with ultraviolet light (hereinafter referred to as initial coloration) is low;
(II) a color developing density (hereinafter referred to as color developing density) quickly reaches a saturation level from the beginning of irradiation with ultraviolet light (hereinafter also referred to as high color developing sensitivity);
(III) a color quickly returns to its original state after irradiation with ultraviolet light is stopped (hereinafter referred to as color fading rate);
(IV) repetition durability of the above-mentioned reversible action is high; and
(V) solubility in a material that makes up a matrix of a lens is high and dispersibility within a cured product is high.

Known photochromic compounds that can satisfy such requirements include chromene compounds having an indeno(2,1-f)naphtho(1,2-b)pyran structure as a basic skeleton. For example, a chromene compound represented by the following formula (A) (Patent Document 1), a chromene compound represented by the following formula (B) (Patent Document 2), and a chromene compound represented by the following formula (C) (Patent Document 3) are known.

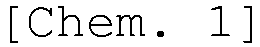

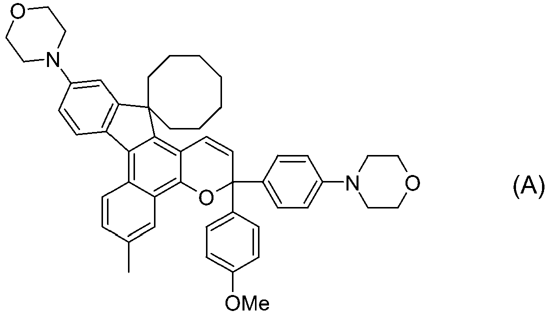

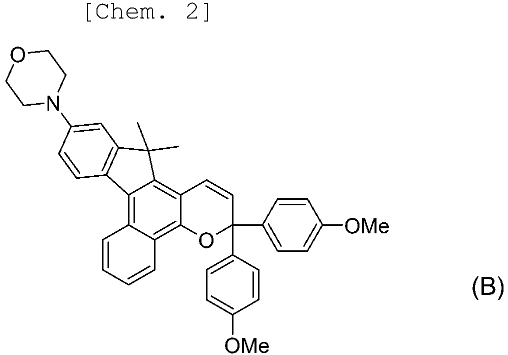

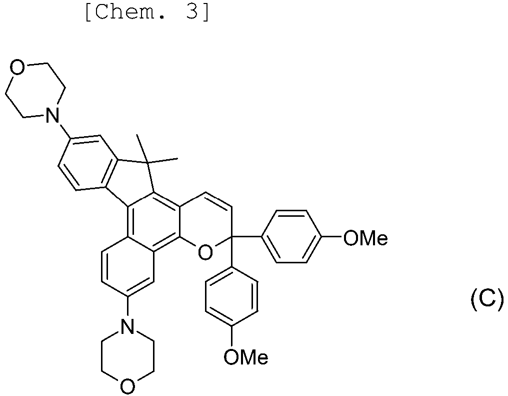

In recent years, the performance required of photochromic compounds has become increasingly sophisticated, and the photochromic compounds are required to exhibit high color developing density even at higher temperatures like in the summer season in addition to the above-mentioned properties (I) to (V). Hereinafter, such a property may be referred to as "low temperature dependence". In general, an improvement of thermal stability in the color-developed state leads to the high color developing density at higher temperatures, but results in a reduction in color fading rate. Thus, the color developing density at higher temperatures and the color fading rate are in a trade-off relationship. Photochromic compounds are needed which combine the color developing density at higher temperatures and the high color fading rate.

### Citation List

### Patent Documents

Patent Document 1: Japanese Unexamined Patent Application, Publication No. 2018-062496
Patent Document 2: PCT International Publication No. WO2005/090327
Patent Document 3: U.S. Published Patent Application Publication, No. 2012/0053341, Specification
Patent Document 4: PCT International Publication No. WO2018/215371
Patent Document 5: PCT International Publication No. WO2013/042800
Patent Document 6: PCT International Publication No. WO2015/035325
Patent Document 7: PCT International Publication No. WO2018/235771
Patent Document 8: PCT International Publication No. WO2012/102410
Patent Document 9: PCT International Publication No. WO2011/053615
Patent Document 10: PCT International Publication No. WO2007/078529
Patent Document 11: PCT International Publication No. WO2019/013249
Patent Document 12: PCT International Publication No. WO2016/143910

### DISCLOSURE OF THE INVENTION

### Problems to be Solved by the Invention

An object of the present invention is to provide a photochromic compound having excellent temperature dependence and color fading rate, a phenol derivative that can be an intermediate of the photochromic compound, a curable composition containing the photochromic compound, an optical article, a lens, and eyeglasses.

### Means for Solving the Problems

According to the present disclosure, a photochromic compound having a skeleton represented by the following formula (1) is provided.

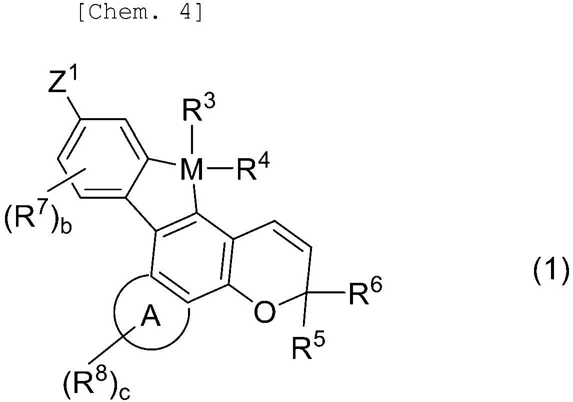

In the formula (1), M is C, Si, or Ge. The ring A is an aromatic ring, an aromatic heterocyclic ring, or a substituted or unsubstituted fused polycyclic ring in which an aromatic ring or an aromatic heterocyclic ring is fused to the aromatic ring or the aromatic heterocyclic ring.

Z¹ is a group represented by the following formula (1a), or a group represented by the following formula (1b).

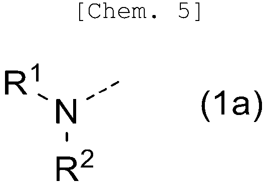

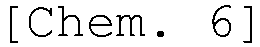

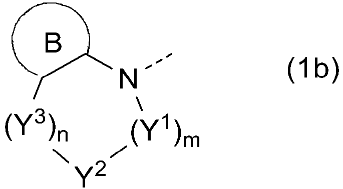

In the formula (1a), R¹ is a substituted or unsubstituted aryl group, a substituted or unsubstituted heteroaryl group, or a substituted or unsubstituted fused polycyclic ring in which an aromatic ring or an aromatic heterocyclic ring is fused to the aryl group or the heteroaryl group.

R² is a hydrogen atom, a substituted or unsubstituted alkyl group, a substituted or unsubstituted haloalkyl group, a substituted or unsubstituted cycloalkyl group, a substituted or unsubstituted alkoxy group, a substituted or unsubstituted aryl group, a substituted or unsubstituted heteroaryl group, or a group represented by the following formula (2a).

-Q¹-(X¹Q²) a-X²Q³ (2a)

In the formula (2a), Q¹ is an alkylene group or a haloalkylene group,
Q² is an alkylene group or a haloalkylene group, and Q³ is an alkyl group or a haloalkyl group. X¹ and X² are each independently O, S, NR⁷⁰⁰, PR⁷⁰¹, or P(=O). R⁷⁰⁰ and R⁷⁰¹ are each independently a hydrogen atom, a substituted or unsubstituted alkyl group, a substituted or unsubstituted cycloalkyl group, a substituted or unsubstituted aryl group, or a substituted or unsubstituted heteroaryl group, and a is 0, or 1 or more and 3 or less.

In the formula (1b), the ring B is a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to the aromatic ring or the aromatic heterocyclic ring.

Y¹ is a substituted or unsubstituted methylene group. Y² is a substituted or unsubstituted methylene group, O, S, SO₂, NR⁶⁰⁰, R⁶⁰¹C=CR⁶⁰², or CC. R⁶⁰⁰, R⁶⁰¹, and R⁶⁰² are each independently a hydrogen atom, a substituted or unsubstituted alkyl group, a substituted or unsubstituted alkoxy group, a substituted or unsubstituted haloalkyl group, a substituted or unsubstituted haloalkoxy group, a substituted or unsubstituted cycloalkyl group, a substituted or unsubstituted aryl group, or a substituted or unsubstituted heteroaryl group. Y³ is a substituted or unsubstituted methylene group.
m is an integer of 1 to 4, n is an integer of 0 to 4, and a sum of m and n is an integer of 1 or more.

R³ and R⁴ are each independently a hydrogen atom, a hydroxyl group, a substituted or unsubstituted alkyl group, a haloalkyl group, the group represented by the formula (2a), a substituted or unsubstituted cycloalkyl group, a substituted or unsubstituted alkoxy group, a formyl group, a hydroxycarbonyl group, an alkylcarbonyl group, an alkoxycarbonyl group, a halogen atom, a substituted or unsubstituted aralkyl group, a substituted or unsubstituted aralkoxy group, a substituted or unsubstituted aryloxy group, a substituted or unsubstituted heteroaryl group, or a group represented by the following formula (X3).

L¹-R⁴⁰⁰ (X3)

In the formula (X3), R⁴⁰⁰ is a hydrogen atom, an alkyl group, an aryl group, a polymerizable group, a photochromic group, or a silyl group having an alkyl group, an alkoxy group or an aryl group as a substituent, and L¹ is a group represented by the following formula (X2).

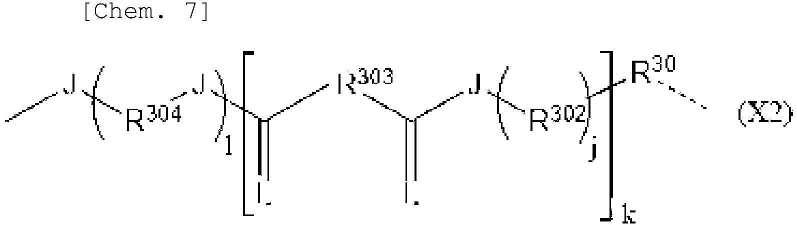

In the formula (X2), R³⁰ is a group represented by the following formula (X2a).

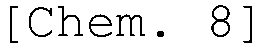

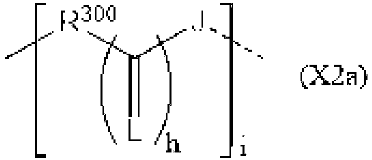

In the formula (X2) and the formula (X2a), J is a divalent group, and is each independently a direct bond, a substituted or unsubstituted methylene group, an oxygen atom, a sulfur atom, or NR³⁰¹, and R³⁰¹ is a hydrogen atom or an alkyl group, L is an oxygen atom or a sulfur atom, R³⁰⁰ is an alkylene group, or a silylene group having an alkyl group or an aryl group as a substituent, R³⁰², R³⁰³, and R³⁰⁴ are each independently an alkylene group, h, j, k and 1 are each independently an integer of 0 or 1, i is an integer of 1 to 200, and when i is 2 or more, a plurality of R³⁰ may be identical or different, and the dashed line represents a bond to R⁴⁰⁰.

R³ and R⁴ are optionally taken together with M to form a substituted or unsubstituted aliphatic ring having 3 to 20 ring member carbon atoms, a substituted or unsubstituted fused polycyclic ring in which an aromatic hydrocarbon ring or an aromatic heterocyclic ring is fused to the aliphatic ring, a substituted or unsubstituted heterocyclic ring having 3 to 20 ring member atoms, or a substituted or unsubstituted fused polycyclic ring in which an aromatic ring or an aromatic heterocyclic ring is fused to the heterocyclic ring.

R⁵ and R⁶ are each independently a hydrogen atom or a substituent, R⁷ and R⁸ are each independently a substituent, b is an integer of 0 to 3, and c is an integer of 0 to 4.

According to the present disclosure, a curable composition is provided. The curable composition contains the photochromic compound according to the embodiment, and at least one selected from the group consisting of a radically-polymerizable monomer, a cationically-polymerizable monomer, a compound having a polymerization-reactive group, and a (thio)urethane(urea) polymer.

According to another embodiment, an optical article is provided. The optical article contains a cured product of the curable composition.

According to the present disclosure, a lens is provided. The lens contains the photochromic compound according to the embodiment.

According to the present disclosure, eyeglasses are provided. The eyeglasses include the lens according to the embodiment.

According to the present disclosure, a phenol derivative is provided. The phenol derivative has a skeleton represented by the following formula (5).

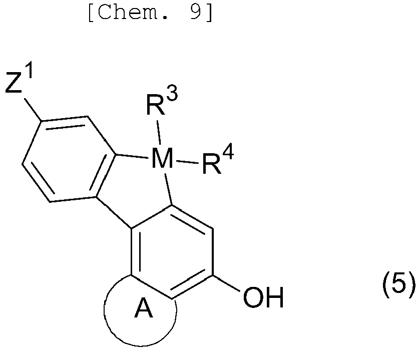

In the formula (5), M, the ring A, Z¹, R³ and R⁴ are each independently as defined in the above formula (1).

### Effects of the Invention

According to the present invention, there is provided a photochromic compound that has an excellent temperature dependence and color fading rate; a phenol derivative that can be an intermediate of the photochromic compound; and a curable composition containing the photochromic compound, an optical article, a lens, and eyeglasses.

### BRIEF DESCRIPTION OF THE DRAWINGS

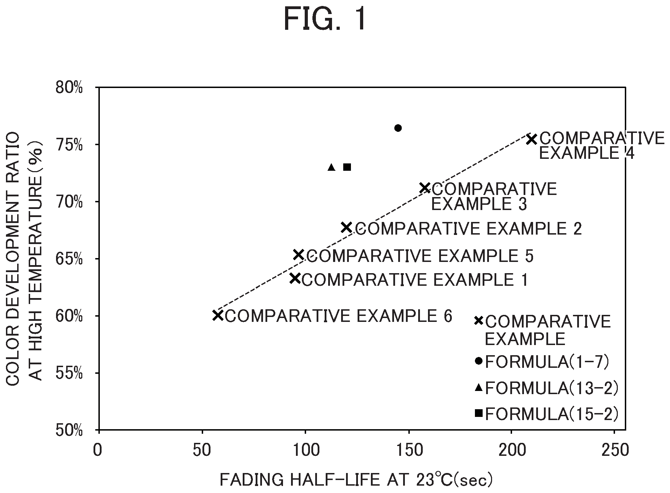

[FIG. 1] FIG. 1 is a graph showing an example of the relationship between the fading half-life at 23°C and the color development ratio at a higher temperature of the photochromic laminates according to Examples and Comparative Examples.

### PREFERRED MODE FOR CARRYING OUT THE INVENTION

### [Photochromic Compound]

According to an embodiment, a photochromic compound represented by the following formula (1) is provided.

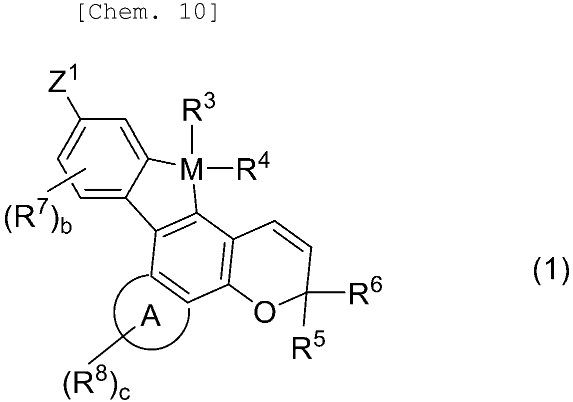

In the formula (1), M is C, Si, or Ge. The ring A is a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to the aromatic ring or the aromatic heterocyclic ring. Z¹ is a group represented by the following formula (1a), or a group represented by the following formula (1b).

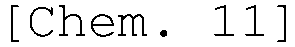

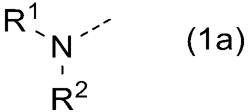

In the formula (1a), R¹ is a substituted or unsubstituted aryl group or a substituted or unsubstituted heteroaryl group. R² is a hydrogen atom, a substituted or unsubstituted alkyl group, a substituted or unsubstituted haloalkyl group, a substituted or unsubstituted cycloalkyl group, a substituted or unsubstituted alkoxy group, a substituted or unsubstituted aryl group, a substituted or unsubstituted heteroaryl group, or a group represented by the following formula (2a).

-Q¹-(X¹Q²) a-X²Q³ (2a)

In the formula (2a), Q¹ is an alkylene group or a haloalkylene group. Q² is an alkylene group or a haloalkylene group. Q³ is an alkyl group or a haloalkyl group. X¹ and X² are each independently O, S, NR⁷⁰⁰, PR⁷⁰¹, or P(=O). R⁷⁰⁰ and R⁷⁰¹ are each independently a hydrogen atom, a substituted or unsubstituted alkyl group, a substituted or unsubstituted cycloalkyl group, a substituted or unsubstituted aryl group, or a substituted or unsubstituted heteroaryl group.
a is 0, or 1 or more and 3 or less.

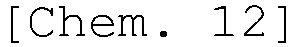

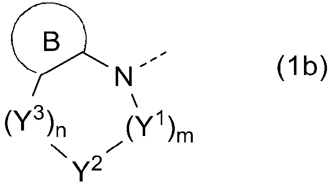

In the formula (1b), the ring B is a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to the aromatic ring or the aromatic heterocyclic ring. Y¹ is a substituted or unsubstituted methylene group. Y² is a substituted or unsubstituted methylene group, O, S, SO₂, NR⁶⁰⁰, R⁶⁰¹C=CR⁶⁰², or CC. R⁶⁰⁰, R⁶⁰¹, and R⁶⁰² are each independently a hydrogen atom, a substituted or unsubstituted alkyl group, a substituted or unsubstituted alkoxy group, a substituted or unsubstituted haloalkyl group, a substituted or unsubstituted haloalkoxy group, a substituted or unsubstituted cycloalkyl group, a substituted or unsubstituted aryl group, or a substituted or unsubstituted heteroaryl group. Y³ is a substituted or unsubstituted methylene group, or oxygen atom.
m is an integer of 1 to 4, n is an integer of 1 to 4, and a sum of m and n is an integer of 1 or more.

R³ and R⁴ are each independently a hydrogen atom, a hydroxyl group, a substituted or unsubstituted alkyl group, a haloalkyl group, the group represented by the formula (2a), a substituted or unsubstituted cycloalkyl group, a substituted or unsubstituted alkoxy group, a formyl group, a hydroxycarbonyl group, an alkylcarbonyl group, an alkoxycarbonyl group, a halogen atom, a substituted or unsubstituted aralkyl group, a substituted or unsubstituted aralkoxy group, a substituted or unsubstituted aryloxy group, a substituted or unsubstituted heteroaryl group, or a group represented by the following formula (X3).

R³ and R⁴ are optionally taken together with M to form a substituted or unsubstituted aliphatic ring having 3 to 20 ring member carbon atoms, a substituted or unsubstituted fused polycyclic ring in which an aromatic hydrocarbon ring or an aromatic heterocyclic ring is fused to the aliphatic ring, a substituted or unsubstituted heterocyclic ring having 3 to 20 ring member atoms, or a substituted or unsubstituted fused polycyclic ring in which an aromatic ring or an aromatic heterocyclic ring is fused to the heterocyclic ring.

L¹- R⁴⁰⁰ (X3)

In the formula (X3), R⁴⁰⁰ is a hydrogen atom, an alkyl group, an aryl group, a polymerizable group, a photochromic group, or a silyl group having an alkyl group, an alkoxy group or an aryl group as a substituent.

L¹ is a group represented by the following formula (X2).

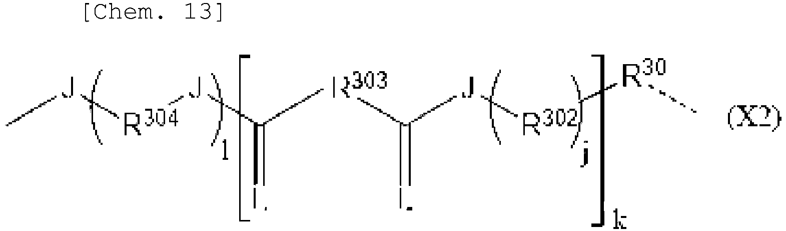

In the formula (X2), R³⁰ is a group represented by the following formula (X2a).

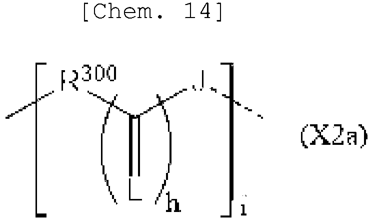

In the formula (X2) and the formula (X2a), J is a divalent group, and is each independently a direct bond, a substituted or unsubstituted methylene group, an oxygen atom, a sulfur atom, or NR³⁰¹, and R³⁰¹ is a hydrogen atom or an alkyl group.

L is an oxygen atom or a sulfur atom. R³⁰⁰ is an alkylene group, or a silylene group having an alkyl group or an aryl group as as a substituent.

R³⁰², R³⁰³, and R³⁰⁴ are each independently an alkylene group.
h, j, k and 1 are each independently an integer of 0 or 1. i is an integer of 1 to 200, and when i is 2 or more, a plurality of R³⁰ may be identical or different, and the dashed line represents a bond to R⁴⁰⁰. R⁵ and R⁶ are each independently a hydrogen atom or a substituent. R⁷ and R⁸ are each independently a substituent.
b is an integer of 0 to 3.
c is an integer of 0 to 4.

The photochromic compound has excellent temperature dependence and color fading rate. The reasons for this are not clear, but the present inventor believes as follows.

The above formula (1a) or the above formula (1b) is used in the substituent Z¹ of the photochromic compound according to the embodiment. It is believed that the above formula (1a) and the above formula (1b) have a structure in which the aryl group or the heteroaryl group directly bonds to the nitrogen atom, and the conjugation between the skeleton represented by the formula (1) and the substituent Z¹ is possible through a p orbital on the nitrogen atom. A compound having such a structure is considered to exhibit extended conjugation compared to a compound in which the substitution by the above formula (1a) and the above formula (1b) is absent. This extension is believed to increase the amount of absorbed light in a decolored state, and improve the color developing density even at higher temperatures.

When the substitution position at which the substituent Z¹ bonds is substituted with a highly electron donating substituent, the color fading rate of the photochromic compound tends to decrease. In the substituent Z¹, the electron donating nature is believed to be reduced due to the conjugation, since the aryl group or the heteroaryl group bonds to the nitrogen atom, as described above. Therefore, it is believed that in the photochromic compound having the substituent Z¹, a decrease in the color fading rate is suppressed.

In light of the above, the photochromic compound according to the embodiment can be used to achieve a cured product with an excellent temperature dependence and color fading rate. Therefore, such a photochromic compound is suitable for an optical article to be used in an environment with a large temperature variation, such as sunglasses.

Hereinafter, the details of the photochromic compound represented by the formula (1) will be explained.

### <M>

In the formula (1), M is C, Si, or Ge. M is preferably C.

### <Ring A>

In the formula (1), the ring A is a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to the aromatic ring or the aromatic heterocyclic ring.

Examples of the aromatic hydrocarbon ring include a benzene ring, a cyclotetradecaheptaene ring, and the like.

Examples of the aromatic heterocyclic ring include a furan ring, a thiophene ring, a pyridine ring, and the like.

Examples of the fused polycyclic ring include a naphthalene ring, a fluorene ring, an anthracene ring, a phenanthrene ring, a tetracene ring, a pentacene ring, a benzo pyrene ring, a chrysene ring, a pyrene ring, a triphenylene ring, a perylene ring, a benzofuran ring, a benzothiophene ring, a quinoline ring, an isoquinoline ring, an indole ring, a pyrimidine ring, a quinazoline ring, a pyridazine ring, a cinnoline ring, a phthalazine ring, a 1,2,3-, 1,2,4- or 1,3,5-triazine ring, a carbazole ring, a benzoxazole ring, an isothiazole ring, and the like.

Of these, a benzene ring, a naphthalene ring, a fluorene ring, a phenanthrene ring, a pyrene ring, a furan ring, a thiophene ring, or a pyridine ring is preferable, a benzene ring, a naphthalene ring, a fluorene ring, or a phenanthrene ring is more preferable, and a benzene ring is most preferable.

### <Z¹>

In the formula (1), Z¹ is a group represented by the following formula (1a), or a group represented by the following formula (1b). The dashed line in the formula (1a) and the formula (1b) means a bond for bonding to the carbon atom at position 11 of the naphthopyran skeleton in the formula (1).

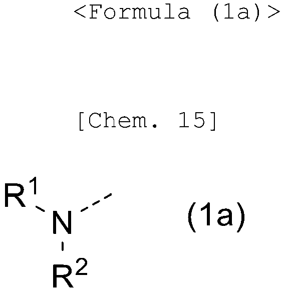

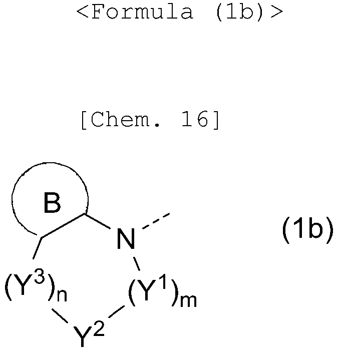

### (R¹)

R¹ is a substituted or unsubstituted aryl group, a substituted or unsubstituted heteroaryl group, or a substituted or unsubstituted fused polycyclic ring in which an aromatic ring or an aromatic heterocyclic ring is fused to the aryl group or the heteroaryl group. The substituent optionally included in R¹ will be described later.

The number of carbon atoms in the substituted or unsubstituted aryl group is, for example, 5 or more and 12 or less, and preferably 6 or more and 10 or less. Examples of the heteroatom in the substituted or unsubstituted heteroaryl group include at least one selected from the group consisting of an oxygen atom, a sulfur atom, a nitrogen atom, and a phosphorus atom. The number of heteroatoms in the substituted or unsubstituted heteroaryl group is, for example, 1 or more and 3 or less, and preferably 1 or 2. The number of carbon atoms in the substituted or unsubstituted heteroaryl group is, for example, 4 or more and 11 or less, and preferably 5 or more and 9 or less.

R¹ is preferably a substituted or unsubstituted phenyl group, a substituted or unsubstituted 1-naphthyl group, a substituted or unsubstituted 2-naphthyl group, a substituted or unsubstituted thienyl group, a substituted or unsubstituted furyl group, a substituted or unsubstituted pyrrolinyl group, a substituted or unsubstituted pyridyl group, a substituted or unsubstituted benzothienyl group, a substituted or unsubstituted benzofuranyl group, or a substituted or unsubstituted benzopyrrolinyl group, and more preferably a substituted or unsubstituted phenyl group, a substituted or unsubstituted 1-naphthyl group, or a substituted or unsubstituted 2-naphthyl group, and particularly preferably a substituted or unsubstituted phenyl group.

The substituent optionally included in R¹ is preferably at least one selected from the group consisting of a linear or branched alkyl group having 1 or more and 6 or less carbon atoms, a linear or branched haloalkyl group having 1 or more and 6 or less carbon atoms, a linear or branched alkoxy group having 1 or more and 6 or less carbon atoms, and a halogen atom. The number of substituents is, for example, 1 or more and 3 or less.

### (R²)

R² is a hydrogen atom, a substituted or unsubstituted alkyl group, a substituted or unsubstituted haloalkyl group, a substituted or unsubstituted cycloalkyl group, a substituted or unsubstituted alkoxy group, a substituted or unsubstituted aryl group, a substituted or unsubstituted heteroaryl group, or a group represented by the following formula (2a).

R² is preferably a hydrogen atom, a substituted or unsubstituted alkyl group, a substituted or unsubstituted haloalkyl group, a substituted or unsubstituted cycloalkyl group, a substituted or unsubstituted alkoxy group, a substituted or unsubstituted aryl group, a substituted or unsubstituted heteroaryl group, or a group represented by the following formula (2a), more preferably a substituted or unsubstituted alkyl group, a substituted or unsubstituted haloalkyl group, a substituted or unsubstituted cycloalkyl group, a substituted or unsubstituted alkoxy group, or a group represented by the following formula (2a), and particularly preferably a substituted or unsubstituted alkyl group, a substituted or unsubstituted haloalkyl group, or a group represented by the following formula (2a).

The alkyl group is preferably an unsubstituted alkyl group having 1 or more and 10 or less carbon atoms, and more preferably an unsubstituted alkyl group having 1 or more and 6 or less carbon atoms. Specific examples of the alkyl group include a methyl group, an ethyl group, a propyl group, a butyl group, a pentyl group, or a hexyl group.

The haloalkyl group is preferably a haloalkyl group having 1 or more and 6 or less carbon atoms. The number of halogen atoms is preferably 1 or more and 10 or less, and more preferably 2 or more and 5 or less. The haloalkyl group having 1 or more and 6 or less carbon atoms is preferably an alkyl group substituted by a fluorine atom, a chlorine atom, or a bromine atom. The haloalkyl group preferably has a terminal perfluoromethyl group. Examples of a suitable haloalkyl group include a trifluoromethyl group, a trifluoroethyl group, a trifluoropropyl group, a tetrafluoroethyl group, a chloromethyl group, a 2-chloroethyl group, a bromomethyl group, and the like.

The cycloalkyl group is preferably a cycloalkyl group having 3 or more and 8 or less carbon atoms (cycloalkyl group having 3 or more and 8 or less carbon atoms forming its ring). Examples of the cycloalkyl group having 3 or more and 8 or less carbon atoms include a cyclopropyl group, a cyclobutyl group, a cyclopentyl group, a cyclohexyl group, and the like. Note that the cycloalkyl group may have a substituent, but the number of carbon atoms in the substituent shall be excluded from the number of carbon atoms of the cycloalkyl group (3 or more and 8 or less carbon atoms).

The alkoxy group is preferably an alkoxy group having 1 or more and 6 or less carbon atoms. Examples of a suitable alkoxy group having 1 or more and 6 or less carbon atoms include a methoxy group, an ethoxy group, an n-propoxy group, an isopropoxy group, an n-butoxy group, a sec-butoxy group, a tert-butoxy group, and the like.

The aryl group may be exemplified by the same aryl groups as those mentioned with regard to R¹. The aryl group is preferably a phenyl group or a naphthyl group.

The heteroaryl group may be exemplified by the same heteroaryl groups as those mentioned with regard to R¹.

The substituent optionally included in R² is preferably at least one selected from the group consisting of a linear or branched alkyl group having 1 or more and 6 or less carbon atoms, a linear or branched haloalkyl group having 1 or more and 6 or less carbon atoms, a linear or branched alkoxy group having 1 or more and 6 or less carbon atoms, and a halogen atom. The number of the substituents is, for example, 1 or more and 3 or less.

R² is preferably any of an alkyl group having 1 or more and 5 or less carbon atoms, a fluoroalkyl group having 1 or more and 5 or less carbon atoms, an alkoxy group having 1 or more and 5 or less carbon atoms, an alkoxyalkyl group having 2 or more and 6 or less carbon atoms, a phenyl group, or a phenyl group having a substituent. The fluoroalkyl group having 1 or more and 5 or less carbon atoms preferably has a terminal perfluoromethyl group.

### (Group Represented by Formula (2a))

-Q¹-(X¹Q²) a-X²Q³ (2a)

In the formula (2a), Q¹ is an alkylene group or a haloalkylene group.

The number of carbon atoms of the alkylene group is preferably 1 or more and 20 or less, more preferably 1 or more and 12 or less, still more preferably 1 or more and 7 or less, and most preferably 2 or more and 6 or less. The halogen atom of the haloalkylene group may be at least one selected from the group consisting of I, Cl, Br, and F. The halogen atom is preferably at least one of Cl and F, and more preferably F. The haloalkylene group preferably has a halogen atom bonded to the terminal carbon thereof, and more preferably has a terminal perfluoromethyl group.

Q² is an alkylene group or a haloalkylene group. Preferred embodiments of the alkylene group or haloalkylene group are identical to those for Q¹. The number of carbon atoms of the alkylene group or haloalkylene group in Q² may be the same as or different from the number of carbon atoms of the alkylene group or haloalkylene group in Q¹.

Q³ is an alkyl group or a haloalkyl group, and may be linear or branched, and is preferably linear.

The number of carbon atoms of the alkyl group is preferably 1 or more and 20 or less, more preferably 1 or more and 12 or less, and most preferably 1 or more and 7 or less. The halogen atom of the haloalkyl group may be at least one selected from the group consisting of I, Cl, Br, and F. The halogen atom is preferably at least one of Cl and F, and more preferably F.

Q³ is preferably a linear alkyl group.

X¹ and X² are each independently O, S, NR⁷⁰⁰, PR⁷⁰¹, or P (=O). X¹ and X² are preferably O, S, or NR⁷⁰⁰, and most preferably O.

R⁷⁰⁰ and R⁷⁰¹ are each independently a hydrogen atom, a substituted or unsubstituted alkyl group, a substituted or unsubstituted cycloalkyl group, a substituted or unsubstituted aryl group, or a substituted or unsubstituted heteroaryl group.

R⁷⁰⁰ is preferably a hydrogen atom, a substituted or unsubstituted alkyl group, or a substituted or unsubstituted aryl group. The alkyl group is preferably a methyl group, an ethyl group, a propyl group, a butyl group, or a pentyl group. The aryl group is preferably a phenyl group or a naphthyl group.

R⁷⁰¹ is preferably a hydrogen atom, a substituted or unsubstituted alkyl group, or a substituted or unsubstituted aryl group. The alkyl group is preferably a methyl group, an ethyl group, a propyl group, a butyl group, or a pentyl group. The aryl group is preferably a phenyl group or a naphthyl group.

a is 0, or 1 or more and 3 or less.

The formula (2a) is preferably an alkylenealkoxy group, an alkylenethioalkyl group, or an alkyleneoxyalkylenealkoxy group.

Specific examples of the group represented by the formula (2a) include -CH₂OCH₃, -CH₂SCH₃, -CH₂CH₂OCH₃, -CH₂CH₂OCH₂CH₃, - CH₂CH₂SCH₃, -CH₂CH₂CH₂OCH₃, -CH₂CH₂CH₂SCH₃, -CH₂CH₂OCH₂CH₂OCH₃, and - CH₂CH₂OCH₂CH₂OCH₂CH₃.

The group represented by the formula (2a) is preferably - CH₂OCH₃, -CH₂CH₂OCH₃, -CH₂CH₂OCH₂CH₃, -CH₂CH₂CH₂OCH₃, - CH₂CH₂OCH₂CH₂OCH₃, or -CH₂CH₂OCH₂CH₂OCH₂CH₃.

### (Specific Examples of Formula (1a))

Suitable formula (1a) is exemplified by the following.

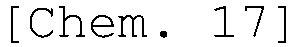

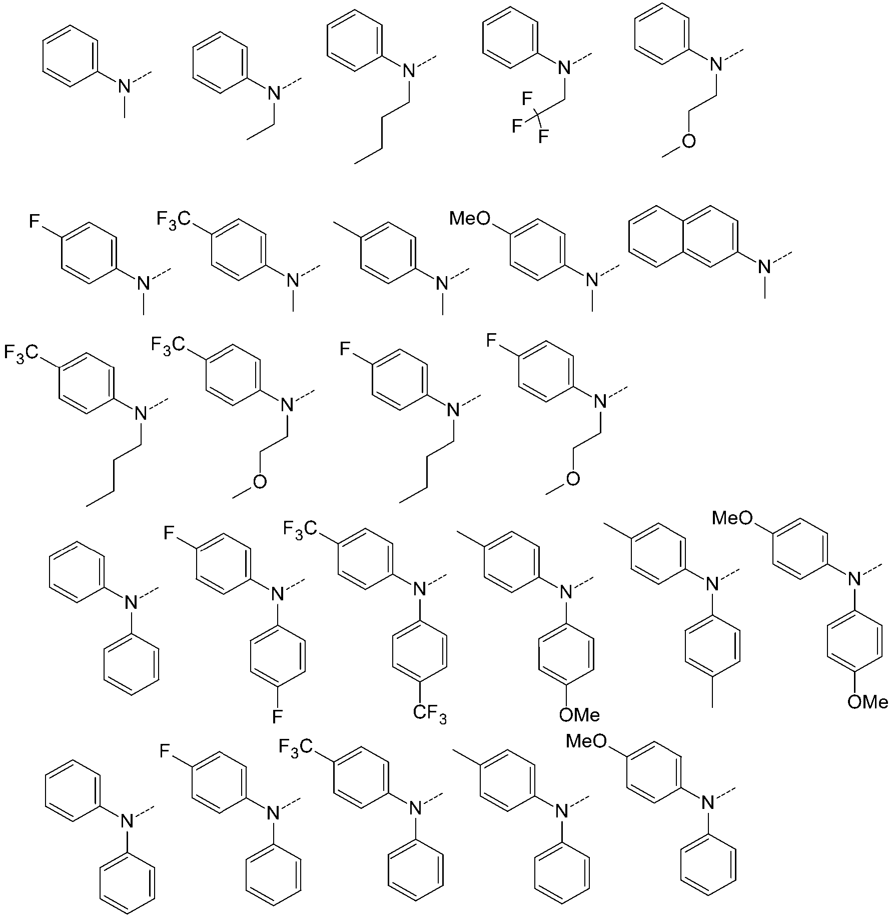

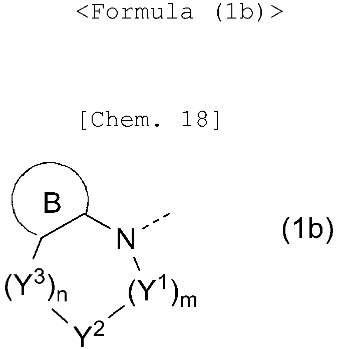

In the formula (1b), the ring B is a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to the aromatic ring or the aromatic heterocyclic ring. Suitable rings B are the same as those exemplified in the ring A.

Y¹ is a substituted or unsubstituted methylene group. When Y¹ has a substituent, the substituent is preferably a halogen atom, a haloalkyl group having 1 or more and 3 or less carbon atoms, or an alkyl group having 1 or more and 3 or less carbon atoms, and more preferably a fluorine atom or a methyl group. When Y¹ has a substituent, the number of the substituents is 1 or 2.

Y¹ is more preferably a methylene group substituted by a methyl group or a fluorine atom, or an unsubstituted methylene group, and particularly preferably an unsubstituted methylene group.

Y² is a substituted or unsubstituted methylene group, O, S, SO₂, NR⁶⁰⁰, R⁶⁰¹C=CR⁶⁰², or CC.

R⁶⁰⁰, R⁶⁰¹, and R⁶⁰² are each independently a hydrogen atom, a substituted or unsubstituted alkyl group, a substituted or unsubstituted alkoxy group, a substituted or unsubstituted haloalkyl group, a substituted or unsubstituted haloalkoxy group, a substituted or unsubstituted cycloalkyl group, a substituted or unsubstituted aryl group, or a substituted or unsubstituted heteroaryl group, and preferably a hydrogen atom, a substituted or unsubstituted alkyl group, a substituted or unsubstituted cycloalkyl group, or a substituted or unsubstituted aryl group.

Y² is preferably a substituted or unsubstituted methylene group, O, S, SO₂, or NR⁶⁰⁰, and more preferably an unsubstituted methylene group, O, S, or SO₂.

Y³ is a substituted or unsubstituted methylene group, and suitable Y³ is the same as those exemplified in Y¹.

m is an integer of 1 to 4.
m is preferably 1 or 2.
n is an integer of 0 to 4.
n is preferably 0 or 1. A sum of m and n is an integer of 1 or more. The sum of m and n is preferably 1 or more and 4 or less, and more preferably 1 or 2. When n is 0, Y² bonds to the ring B.

Suitable formula (1b) is exemplified by the following.

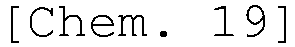

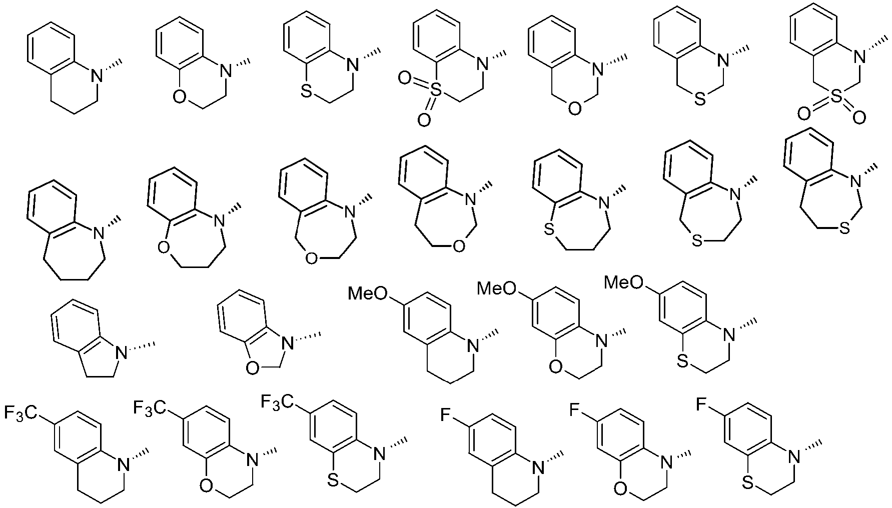

### <R³ and R⁴>

R³ and R⁴ are each independently a hydroxyl group, a substituted or unsubstituted alkyl group, a haloalkyl group, a substituted or unsubstituted cycloalkyl group, a substituted or unsubstituted alkoxy group, a substituted or unsubstituted amino group, a substituted or unsubstituted heterocyclic group, a cyano group, a halogen atom, a substituted or unsubstituted alkylthio group, a substituted or unsubstituted arylthio group, a nitro group, a formyl group, a hydroxycarbonyl group, a substituted or unsubstituted alkylcarbonyl group, a substituted or unsubstituted alkoxycarbonyl group, a substituted or unsubstituted aralkyl group, a substituted or unsubstituted aralkoxy group, a substituted or unsubstituted aryloxy group, a substituted or unsubstituted heteroaryl group, a thiol group, a substituted or unsubstituted alkoxyalkylthio group, a haloalkylthio group, a substituted or unsubstituted cycloalkylthio group, a substituted or unsubstituted silyl group, a substituted or unsubstituted oxysilyl group, the group represented by the formula (2a), or a group represented by the following formula (X3). R³ and R⁴ are a group excluding a substituted or unsubstituted phenyl group.

The substituted or unsubstituted alkyl group, the haloalkyl group, the group represented by the formula (2a), the substituted or unsubstituted cycloalkyl group, the substituted or unsubstituted alkoxy group, and the substituted or unsubstituted heteroaryl group may be exemplified by the same groups as those mentioned with regard to R¹ or R².

The amino group may be a primary amino group (-NH₂), or a secondary or tertiary amino group in which one or two hydrogen atoms are substituted. A substituent included in the substituted amino groups include an alkyl group having 1 or more and 6 or less carbon atoms, a haloalkyl group having 1 or more and 6 or less carbon atoms, an alkoxy group having 1 or more and 6 or less carbon atoms, a cycloalkyl group having 3 or more and 7 or less carbon atoms, an aryl group having 6 or more and 14 or less carbon atoms, a heteroaryl group having 4 or more and 14 or less carbon atoms, etc. Examples of a suitable amino group include an amino group, a methylamino group, a dimethylamino group, an ethylamino group, a diethylamino group, a methylphenylamino group, a diphenylamino group, etc.

The heterocyclic group is preferably a heterocyclic group having 3 or more and 10 or less ring member atoms. The heteroatom of the heterocyclic group is preferably at least one selected from the group consisting of an oxygen atom, a nitrogen atom, a sulfur atom, and a phosphorus atom. The number of heteroatoms is, for example, 1 or more and 5 or less, and preferably 1 or 2. Specifically, for example, aliphatic heterocyclic groups such as a morpholino group, a piperidino group, a pyrrolidinyl group, a piperazino group, and an N-methylpiperazino group, or aromatic heterocyclic groups such as an indolinyl group, etc. may be mentioned. The heterocyclic group may be a 2,6-dimethylmorpholino group, a 2,6-dimethylpiperidino group, or a 2,2,6,6-tetramethylpiperidino group.

The alkylthio group is preferably an alkylthio group having 1 or more and 6 or less carbon atoms. Examples of the alkylthio group having 1 or more and 6 or less carbon atoms include a methylthio group, an ethylthio group, an n-propylthio group, an isopropylthio group, an n-butylthio group, a sec-butylthio group, a t-butylthio group, etc.

The arylthio group is preferably an arylthio group having 6 or more and 10 or less carbon atoms. Examples of the arylthio group having 6 to 10 carbon atoms include a phenylthio group, a 1-naphthylthio group, a 2-naphthylthio group, etc.

The alkylcarbonyl group is preferably an alkylcarbonyl group having 2 or more and 7 or less carbon atoms. Examples of the alkylcarbonyl group having 2 to 7 carbon atoms include an acetyl group and an ethylcarbonyl group.

The alkoxycarbonyl group is preferably an alkoxycarbonyl group having 2 or more and 7 or less carbon atoms. Examples of the alkoxycarbonyl group having 2 to 7 carbon atoms include a methoxycarbonyl group and an ethoxycarbonyl group.

Examples of the halogen atom include a fluorine atom, a chlorine atom, a bromine atom, and an iodine atom.

The aralkyl group is preferably an aralkyl group having 7 or more and 11 or less carbon atoms. Examples of the aralkyl group having 7 to 11 carbon atoms include a benzyl group, a phenylethyl group, a phenylpropyl group, a phenylbutyl group, a naphthylmethyl group, etc.

The aralkoxy group is preferably an aralkoxy group having 7 or more and 11 or less carbon atoms. Examples of the aralkoxy group having 7 to 11 carbon atoms include a benzyloxy group, a naphthylmethoxy group, etc.

The aryloxy group is preferably an aryloxy group having 6 or more and 12 or less carbon atoms. Examples of the aryloxy group having 6 to 12 carbon atoms include a phenyloxy group, a naphthyloxy group, etc.

The alkoxyalkylthio group is preferably an alkoxyalkylthio group having 2 or more and 9 or less carbon atoms. Examples of the alkoxyalkylthio group having 2 or more and 9 or less carbon atoms include a methoxymethylthio group, a methoxyethylthio group, a methoxy-n-propylthio group, a methoxy-n-butylthio group, an ethoxyethylthio group, an n-propoxypropylthio group, etc.

The haloalkylthio group is preferably a haloalkylthio group having 1 or more and 6 or less carbon atoms. Examples of the haloalkylthio group having 1 or more and 6 or less carbon atoms include a trifluoromethylthio group, a tetrafluoroethylthio group, a chloromethylthio group, a 2-chloroethylthio group, a bromomethylthio group, etc.

The cycloalkylthio group is preferably a cycloalkylthio group having 3 or more and 8 or less carbon atoms. Examples of the cycloalkylthio group having 3 or more and 8 or less carbon atoms include a cyclopropylthio group, a cyclobutylthio group, a cyclopentylthio group, a cyclohexylthio group, etc. Note that the cycloalkylthio group may have a substituent, but the number of carbon atoms in the substituent shall be excluded from the number of carbon atoms of the cycloalkylthio group (3 or more and 8 or less carbon atoms).

The silyl group is optionally substituted. The substituent included in the substituted silyl group is not limited, and examples thereof include an alkyl group having 1 or more and 6 or less carbon atoms, a haloalkyl group having 1 or more and 6 or less carbon atoms, an alkoxy group having 1 or more and 6 or less carbon atoms, a cycloalkyl group having 3 or more and 7 or less carbon atoms, an aryl group having 6 or more and 14 or less carbon atoms, a heteroaryl group having 4 or more and 14 or less carbon atoms, etc.

The oxysilyl group is optionally substituted. The substituent included in the substituted oxysilyl group is not limited, and examples thereof include an alkyl group having 1 or more and 6 or less carbon atoms, a haloalkyl group having 1 or more and 6 or less carbon atoms, an alkoxy group having 1 or more and 6 or less carbon atoms, a cycloalkyl group having 3 to 7 carbon atoms, an aryl group having 6 or more and 14 or less carbon atoms, a heteroaryl group having 4 or more and 14 or less carbon atoms, etc.

Note that the cycloalkyl group, the arylthio group, the aralkyl group, the aralkoxy group, the aryloxy group, the aryl group, the heteroaryl group, and the cycloalkylthio group may be unsubstituted. However, when they have a substituent, 1 to 8 hydrogen atoms, particularly preferably 1 to 4 hydrogen atoms in the group forming a ring is preferably substituted with a substituent selected from a hydroxyl group, an alkyl group having 1 or more and 6 or less carbon atoms, a haloalkyl group having 1 or more and 6 or less carbon atoms, a cycloalkyl group having 3 or more and 8 or less carbon atoms, an alkoxy group having 1 or more and 6 or less carbon atoms, an amino group, a substituted amino group, a heterocyclic group having 3 or more and 8 or less atoms, a cyano group, a nitro group, and a halogen atom. Specific examples of these substituents will be described later.

R³ and R⁴ may be a hydrogen atom, a hydroxyl group, a substituted or unsubstituted alkyl group, a haloalkyl group, the group represented by the formula (2a), a substituted or unsubstituted cycloalkyl group, a substituted or unsubstituted alkoxy group, a formyl group, a hydroxycarbonyl group, a substituted or unsubstituted alkylcarbonyl group, a substituted or unsubstituted alkoxycarbonyl group, a halogen atom, a substituted or unsubstituted aralkyl group, a substituted or unsubstituted aralkoxy group, a substituted or unsubstituted aryloxy group, a substituted or unsubstituted heteroaryl group, or a group represented by the following formula (X3).

R³ and R⁴ are preferably a substituted or unsubstituted alkyl group, a haloalkyl group, the group represented by the formula (2a), or the group represented by the formula (X3). When R³ and R⁴ are these groups, the color fading rate of the photochromic compound tends to be increased.

R³ and R⁴ are optionally taken together with M to form a substituted or unsubstituted aliphatic ring having 3 or more and 20 or less ring member carbon atoms, a substituted or unsubstituted fused polycyclic ring in which an aromatic hydrocarbon ring or an aromatic heterocyclic ring is fused to an aliphatic ring, a substituted or unsubstituted heterocyclic ring having 3 or more and 20 or less ring member atoms, or a substituted or unsubstituted fused polycyclic ring in which an aromatic ring or an aromatic heterocyclic ring is fused to a heterocyclic ring.

R³ and R⁴ preferably form a substituted or unsubstituted aliphatic ring having 3 or more and 20 or less ring member carbon atoms, a substituted or unsubstituted fused polycyclic ring in which an aromatic hydrocarbon ring or an aromatic heterocyclic ring is fused to an aliphatic ring, a substituted or unsubstituted heterocyclic ring having 3 or more and 20 or less ring member atoms, and more preferably, together with M, form a ring selected from a cyclopentane ring, a cyclohexane ring, a cycloheptane ring, a cyclooctane ring, a cyclononane ring, a cyclodecane ring, a cycloundecane ring, a cyclododecane ring, and a spirodicyclohexane ring. Further, the rings may have 1 to 10 alkyl groups having 1 or more and 5 or less carbon atoms or cycloalkyl groups having 5 or more and 7 or less carbon atoms as substituents, and may be fused to a cycloalkyl group having 5 or more and 7 or less carbon atoms. Specifically, R³ and R⁴ more preferably form any of the rings shown below.

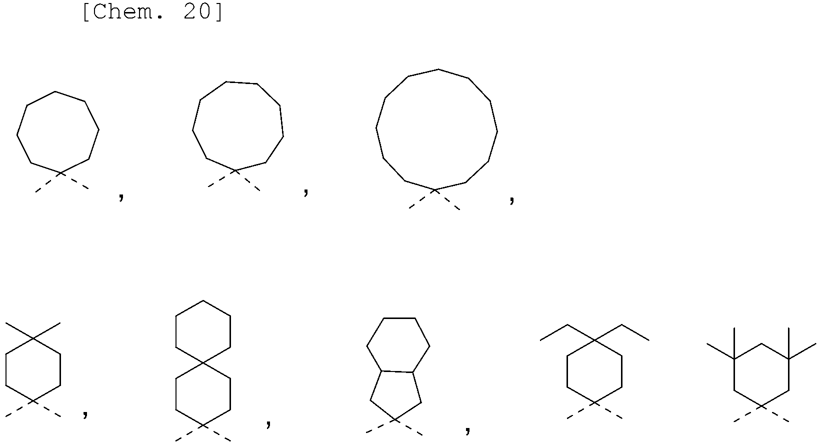

The substituent optionally included in R³ and R⁴ is preferably a linear or branched alkyl group having 1 or more and 6 or less carbon atoms.

### <Group Represented by Formula (X3)>

L¹-R⁴⁰⁰ (X3)

In the formula (X3), R⁴⁰⁰ is a hydrogen atom, an alkyl group, an aryl group, a polymerizable group, a photochromic group, or a silyl group having an alkyl group, an alkoxy group or an aryl group as a substituent.

L¹ is a group represented by the following formula (X2).

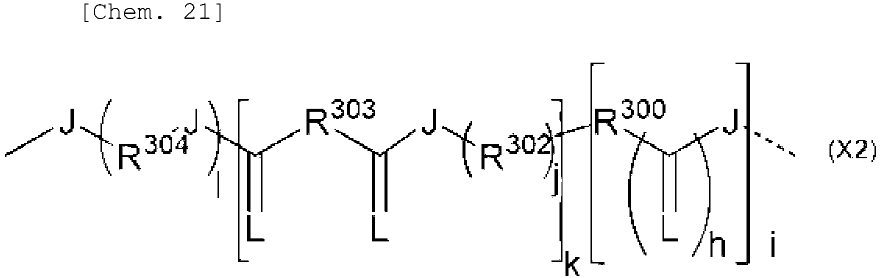

In the formula (X2), J is a divalent group. A plurality of J are each independently a direct bond, a substituted or unsubstituted methylene group, an oxygen atom, a sulfur atom, or NR³⁰¹. R³⁰¹ is a hydrogen atom or an alkyl group. R³⁰¹ is preferably an alkyl group having 1 or more and 20 or less carbon atoms. The alkyl group preferably has, as a substituent, a silyl group having an alkyl group having 1 or more and 10 or less carbon atoms, a polymerizable group, or a photochromic group.

Examples of the polymerizable group include radically-polymerizable groups such as a vinyl group, a 1-chlorovinyl group, an allyl group, a styryl group, a (meth)acryl group, a 2-(methacryloxy)ethylcarbamyl group, a 2-(methacryloxy)ethoxycarbonyl group, and a crotyl group. Further, in addition to these groups, an epoxy group, an episulfide group, a thietanyl group, an OH group, an SH group, an NH₂ group, a COOH group, an NCO group, or an NCS group and the like may be mentioned. The polymerizable group is preferably at least one selected from the group consisting of a (meth)acryl group, a 2-(methacryloxy)ethylcarbamyl group, a 2-(methacryloxy)ethoxycarbonyl group, an epoxy group, an OH group, an SH group, an NH₂ group, and a COOH group.

The photochromic group is a group that includes a photochromic moiety. The photochromic group is exemplified by naphthopyrans, spirooxazines, spiropyrans, fulgides, fulgimides, and diarylethenes as typical ones. From the viewpoint of exhibiting excellent photochromic properties, indenonaphthopyrans are preferable, and, among those, indeno[2,1-f]naphtho[1,2-b]pyran is particularly preferable.

The indeno[2,1-f]naphtho[1,2-b]pyran is preferably a group represented by the following formula (X4).

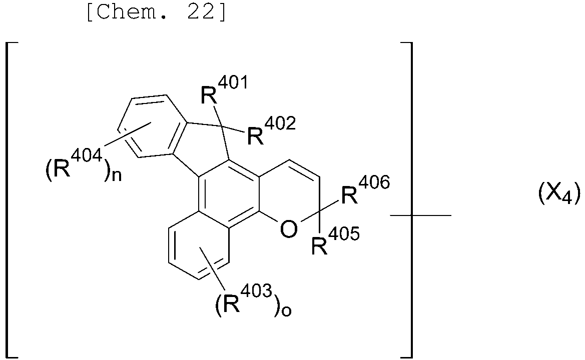

In the formula (X4), R⁴⁰¹ and R⁴⁰² may the same group as those used in R³ and R⁴ mentioned above.

R⁴⁰³ and R⁴⁰⁴ may be each independently the same groups as those used in R³ and R⁴ mentioned above.

R⁴⁰⁵ and R⁴⁰⁶ are each independently a substituted or unsubstituted aryl group or a substituted or unsubstituted heteroaryl group. R⁴⁰⁵ and R⁴⁰⁶ may the same groups as those used in R⁵ and R⁶ as described later.

o is an integer of 0 to 4.
n is an integer of 0 to 4. When o is 2 to 4, a plurality of R⁴⁰³ may be identical to or different from one another. When n is 2 to 4, a plurality of R⁴⁰⁴ may be identical to or different from one another.

At least one of the substituents on the aryl group or the heteroaryl group of R⁴⁰¹, R⁴⁰², R⁴⁰³, R⁴⁰⁴, and R⁴⁰⁵ bonds to L¹.

Particularly suitable groups represented by the formula (X2) are shown by the following formulas.

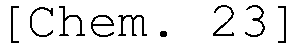

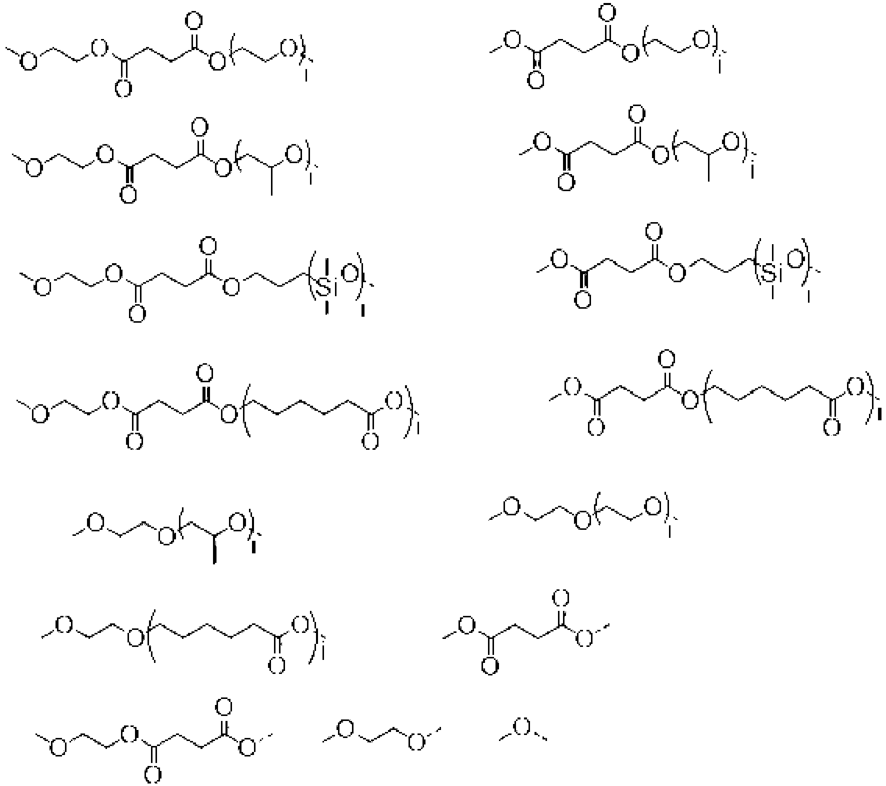

In the formula (X2), L is an oxygen atom or a sulfur atom.

R³⁰⁰ is an alkylene group, or a silylene group having an alkyl group or an aryl group as as a substituent. R³⁰⁰ is preferably an alkylene group having 1 or more and 6 or less carbon atoms, or a silylene group having an alkyl group having 1 or more and 6 or less carbon atoms as a substituent.

R³⁰², R³⁰³, and R³⁰⁴ are each independently an alkylene group. R³⁰² is preferably an alkylene group having 1 or more and 6 or less carbon atoms. R³⁰³ is preferably an alkylene group having 1 or more and 6 or less carbon atoms. R³⁰⁴ is preferably an alkylene group having 1 or more and 6 or less carbon atoms.

h, j, k and 1 are each independently an integer of 0 or 1.

i is an integer of 1 to 200. When multiple groups with a suffix i are present, the structures of the respective groups may be identical or different.
i is in the range of preferably 5 to 100, more preferably 8 to 75, and most preferably 10 to 70.

The dashed line in the formula (X2) is a bond to R⁴⁰⁰.

### <R⁵ and R⁶>

In the formula (1), R⁵ and R⁶ are each independently a hydrogen atom or a substituent. Preferably, R⁵ and R⁶ are each independently a substituted or unsubstituted aryl group or a substituted or unsubstituted heteroaryl group. These groups may be exemplified by the same groups as those mentioned with regard to R¹.

It is more preferable that R⁵ and R⁶ are each independently a substituted or unsubstituted phenyl group, a substituted or unsubstituted 1-naphthyl group, a substituted or unsubstituted 2-naphthyl group, a substituted or unsubstituted thienyl group, a substituted or unsubstituted furyl group, a substituted or unsubstituted pyrrolinyl group, a substituted or unsubstituted pyridyl group, a substituted or unsubstituted benzothienyl group, a substituted or unsubstituted benzofuranyl group, or a substituted or unsubstituted benzopyrrolinyl group. Furthermore, preferably, at least one of R⁵ and R⁶ is a substituted or unsubstituted phenyl group, and still more preferably, both of R⁵ and R⁶ are a substituted phenyl group.

The substituent included in the phenyl group is preferably the group represented by the formula (2a), a hydroxyl group, an alkyl group, a haloalkyl group, a substituted or unsubstituted cycloalkyl group, an alkoxy group, an amino group, a substituted amino group, a substituted or unsubstituted heterocyclic group, a cyano group, a halogen atom, an alkylthio group, a substituted or unsubstituted arylthio group, a nitro group, a formyl group, a hydroxycarbonyl group, an alkylcarbonyl group, an alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, an alkoxyalkylthio group, a haloalkylthio group, a substituted or unsubstituted cycloalkylthio group, a substituted or unsubstituted silyl group, a substituted or unsubstituted oxysilyl group, or the group represented by the formula (X3). The substituent is more preferably an alkyl group, a haloalkyl group, an alkoxy group, an amino group, a substituted amino group, a substituted or unsubstituted heterocyclic group, a cyano group, a halogen atom, an alkylthio group, a substituted or unsubstituted arylthio group, a substituted or unsubstituted aryloxy group, a substituted or unsubstituted aryl group, a substituted or unsubstituted heteroaryl group, an alkoxyalkylthio group, a haloalkylthio group, a substituted or unsubstituted silyl group, a substituted or unsubstituted oxysilyl group, or the group represented by the formula (X3).

### <R⁷ and R⁸>

In the formula (1), preferably, R⁵ and R⁶ are each independently a substituent. Preferably, R⁷ and R⁸ are each independently a hydroxyl group, a substituted or unsubstituted alkyl group, a haloalkyl group, a substituted or unsubstituted cycloalkyl group, a substituted or unsubstituted alkoxy group, a substituted or unsubstituted amino group, a substituted or unsubstituted heterocyclic group, a cyano group, a halogen atom, an alkylthio group, a substituted or unsubstituted arylthio group, a nitro group, a formyl group, a hydroxycarbonyl group, an alkylcarbonyl group, an alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, an alkoxyalkylthio group, a haloalkylthio group, a substituted or unsubstituted cycloalkylthio group, a substituted or unsubstituted silyl group, a substituted or unsubstituted oxysilyl group, the group represented by the formula (2a), a group represented by the following formula (X), or the group represented by the formula (X3). These groups may be exemplified by the same groups as those mentioned with regard to R¹ to R⁴.

b is an integer of 0 to 3.
c is an integer of 0 to 4. When b is 2 to 3, a plurality of R⁷ may be identical to or different from one another. When b is 2 to 3, and adjacent R⁷ are present, two adjacent R⁷ are optionally taken together with the carbon atoms bonded to R⁷ to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to the aliphatic ring, the aliphatic heterocyclic ring, the aromatic ring, or the aromatic heterocyclic ring. The combination of adjacent R⁷ is a combination of R⁷ at the positions 9 and 10 of the chromene compound.

When c is 2 to 4, a plurality of R⁸ may be identical to or different from one another. When c is 2 to 4, and adjacent R⁸ are present, two adjacent R⁸ are optionally taken together with the carbon atoms bonded to R⁸ to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to the aliphatic ring, the aliphatic heterocyclic ring, the aromatic ring, or the aromatic heterocyclic ring. The combination of adjacent R⁸ is a combination of R⁸ at the positions 5 and 6, 6 and 7, or 7 and 8 of the chromene compound.

A ring having 5 or more and 8 or less atoms including the carbon atom to which R⁷ or R⁸ bonds may be formed. Further, the ring may have a substituent, and examples of the substituent include a substituent selected from a hydroxyl group, an alkyl group having 1 or more and 6 or less carbon atoms, a haloalkyl group having 1 or more and 6 or less carbon atoms, a cycloalkyl group having 3 or more and 8 or less carbon atoms, an alkoxy group having 1 or more and 6 or less carbon atoms, an amino group, a substituted amino group, a heterocyclic group having 3 or more and 8 or less atoms, a cyano group, a nitro group, and a halogen atom. Specific examples of these substituents will be described later.

A suitable ring formed by R⁷ or R⁸ includes a ring represented by the following formula (X5).

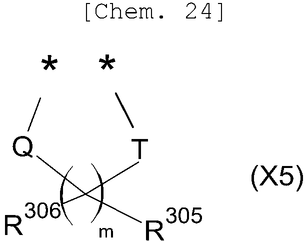

In the formula (X5), Q and T are each independently a sulfur atom, a substituted or unsubstituted methylene group, an oxygen atom, or a group represented by NR³⁰⁷. R³⁰⁷ is a hydrogen atom, a hydroxyl group, an alkyl group, a haloalkyl group, a cycloalkyl group, an alkoxy group, a substituted or unsubstituted aryl group, a substituted or unsubstituted heteroaryl group, or the group represented by the formula (2a).

Preferably, R³⁰⁵ and R³⁰⁶ are each independently a hydroxy group, an alkyl group, a haloalkyl group, a cycloalkyl group, an alkoxy group, an amino group, a substituted amino group, a substituted or unsubstituted heterocyclic group, a cyano group, a nitro group, a formyl group, a hydroxycarbonyl group, an alkylcarbonyl group, an alkoxycarbonyl group, a halogen atom, a substituted or unsubstituted aralkyl group, a substituted or unsubstituted aralkoxy group, a substituted or unsubstituted aryl group, a thiol group, an alkylthio group, an alkoxyalkylthio group, a haloalkylthio group, a cycloalkylthio group, or a substituted or unsubstituted arylthio group.

Alternatively, R³⁰⁵ and R³⁰⁶ are optionally taken together with the carbon atom(s) to which they bind to form a substituted or unsubstituted aliphatic ring. Specific examples of the aliphatic ring include a cyclopentane ring, a cyclohexane ring, and the like. In addition, in the aliphatic ring, 1 to 8 hydrogen atoms, and particularly preferably 1 to 4 hydrogen atoms may be substituted with at least one group selected from the group consisting of a hydroxyl group, an alkyl group, a haloalkyl group, a cycloalkyl group, an alkoxy group, an amino group, a substituted amino group, a heterocyclic group, a cyano group, a nitro group, and a halogen atom. Specific examples of these substituents will be described later.

In the formula, m is an integer of 1 to 4.

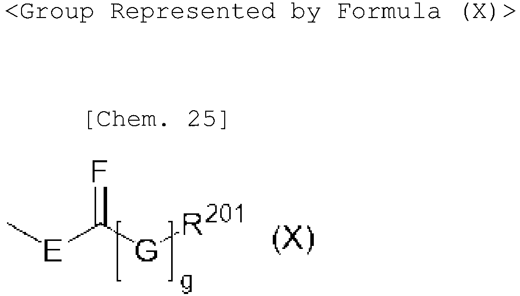

In the formula (X), E is an oxygen atom or NR¹⁰¹. R¹⁰¹ is a hydrogen atom or an alkyl group. Preferably, E is NR¹⁰¹, and R¹⁰¹ is a hydrogen atom or an alkyl group having 1 to 6 carbon atoms.

F is an oxygen atom or a sulfur atom. F is preferably an oxygen atom.

G is an oxygen atom, a sulfur atom, or NR²⁰². R²⁰² is a hydrogen atom, an alkyl group, a cycloalkyl group, an aryl group, or a heteroaryl group. G is preferably NH.

g is 0 or 1.

R²⁰¹ is a hydrogen atom, an alkyl group, a cycloalkyl group, an aryl group, or a heteroaryl group. When G is an oxygen atom or a sulfur atom, R²⁰¹ is a group other than a hydrogen atom. R²⁰¹ is preferably an alkyl group having 1 or more and 6 or less carbon atoms, an alkoxy group having 1 or more and 6 or less carbon atoms, or an aryl group having 6 or more and 12 or less carbon atoms.

Suitable groups represented by the formula (X) are as follows.

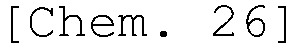

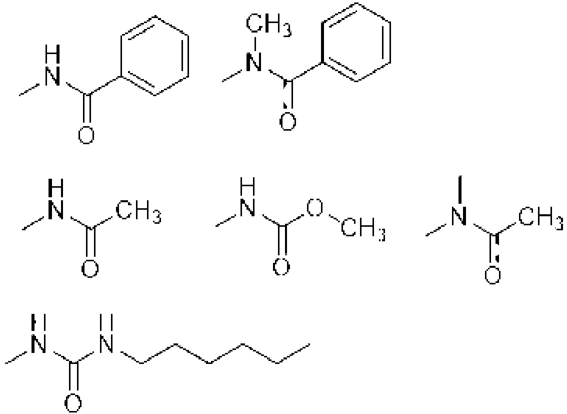

### <Skeleton Represented by Formula (2)>

The photochromic compound according to the embodiment preferably has a naphthopyran skeleton represented by the following formula (2). In the following formula (2), Z¹, R³, R⁴, and M are each as defined in the formula (1).

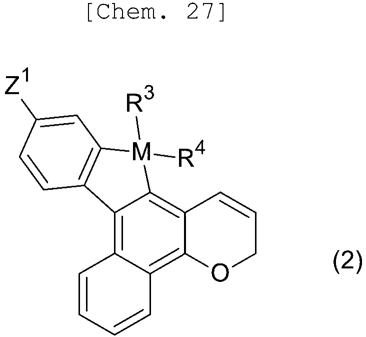

### <Compound Represented by Formula (3)>

The photochromic compound according to the embodiment is preferably a compound represented by the following formula (3). In the following formula (3), Z¹, R³, R⁴, R⁵, R⁶, R⁷, R⁸, b, a, and M are each as defined in the formula (1).

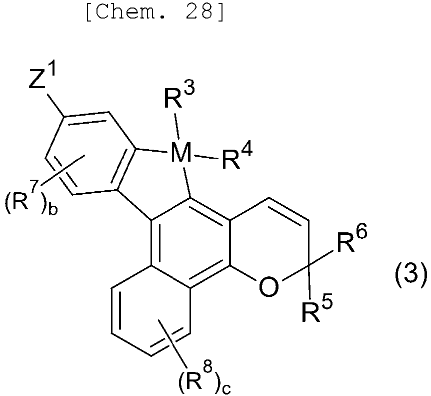

### <Photochromic Compound Represented by Formula (4)>

Particularly suitable chromene compounds include the compounds represented by the following formula (4).

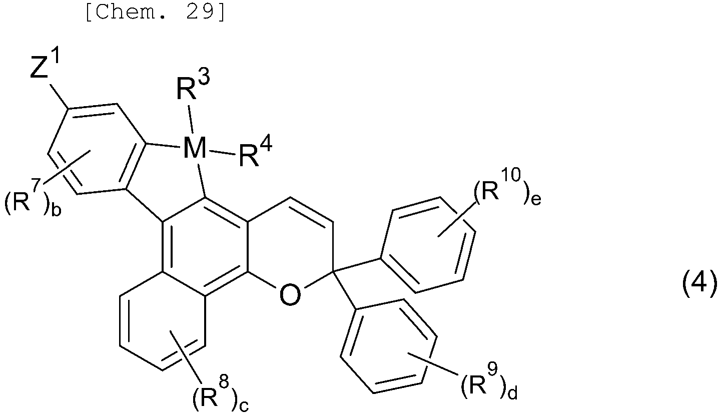

In the formula (4), R³, R⁴, R⁷, R⁸, Z¹, M, b, and c are each independently as defined in the formula (3).

### <R⁹ and R¹⁰>

R⁹ and R¹⁰ are each independently a hydroxyl group, an alkyl group having 1 or more and 6 or less carbon atoms, a haloalkyl group having 1 or more and 6 or less carbon atoms, a cycloalkyl group having 3 or more and 8 or less carbon atoms, an alkoxy group having 1 or more and 6 or less carbon atoms, a substituted or unsubstituted amino group, a heterocyclic group, a cyano group, a halogen atom, an alkylthio group having 1 or more and 6 or less carbon atoms, a substituted or unsubstituted arylthio group, a substituted or unsubstituted aryloxy group, a substituted or unsubstituted aryl group, a substituted or unsubstituted heteroaryl group, a nitro group, a substituted or unsubstituted silyl group, a substituted or unsubstituted oxysilyl group, the group represented by the formula (2a), or the group represented by (X3).

d denotes the number of R⁹, and is an integer of 0 to 5. When d is 2 or more, a plurality of R⁹ may be identical to or different from one another.

When d is 2 to 5, and adjacent R⁹ are present, two adjacent R⁹ are optionally taken together with the carbon atoms bonded to R⁹ to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to the aliphatic ring, the aliphatic heterocyclic ring, the aromatic ring, or the aromatic heterocyclic ring.

e denotes the number of R¹⁰, and is an integer of 0 to 5. When e is 2 or more, a plurality of R¹⁰ may be identical to or different from one another.

When e is 2 to 5, and adjacent R¹⁰ are present, two adjacent R¹⁰ are optionally taken together with the carbon atoms bonded to R¹⁰ to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to the aliphatic ring, the aliphatic heterocyclic ring, the aromatic ring, or the aromatic heterocyclic ring.

R⁹ and R¹⁰ may each independently form a cyclic group which may include at least one selected from the group consisting of an oxygen atom, a sulfur atom, a carbon atom, and a nitrogen atom as a result of two adjacent R⁹ or two adjacent R¹⁰ being combined with each other. The cyclic group is not limited, and is preferably a ring having 5 or more and 8 or less atoms including the carbon atom to which R⁹ or R¹⁰ bonds. Further, the ring may also have a substituent. Examples of the substituent include a substituent selected from a hydroxyl group, an alkyl group having 1 or more and 6 or less carbon atoms, a haloalkyl group having 1 or more and 6 or less carbon atoms, a cycloalkyl group having 3 or more and 8 or less carbon atoms, an alkoxy group having 1 or more and 6 or less carbon atoms, an amino group, a substituted amino group, a heterocyclic group having 3 or more and 8 or less atoms, a cyano group, a nitro group, and a halogen atom. Specific examples of these substituents include the same groups as those described with regard to the groups exemplified in R¹ to R⁴. It is preferrable that R⁹ and R¹⁰, taken together, form a ring represented by the formula (X5).

### <Detailed Description of Substituent, etc.>

The substituents optionally included in the groups of R¹ to R¹⁰ described above include a hydroxyl group, a cyano group, a halogen atom, a nitro group, a formyl group, a hydroxycarbonyl group, a thiol group, the group represented by the formula (2a), the group represented by the formula (X), the group represented by the formula (X3), an alkyl group, a haloalkyl group, a cycloalkyl group, an alkoxy group, an amino group, a substituted amino group, a heterocyclic group, a halogen atom, an alkylthio group, an arylthio group, an alkylcarbonyl group, an alkoxycarbonyl group, aralkyl, an aralkoxy group, an aryloxy group, an aryl group, a heteroaryl group, an alkoxyalkylthio group, a haloalkylthio group, a cycloalkylthio group, a silyl group, or an oxysilyl group. These groups may be the same as described in detail in R¹ to R⁴.

### <Specific Examples of Suitable Photochromic Compound>

Specific examples of a particularly suitable photochromic compound include photochromic compounds represented by the following formulas.

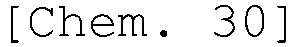

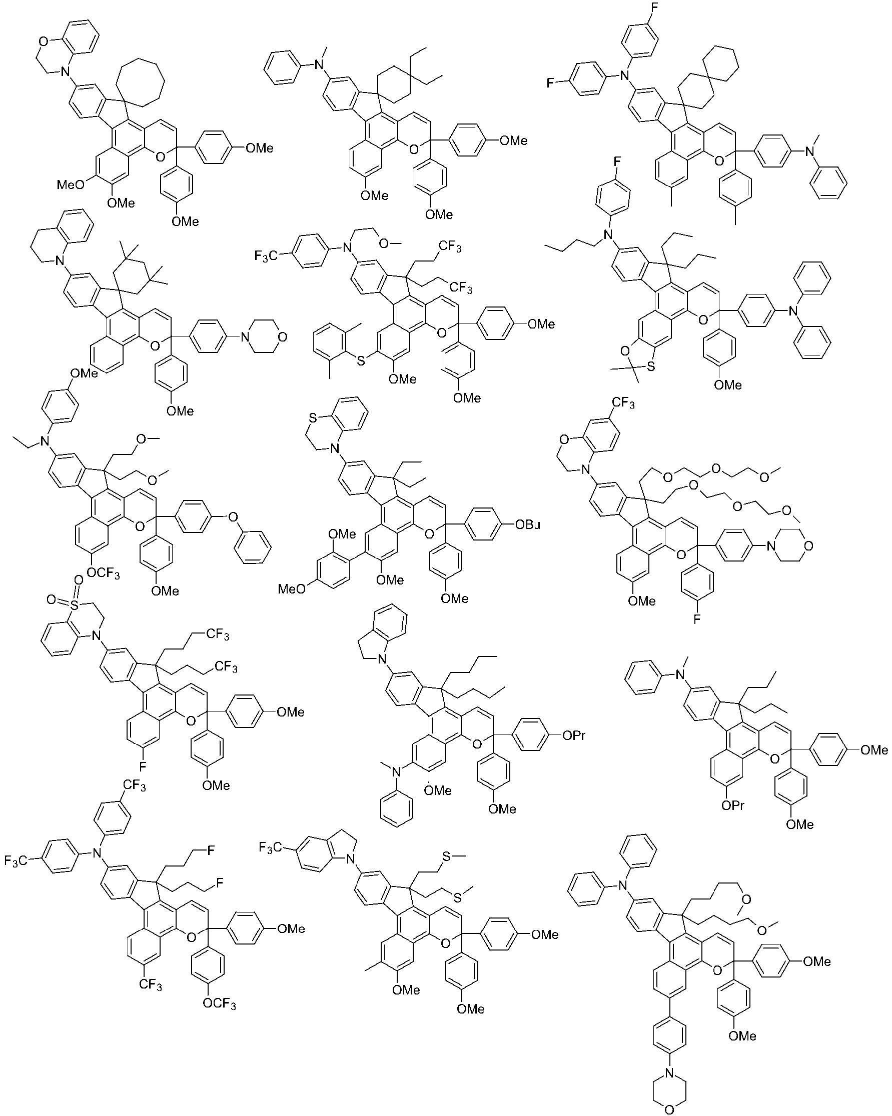

### [Phenol Derivative]

The phenol derivative according to the embodiment is represented by the following formula (5). The phenol derivative may be used as an intermediate for synthesizing the photochromic compound represented by the above formula (1).

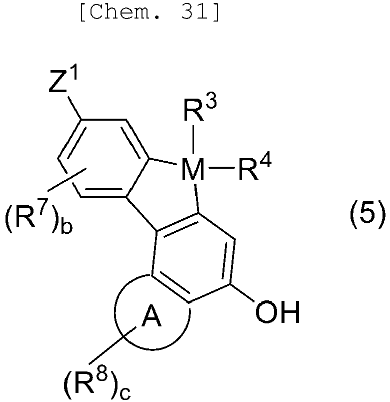

In the formula (5), M, R³, R⁴, R⁷, R⁸, Z¹, the ring A, b, and c are each independently as defined in the above formula (1).

### <Phenol Derivative Represented by Formula (6)>

The phenol derivative according to the embodiment may have a skeleton represented by the following formula (6). The phenol derivative may be used as an intermediate for synthesizing the photochromic compound having a skeleton represented by the above formula (2).

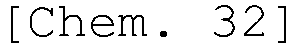

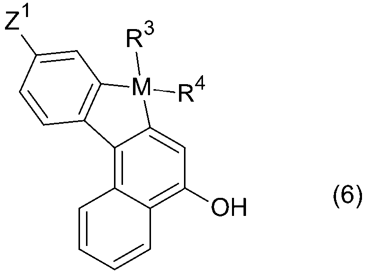

In the formula (6), M, R³, R⁴, and Z¹ are each independently as defined in the above formula (2).

### <Phenol Derivative Represented by Formula (7)>

The phenol derivative according to the embodiment is represented by the following formula (7). The phenol derivative may be used as an intermediate for synthesizing the photochromic compound represented by the above formula (3), and the photochromic compound represented by the above formula (4).

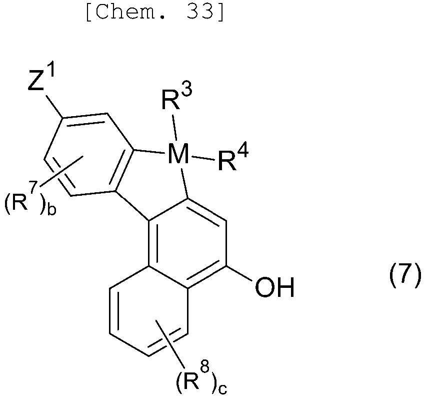

In the formula (7), M, R³, R⁴, R⁷, R⁸, Z¹, b, and c are each independently as defined in the above formula (4).

### <Specific Examples of Phenol Derivative>

Specific examples of the phenol derivative according to the embodiment include include compounds represented by the following formulas.

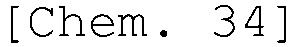

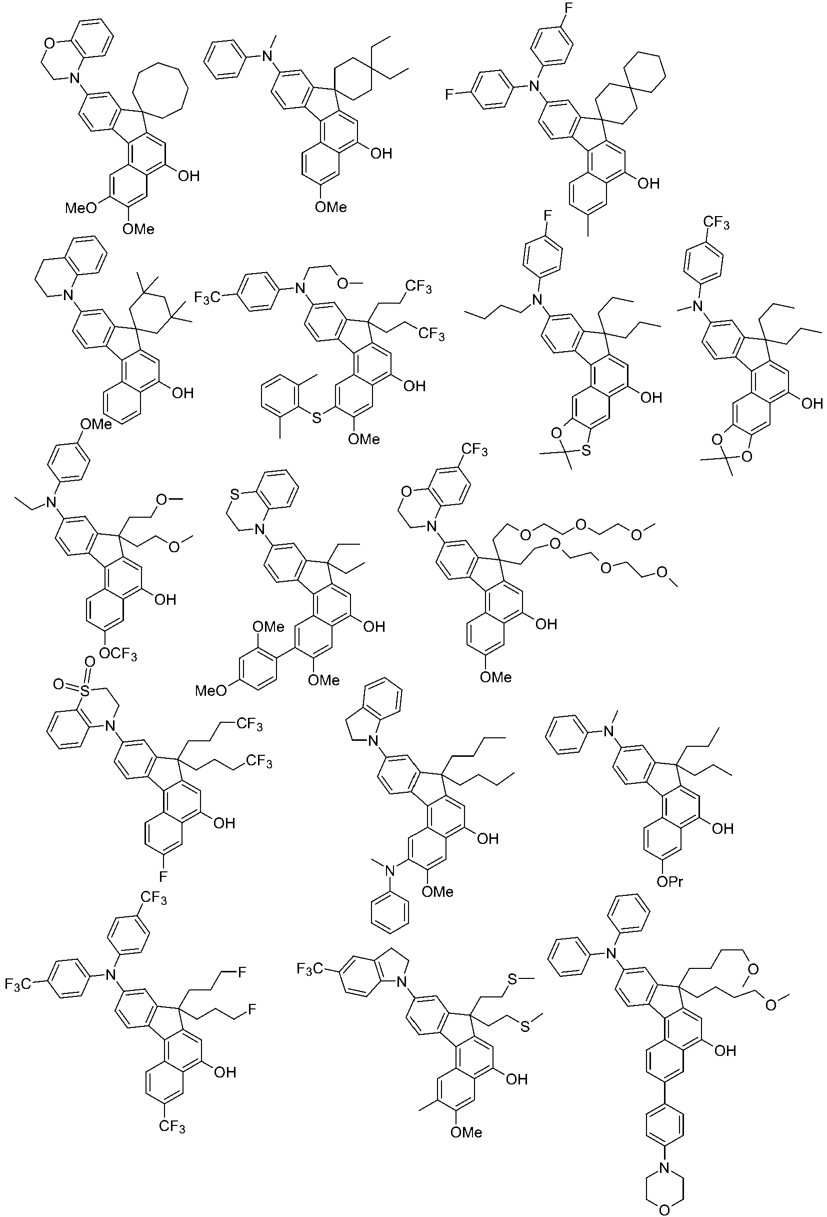

### [Method for Producing Photochromic Compound]

The photochromic compound according to the embodiment may be produced according to any synthesis method. A representative example of the method for producing a photochromic compound will be described, but the synthesis of the photochromic compounds according to the embodiment is not limited to such a method. Note that in the following description, symbols in each formula have the same meaning as defined in the formulas described above, unless otherwise specified.

The production of the photochromic compound can be achieved by a method that involves reacting phenol derivative represented by the above formula (7) with a propargyl alcohol compound represented by the following formula (8) in the presence of an acid catalyst.

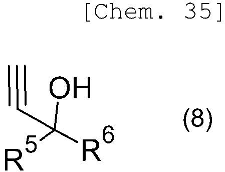

The stoichiometric ratio of the phenol derivative to the propargyl alcohol compound is preferably selected from the range of 1:10 to 10:1 (mole ratio). Examples of the acid catalyst include sulfuric acid, benzenesulfonic acid, p-toluenesulfonic acid, acidic alumina, etc. The acid catalyst is used in an amount in the range of preferably 0.1 to 10 parts by weight per 100 parts by weight of the total of the phenol derivative and the propargyl alcohol compound. The reaction temperature is preferably 0 to 200°C. Preferably, an aprotic organic solvent, for example, N-methylpyrrolidone, dimethylformamide, tetrahydrofuran, benzene, toluene, is used as a solvent. The purification method for the resulting product in the reaction is not limited. For example, the purification of the product may be carried out by silica gel column purification, and additional recrystallization.

### <Method for Synthesizing Phenol Derivative>

The phenol derivative may be synthesized according to any known method. The method for synthesizing the phenol derivative represented by the formula (7) is not limited, and, for example, when M is the carbon atom, the phenol derivative can be synthesized as follows.

First, a benzene compound represented by the following formula (9) is reacted with an acid chloride compound represented by the following formula (10), to obtain a benzophenone compound represented by the following formula (11). Incidentally, in the formula (11), Z¹, R⁷, R⁸, b, and c are as defined in the formula (3).

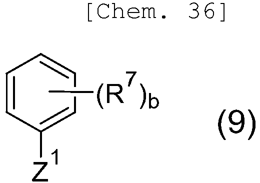

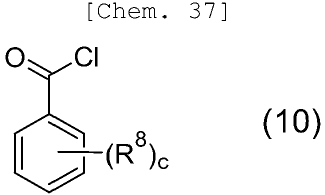

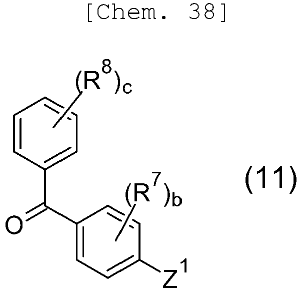

Note that the substituent(s) of the acid chloride compound and the substituent(s) of the benzene compound may be interchanged depending on the structure and/or the substituent of the photochromic compound desired to be synthesized. In other words, an acid chloride compound having Z¹ and R⁷ may be reacted with a benzene compound having R⁸.

Further, a benzophenone compound (11) is subjected to the Stobbe reaction, a cyclization reaction, a hydrolysis reaction using an alkali or an acid, benzyl protection, or debenzylation via a hydrolysis reaction using an alkali or an acid, to obtain a carboxylic acid with a hydroxyl group being protected by benzyl (Bn), represented by the following formula (12). Then, the benzyl-protected carboxylic acid represented by the formula (12) is converted to an amine via a method such as the Curtius rearrangement, the Hofmann rearrangement, and the Lossen rearrangement, and a diazonium salt is prepared from the amine. The diazonium salt is converted to a halide such as a bromide or an iodide via the Sandmeyer reaction, or the like, to obtain a halide represented by the following formula (13) (wherein Hal represents a halogen).

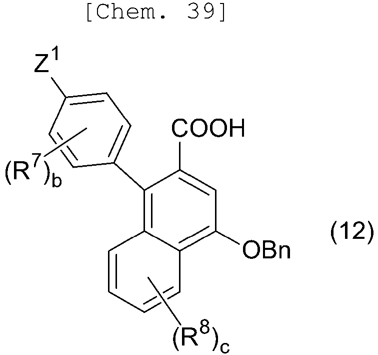

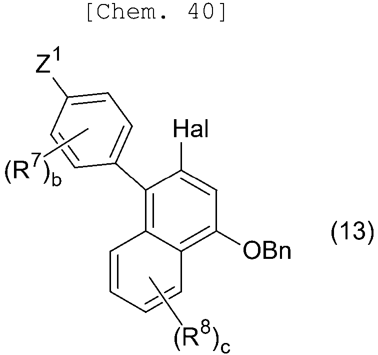

The resulting halide is reacted with magnesium, lithium, or the like to prepare an organometallic reagent. The organometallic reagent is reacted with a ketone represented by the following formula (14) (wherein R³ and R⁴ are as defined in the formula (3)) in an organic solvent at -100 to 70°C, to obtain a compound represented by the following formula (15).

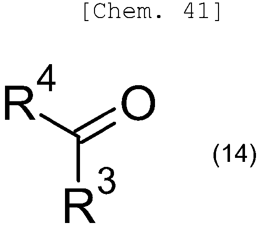

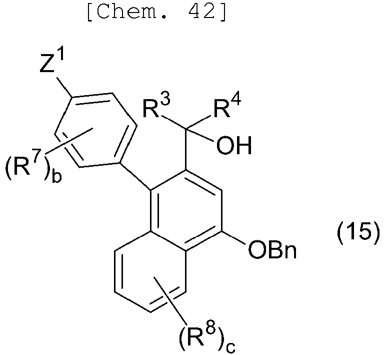

The resulting compound represented by the formula (15) is debenzylated, followed by a reaction under neutral to acidic conditions at 10 to 120°C for 10 minutes to 2 hours, to subject the alcohol to spirocyclization, whereby the desired phenol derivative according to the formula (7) can be synthesized. In such reactions, a wide range of stoichiometric ratio of the organometallic reagent to the ketone represented by the formula (14) is employed, but the stoichiometric ratio is preferably selected from the range of 1:10 to 10:1 (mole ratio). The reaction temperature is preferably -100 to 70°C. An aprotic organic solvent, for example, diethyl ether, tetrahydrofuran, benzene, or toluene is preferably used as a solvent. The spirocyclization of the alcohol product under neutral to acidic conditions is preferably carried out in the presence of an acid catalyst. Examples of the acid catalyst used include acetic acid, hydrochloric acid, sulfuric acid, benzenesulfonic acid, p-toluenesulfonic acid, and acidic alumina. Such an acid catalyst is suitably used in an amount in the range of 0.1 to 10 parts by weight per 100 parts by weight of the alcohol product. The spirocyclization is preferably carried out in the presence of a solvent such as tetrahydrofuran, benzene, or toluene.

The method above shows an example of a method that employs a starting material having the substituent Z¹, but the substituent Z¹ can also be introduced by, for example, synthesizing a precursor having a halogen atom, and then using the Buchwald reaction thereof with an amine of the Z¹-H structure in the presence of a Pd catalyst, or the like.

The reaction for introducing the substituent Z¹ is not limited, and for example, the substituent Z¹ may be introduced after the synthesis of a photochromic compound having a halogen atom. An example of the alternative pathway will be shown in the following.

First, the reaction is carried out in the same manner as the above example using a compound having a halogen atom in place of the substituent Z¹, then the benzophenone compound having a halogen atom in place of Z¹ is used to carry out the Stobbe reaction, the cyclization reaction, and the hydrolysis reaction using an alkali or an acid, thereby to obtain a carboxylic acid compound represented by the following formula (16), and the carboxylic acid compound represented by formula (16) is subjected to the spirocyclization under neutral to acidic conditions, and a reduction reaction using hydrogen, hydrazine, or the like, to obtain a phenol derivative represented by the following formula (17).

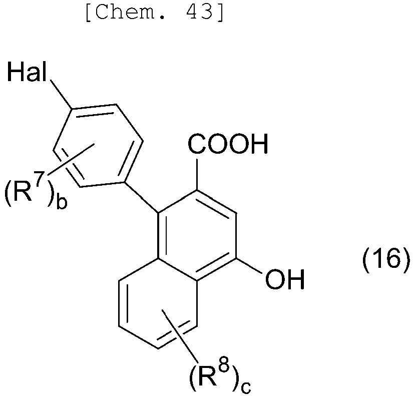

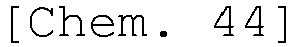

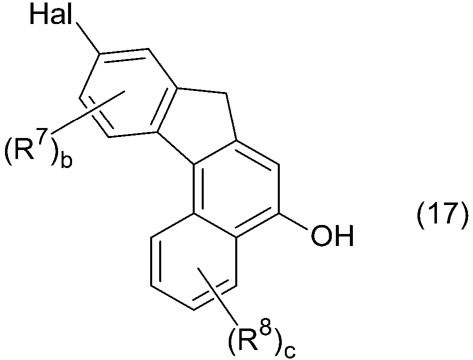

In addition to the method described above, the compound represented by the formula (17) can also be synthesized by the intramolecular cyclization of a halogenated compound represented by the formula (13) in the presence of a palladium catalyst.

The resulting phenol derivative is used to obtain a photochromic compound represented by the following formula (18).

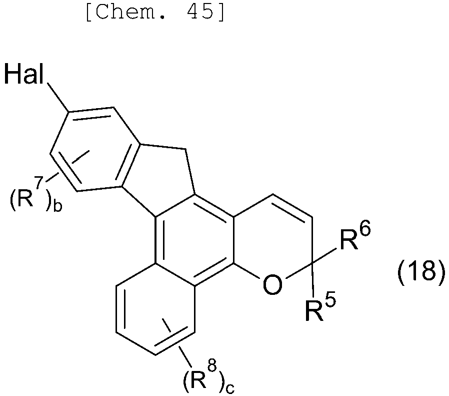

The resulting photochromic compound is reacted with a halide of R³ under basic conditions, to obtain a photochromic compound precursor represented by the following formula (19).

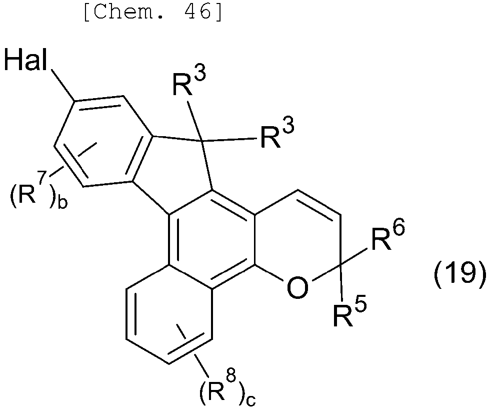

The Buchwald reaction between the halogen atom of the resulting precursor and an amine of the Z¹-H structure in the presence of a Pd catalyst, and the like can be used to obtain the photochromic compound according to the present invention.

### <Method for Synthesizing Si- or Ge-Containing Phenol Derivative>

In the following, an example of the method for producing of a phenol derivative represented by the formula (7) in which M is Si or Ge will be described.

First, a halide represented by the formula (13) is reacted with magnesium, lithium, or the like to prepare an organometallic reagent. The organometallic reagent is reacted with a monohalide represented by the following formula (20) (wherein R³ and R⁴ are as defined in the formula (7)) in an organic solvent at -100 to 70°C, to obtain a compound represented by the following formula (21).

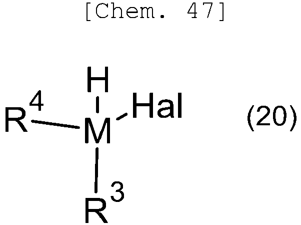

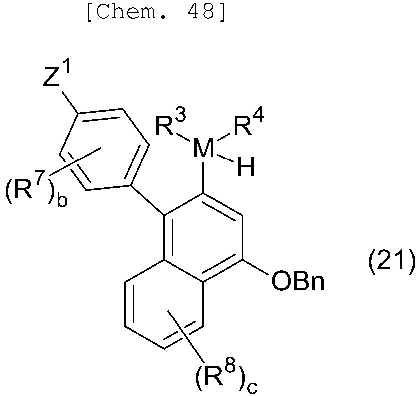

Any known method using the resulting compound represented by the formula (21) yields a cyclization product represented by the following formula (22).

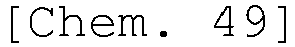

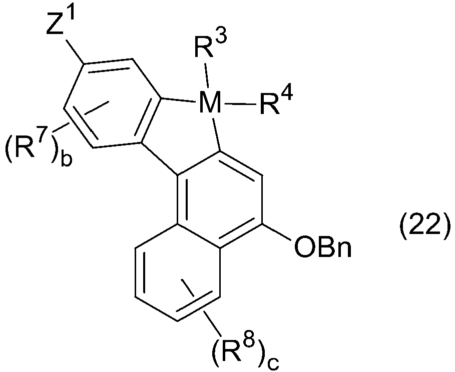

The debenzylation of the resulting cyclization product can yield the phenol derivative according to the formula (7).

### <Identification of Photochromic Compound>

The photochromic compound according to the embodiment is a solid or viscous liquid, for example, at ambient temperature and pressure. A separation operation involving thin layer chromatography, silica gel column chromatography, high performance liquid chromatography, gas chromatography or the like on the solid or liquid allows the photochromic compound to be isolated. Further, the absence of the starting compounds and by-products such as colored matter other than the photochromic compound should be confirmed.

In the measurement of the resulting photochromic compound by proton nuclear magnetic resonance spectrum (¹H-NMR), peaks assigned to the aromatic protons and alkene protons appear at around δ: 5.0 to 9.0 ppm, and peaks assigned to protons of alkyl groups and alkylene groups appear at around δ: 1.0 to 4.0 ppm. Further, the number of protons of the respective bonded groups can be determined by the relative comparison of spectral intensities of the respective peaks. This in turn enables the identification of the skeleton and substituents of the photochromic compound, etc.

In addition, when the photochromic compound is contained in a cured product of a resin or the like, the photochromic compound can be isolated by dissolving the resin and using the separation means mentioned above.

### <Photochromic Composition>

The photochromic compound according to the embodiment can be dissolved in a common organic solvent such as toluene, chloroform, or tetrahydrofuran. When the photochromic compound represented by the formula (1) is dissolved in such a solvent, a clear and colorless solution is obtained. This solution exhibits good photochromic behavior, that is, when the solution is irradiated with sunlight or ultraviolet light, it quickly develops a color and when the sunlight or the like is blocked, it quickly reversibly returns to its original colorless state.

The photochromic compound according to the embodiment can be used in combination with a photochromic compound having another structure, depending on the intended use. For example, it can be used in combination with another photochromic compound in order to obtain various color tones required for a photochromic lens. A photochromic compound to be combined may be any known compound without any limitation. Examples thereof include indenonaphthopyrans, naphthopyrans, spirooxazines, spiropyrans, fulgides, fulgimides, diarylethenes, and the like. Among them, indenonaphthopyran compounds are particularly preferable since they can maintain a uniform color tone upon color development and fading, can suppress a color shift upon color development associated with degradation of a photochromic property, and can reduce initial coloration. It is preferable to use multiple photochromic compounds according to the embodiment to adjust a color tone since such a combination especially can have both a color developing density at higher temperatures and a fast color fading rate, and further have excellent durability.

When a photochromic composition including the photochromic compound according to the embodiment and another photochromic compound is produced, a blending ratio of the photochromic compounds is appropriately determined in accordance with a desired color tone.

### <Photochromic Curable Composition>

The curable composition according to the embodiment contains the photochromic compound according to the embodiment, and at least one selected from the group consisting of a radically-polymerizable monomer, a cationically-polymerizable monomer, a compound having a polymerization-reactive group, and a (thio)urethane(urea) polymer. Herein, the (thio) urethane (urea) polymer includes at least one selected from the group consisting of a urethane polymer, a thiourethane polymer, a urethaneurea polymer, and a thiourethaneurea polymer.

The photochromic compound and photochromic composition according to the embodiment is preferably used in combination with a polymerizable compound as a photochromic curable composition.

For the photochromic curable composition, the photochromic compound (or photochromic composition) is preferably used in an amount of 0.001 to 10 parts by mass relative to 100 parts by mass of the polymerizable compound, although it all depends on a color developing intensity of a photochromic compound, a lens material selected, and a thickness of a lens. An optimal amount to be incorporated in terms of the amount of the photochromic compound mentioned above depends on application for which the photochromic curable composition is used. For example, the case where the photochromic curable composition is used as a thin-film optical article or as a thick-film optical article will be described below.

### (Use as Thin-Film Optical Article)

For example, when a thin film of 10 µm or more and less than 1000 µm, e.g., about 100 µm (polymer film made by polymerization of the photochromic curable composition) is formed from the photochromic curable composition, 0.001 to 10 parts by mass of the photochromic compound (or photochromic composition) according to the above embodiment relative to 100 parts by mass of another polymerizable monomer may be used in a blend to adjust a color tone.

### (Use as Thick Film Optical Article)

In the case of a thick cured product (polymeric molded product made by polymerization of the photochromic curable composition), e.g., a cured product having a thickness of 1 mm or more, 0.001 to 1 part by mass of the photochromic compound (or photochromic composition) according to the above embodiment relative to 100 parts by mass of the thick cured product or another polymerizable monomer that gives the thick cured product may be used in a blend to adjust a color tone.

### <Polymerizable Compound>

As described above, the photochromic compound is preferably used in combination with a polymerizable compound as a photochromic curable composition. The polymerizable compound can be exemplified by a urethane- or urea-forming polymerizable compound capable of forming a urethane bond or a urea bond, etc., a compound having a polymerization-reactive group, a radically-polymerizable compound, an epoxy-based polymerizable compound, and the like. These polymerizable compounds are not limited, and for example, the polymerizable compounds described in Patent Document 7 can be suitably used.

Of these, especially, compounds having a polymerization-reactive group as shown below are suitably used.

### <Compound Having Polymerization-Reactive Group>

Examples of the compound having a polymerization-reactive group include a compound having an iso(thio)cyanate group (iso(thio)cyanate compound). The iso(thio)cyanate compound is a compound having an isocyanate group or an isothiocyanate group, and may have both an isocyanate group and an isothiocyanate group. The compound is suitably used in combination with a compound having active hydrogen, as described later. Examples of such an iso(thio)cyanate compound include, but are not limited to, the following compounds.

### (Polyiso(thio) cyanate)

The polyiso(thio)cyanate is a compound having at least 2 or more iso(thio)cyanate groups within a single molecule. Examples of the polyiso(thio)cyanate include aromatic polyiso(thio)cyanates having an aromatic ring such as m-xylene diisocyanate and 4,4'-diphenylmethane diisocyanate, and aliphatic polyiso(thio)cyanates such as norbornane diisocyanate and dicyclohexylmethane-4,4'-diisocyanate.

### (Compound Having Active Hydrogen)

The compound having active hydrogen is preferably, but not limited to, a compound having a hydroxyl group and/or thiol group, and particularly preferably a polyfunctional compound having 2 or more active hydrogens within a single molecule. Specific examples of compound having active hydrogen include polyfunctional thiol compounds such as pentaerythritol tetrakis(3-mercaptopropionate) and 4-mercaptomethyl-3,6-dithia-octanedithiol; polyfunctional alcohols such as trimethylolpropane and pentaerythritol.

### (Radically-Polymerizable Compound)

The radically-polymerizable compound includes a polyfunctional radically-polymerizable compound and a monofunctional radically-polymerizable compound. Each of these compounds may be used alone, or these compounds may be used in combination. Examples of the radically-polymerizable substituent includes groups with an unsaturated double bond, i.e., a vinyl group (including a styryl group, a (meth)acryl group, and an allyl group, etc.).

The polyfunctional radically-polymerizable compound is a compound having 2 or more radically-polymerizable substituents in the molecule. The polyfunctional radically-polymerizable compound includes a first polyfunctional radically-polymerizable compound having 2 to 10 radically-polymerizable substituents, and a second polyfunctional radically-polymerizable compound having more than 10 radically-polymerizable substituents.

The first polyfunctional radically-polymerizable compound is not limited, but more preferably has 2 to 6 radically-polymerizable substituents. Specific examples of the compound are as follows.

(Polyfunctional (Meth)Acrylic Acid Ester Compound) Ethylene glycol di(meth)acrylate, diethylene glycol di(meth)acrylate, triethylene glycol di(meth)acrylate, tetraethylene glycol di(meth)acrylate, ethylene glycol bisglycidyl (meth)acrylate, bisphenol A di(meth)acrylate, 2,2-bis(4-(meth)acryloyloxyethoxyphenyl)propane, 2,2-bis(3,5-dibromo-4-(meth)acryloyl, oxyethoxyphenyl)propane.

### (Polyfunctional Allyl Compound)

Diallyl phthalate, diallyl terephthalate, diallyl isophthalate, diallyl tartrate, diallyl epoxysuccinate, diallyl fumarate, diallyl chlorendate, diallyl hexaphthalate, diallyl carbonate, allyl diglycol carbonate, trimethylolpropane triallyl carbonate.

### (Polyfunctional Thio(Meth)Acrylic Acid Ester Compound)

1,2-bis(methacryloylthio)ethane, bis(2-acryloylthioethyl) ether, 1,4-bis(methacryloylthiomethyl)benzene.

### (Radically-Polymerizable Compound)

Examples of the second polyfunctional radically-polymerizable compound having more than 10 radically-polymerizable substituents include compounds having a relatively higher molecular weight such as a silsesquioxane compound having radically-polymerizable substituents, a polyrotaxane compound having radically-polymerizable substituents, etc.

The monofunctional radically-polymerizable compound is a compound having one radically-polymerizable substituent in the molecule, and specific examples thereof include, but are not limited to, the following compounds.

### (Unsaturated Carboxylic Acid)

Acrylic acid, methacrylic acid, maleic anhydride.

### ((Meth)Acrylic Acid Ester)

Methyl (meth)acrylate, benzyl methacrylate, phenyl methacrylate.

2-Hydroxyethyl methacrylate, glycidyl (meth)acrylate, β-methylglycidyl (meth)acrylate, bisphenol A-monoglycidyl ether methacrylate, 4-glycidyloxy methacrylate, 3-(glycidyl-2-oxyethoxy)-2-hydroxypropyl methacrylate, 3-(glycidyloxy-1-isopropyloxy)-2-hydroxypropyl acrylate, 3-glycidyloxy-2-hydroxypropyloxy)-2-hydroxypropyl acrylate.

### (Fumaric Acid Ester)

Diethyl fumarate, diphenyl fumarate.

### (Thio(Meth)Acrylic Acid)

Methyl thioacrylate, benzyl thioacrylate, benzyl thiomethacrylate.

### (Vinyl Compound)

Divinylbenzene, styrene, chlorostyrene, thylstyrene, vinylnaphthalene, α-methylstyrene dimer, bromostyrene.

The radically-polymerizable compound may be used alone or a mixture of multiple types of them may also be used. In this case, preferably 80 to 100 parts by mass of the polyfunctional radically-polymerizable compound and 0 to 20 parts by mass of the monofunctional radically-polymerizable compound, and more preferably 90 to 100 parts by mass of the polyfunctional radically-polymerizable compound and 0 to 10 parts by mass of the monofunctional radically-polymerizable compound are used per 100 parts by mass of a total of the radically-polymerizable compounds. Furthermore, preferably 80 to 100 parts by mass of the first polyfunctional radically-polymerizable compound, 0 to 20 parts by mass of the second radically-polymerizable compound, and 0 to 20 parts by mass of the monofunctional radically-polymerizable compound, and more preferably 85 to 100 parts by mass of the first polyfunctional radically-polymerizable compound, 0 to 10 parts by mass of the second polyfunctional radically-polymerizable compound, and 0 to 10 parts by mass of the monofunctional radically-polymerizable compound are used per 100 parts by mass of a total of the radically-polymerizable compounds.

### (Various Compounding Agent)

Various known compounding agents may be incorporated into the curable composition as long as the effect is not impaired. Examples of the compounding agent include a releasing agent, a UV absorber, an IR absorber, a UV stabilizer, an antioxidant, an anti-coloring agent, an antistatic agent, a fluorescent dye, a dye, a pigment, a perfume, and various other stabilizers. A solvent or a leveling agent may also be incorporated. A thiol such as t-dodecyl mercaptan may also be incorporated as a polymerization regulator.

Among the above-mentioned compounding agents, the UV stabilizer is suitably used from the viewpoint of improving durability of a photochromic moiety. A hindered amine light stabilizer, a hindered phenol antioxidant, a sulfur antioxidant, and the like are known as such a UV stabilizer. A particularly suitable UV stabilizer is as follows.

Bis(1,2,2,6,6-pentamethyl-4-piperidyl) sebacate; ADK STAB LA-52, LA-57, LA-62, LA-63, LA-67, LA-77, LA-82, LA-87 (manufactured by ADEKA CORPORATION); 2,6-di-tert-butyl-4-methyl-phenol, ethylenebis(oxyethylene)bis[3-(5-tert-butyl-4-hydroxy-m-tolyl)propionate]; IRGANOX 1010, 1035, 1075, 1098, 1135, 1141, 1222, 1330, 1425, 1520, 259, 3114, 3790, 5057, 565 (manufactured by BASF Japan Ltd.). An amount of such a UV stabilizer to be used is not limited as long as the effect is impaired, and is usually in the range of 0.001 to 10 parts by mass, especially 0.01 to 1 part by mass per 100 parts by mass of the photochromic curable composition.

A UV absorber may also be used in addition to the UV stabilizer. Known UV absorbers such as a benzophenone compound, a benzotriazole compound, a cyanoacrylate compound, a triazine compound, or a benzoate compound can be used as the UV absorber, with a cyanoacrylate compound or a benzophenone compound being particularly preferred. The above-mentioned UV stabilizer is preferably used in the range of 0.001 to 5 parts by mass relative to 100 parts by mass of a photochromic curable composition containing the photochromic compound and the polymerizable compound.

### <Method for Using Photochromic Curable Composition; Optical Article>

A photochromic curable composition is cured to obtain a photochromic cured product. Polymerization curing for producing the photochromic cured product is performed by radical polymerization, ring-opening polymerization, anionic polymerization, or condensation polymerization using irradiation with an active energy ray such as an ultraviolet ray, an α-ray, a β-ray, or a γ-ray; heat; or a combination thereof. In other words, an appropriate polymerization method may be employed depending on the type of the polymerizable compound or the polymerization accelerator and a form of a photochromic cured product to be formed.

When a curable composition containing a polymerizable compound is thermally polymerized, the temperature affects a property of the resulting photochromic cured product.

Such a temperature condition cannot be definitely limited since it is affected by the type or amount of a thermal polymerization initiator or the type of polymerizable compound. However, in general, a procedure in which polymerization is started at a relatively low temperature, followed by heating slowly is suitable. Polymerization time also varies depending on various factors as is the case with the temperature. Therefore, although it is suitable to determine an optimum time beforehand in accordance with these conditions, in general, the conditions are preferably selected so that polymerization is completed in 2 to 48 hours. When obtaining a photochromic laminated sheet, polymerization is performed at a temperature at which a reaction between polymerizable functional groups proceeds, and optimal temperature and time are preferably determined to achieve a desired molecular weight.

When a curable composition is photo-polymerized, among the polymerization conditions, especially UV intensity affects a property of the resulting photochromic cured product. Such an irradiation condition cannot be definitely limited since it is affected by the type or amount of a photoinitiator or the type of a polymerizable monomer. However, in general, the conditions are preferably selected so as to give photoirradiation with 50 to 500 mW/cm² of UV light at a wavelength of 365 nm for 0.5 to 5 minutes.

### [Optical Article]

The photochromic compound according to the embodiment can be widely used as a photochromic material, and can be used, for example, as various memory materials alternative to a silver halide photosensitive material, a reprographic material, a photosensitive material for printing, a memory material for a cathode ray tube, a photosensitive material for a laser, a photosensitive material for holography, and various other memory materials. The photochromic material can also be used as a material for a photochromic lens material, an optical filter material, a display material, a light meter, and a decoration material.

The photochromic compound according to the embodiment is particularly suitable for a photochromic lens application. A photochromic lens is suitable as a lens for eyeglasses such as sunglasses. Any known method for producing a photochromic lens can be employed, as long as the method provides uniform photochromic performance.

In the case where a photochromic property is developed by a kneading method, a photochromic cured product in the form of an optical material such as a lens may be obtained by injecting the above-mentioned curable composition between glass molds held with an elastomeric gasket or a spacer and then cast-polymerizing the curable composition by heating the curable composition in an air furnace or irradiation with an active energy ray such as ultraviolet ray depending on the type of a polymerizable compound or a polymerization accelerator.

In the case where a photochromic property is developed by a lamination method, a photochromic layer made of a photochromic cured product is formed on a surface of an optical substrate such as a lens substrate by: dissolving a curable composition in an organic solvent as appropriate to prepare a coating solution; coating the surface of the optical substrate with the coating solution by spin coating, dipping, or the like; drying to remove the organic solvent; and then polymerization-curing the curable composition with UV irradiation, heating, or the like in an inert gas such as nitrogen (coating method).

Furthermore, a photochromic layer made of a photochromic cured product can also be formed on a surface of an optical substrate such as a lens substrate by casting polymerization using an inner mold in which the optical substrate is placed face-to-face in a glass mold so that a predetermined gap is formed, the curable composition is injected into this gap, and the curable composition is polymerization-cured in this state by UV irradiation, heating, or the like (casting polymerization method).

In the case where a photochromic layer is formed on a surface of an optical substrate by the lamination method (coating method and casting polymerization method) as described above, the surface of the optical substrate may be chemically treated with an alkali solution, an acid solution, or the like, or physically treated with corona discharge, plasma discharge, polishing, or the like beforehand in order to enhance adhesion between the photochromic layer and the optical substrate. Of course, it is also possible to provide a transparent adhesive resin layer on the surface of the optical substrate.

In addition, when a photochromic property is developed by a binder method, a photochromic sheet is made by sheet-molding using a curable composition, sandwiched between two transparent sheets (optical sheets), and polymerization-cured as described above to obtain a photochromic laminate with a photochromic layer as an adhesive layer.

In this case, coating with a coating solution prepared by dissolving a curable composition in an organic solvent can also be employed to produce the photochromic sheet.

The thus-produced photochromic laminate is, for example, mounted in a mold and then a thermoplastic resin (e.g., polycarbonate) for an optical substrate such as a lens is injection-molded thereon to obtain an optical substrate such as a lens with a photochromic property in a predetermined shape.

The photochromic laminate can also be glued to a surface of an optical substrate with, for example, an adhesive to obtain a photochromic lens.

Note that when a photochromic laminate is produced as described above, it is preferable to use, as the polymerizable compound, a urethane- or urea-forming polymerizable compound, especially a urethane-forming polymerizable compound and to adjust the polymerizable compound so as to form polyurethane, especially from the viewpoint of high adhesion to the optical substrate.

The above-mentioned curable composition can develop a photochromic property with an excellent color developing density at a higher temperature.

The photochromic layer or the photochromic cured product formed from the curable composition can be subjected to post-processing such as: staining using a dye such as a disperse dye; formation of a hard coat film using a silane coupling agent or a hard coat agent containing as a main component a sol of silicon, zirconium, antimony, aluminum, tin, tungsten, or the like; formation of a thin film by vapor deposition of a metal oxide such as SiO₂, TiO₂, ZrO₂, or the like; antireflection treatment using a thin film by the application of an organic polymer; or antistatic treatment; depending on its application.

### EXAMPLES

The present invention will be described in more detail with reference to a number of Examples provided below by way of example. These Examples are merely illustrative of the present invention, and the spirit and scope of the present invention is not limited to these Examples.

### (Example 1)

### First Step

Charged were 28.9 g (62.4 mmol) of a compound represented by the following formula (1-1), which was synthesized from 4-bromo-4'-methoxybenzophenone with reference to the method described in Patent Document 8, 550 mL of toluene, 9.36 g (87.4 mmol) of N-methylaniline, and 24.0 g (249.6 mmol) of sodium tertiary butoxide into a reaction vessel, and the mixture was stirred under reduced pressure to eliminate dissolved oxygen. Subsequently, 0.57 g (0.6 mmol) of Pd₂(dba)₃ and 1.19 g (2.5 mmol) of X-phos were added to the reaction solution, and the mixture was heated to 80°C.

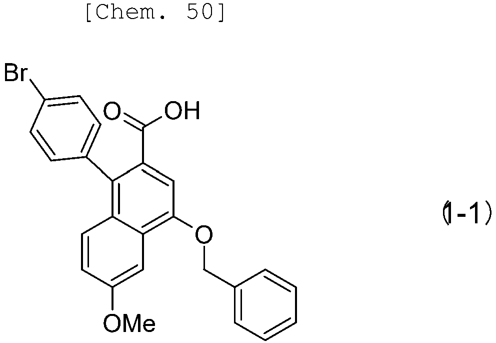

The heating was continued until the starting material was completely consumed, and after the completion of the reaction, the reaction mixture was cooled to room temperature, followed by filtration. The resulting filtrate was neutralized by adding 500 mL of tetrahydrofuran and 10% hydrochloric acid, and then liquid-liquid separation was performed. The resulting organic layer was concentrated, and then a slurry was prepared by adding 100 mL of methanol for purification to the concentrated organic layer, to give a carboxylic acid compound represented by the following formula (1-2) in 92% yield.

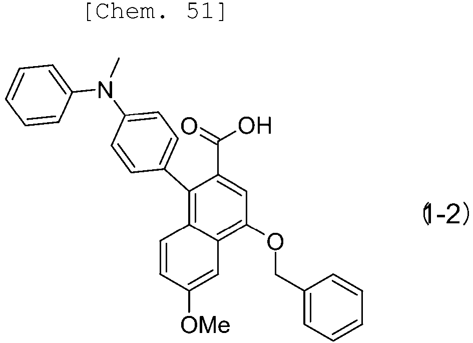

### Second Step

An iodide compound represented by the following formula (1-3) was obtained in 86% yield with reference to the method described in Patent Document 8 except that the carboxylic acid compound obtained in Example 1 was used.

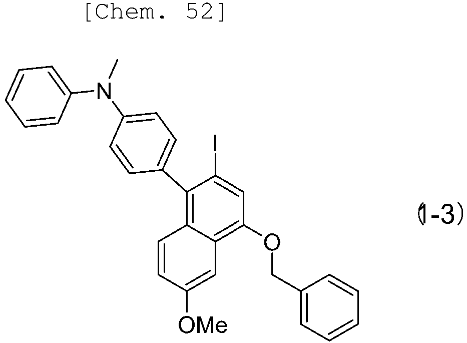

### Third Step

To 22.5 g (41.5 mmol) of the compound according to the formula (1-3) obtained in the second step was added 360 mL of toluene, and azeotropic dehydration was performed until a water content in the toluene was 100 ppm or less. After the azeotropic dehydration, the resulting solution was slowly cooled to - 20°C, and 31.2 mL of n-BuLi (1.6 mol/L hexane solution) was slowly added dropwise while maintaining -15 to -20°C. After the confirmation of the consumption of the starting material, 4.5 g of dehydrated acetone was added dropwise to the mixture. After the dropwise addition, the solution was slowly warmed to room temperature. After the warming, 200 mL of water was added, and liquid-liquid separation was performed. Washing with water was repeated until the pH of the aqueous layer reached 7-8. The solvent of the resulting organic layer was removed, and purification by chromatography on silica gel was carried out, to give a compound represented by the following formula (1-4) in 80% yield.

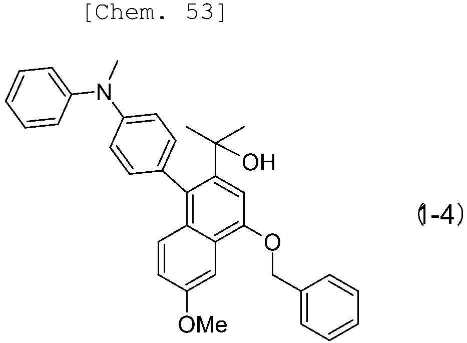

### Fourth Step

To 28.4 g (150.0 mmol) of p-toluenesulfonic acid monohydrate was added 750 mL of toluene, and azeotropic dehydration was performed until a water content in the toluene was 300 ppm or less. Subsequently, a toluene solution prepared by dissolving 17.7 g (35.0 mmol) of the formula (1-4) in 100 mL of toluene was slowly added while maintaining 85-100°C, and after the dropwise addition, the mixture was refluxed. After the confirmation of the consumption of the starting material, the mixture was cooled to room temperature, 500 mL of water was added thereto, and liquid-liquid separation was performed. This operation was repeated five times, then the solvent of the resulting organic layer was removed, and purification by chromatography on silica gel was carried out, to give a phenol derivative represented by the following formula (1-5) in 78% yield.

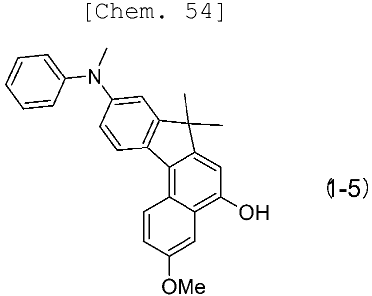

### Fifth Step

Dissolved were 2.38 g (6.0 mmol) of the phenol derivative according to the formula (1-5) and 1.92 g (7.2 mmol) of a propargyl alcohol represented by the following formula (1-6) in 40 mL of toluene, then 0.15 g (0.6 mmol) of pyridinium p-toluenesulfonate was added thereto, and the mixture was stirred at 85°C for 2 hours.

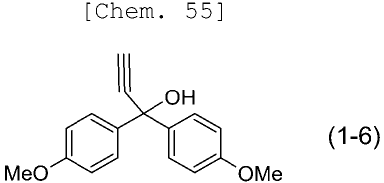

After the consumption of the starting material phenol derivative, the resulting mixture was cooled to room temperature, 40 mL of water was added thereto, and liquid-liquid separation was performed. The solvent of the resulting organic layer was removed, and purification by chromatography on silica gel was carried out, to give a photochromic compound represented by the following formula (1-7) in 80% yield.

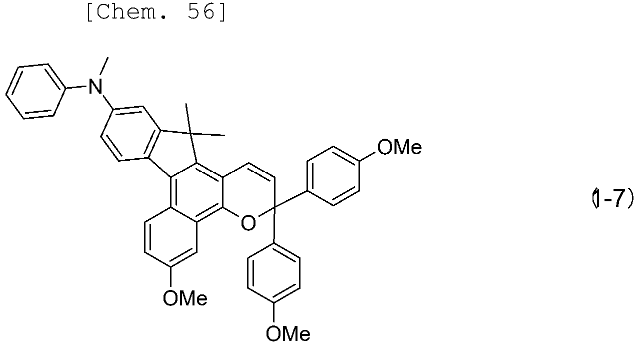

Elemental analysis values for the photochromic compound represented by the formula (1-7) were determined to be C: 81.82%, H: 6.06%, N: 2.16%, which are in good agreement with the calculated values for C₄₄H₄₉NO₄ of C: 81.83%, H: 6.09%, N: 2.17%.

A proton nuclear magnetic resonance spectrum was measured, and showed 9H peaks assigned to the methyl groups at around δ 0.5 to 3.5 ppm, 9H peaks assigned to the methoxy groups at around δ 3.5 to 5.0 ppm, and 21H peaks assigned to the aromatic protons and alkene protons at around δ 5.0 to 9.0 ppm.

Furthermore, a ¹³C-nuclear magnetic resonance spectrum was measured, and showed peaks assigned to the carbons of the aromatic rings around at δ 110 to 160 ppm, peaks assigned to the carbons of the alkene around at δ 80 to 140 ppm, and peaks assigned to the carbons of the alkyls at δ 20 to 60 ppm.

### (Example 2)

### First Step

The reaction was carried out similarly to the first step in Example 1, except that 4-bromo-3',4'-dimethoxybenzophenone was used in place of 4-bromo-4'-methoxybenzophenone, to give a carboxylic acid represented by the following formula (2-1) in 92% yield.

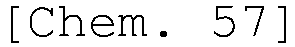

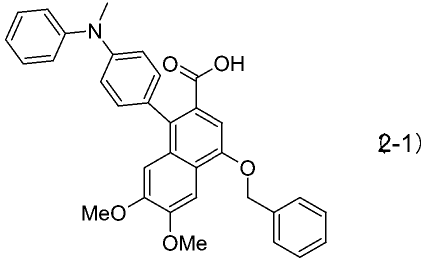

### Second Step

Dissolved was 10.4 g (20.0 mmol) of the compound according to the formula (2-1) in 400 mL of THF, then 3 g of 5% Pd/C (wetted with 50% water) was added thereto, and the mixture was stirred under hydrogen pressure conditions for 19 hours. After the completion of the reaction, filtration was carried out, THF was removed from the resulting filtrate, and then purification by chromatography on silica gel was carried out, to give to a debenzylation product represented by the following formula (2-2) in 100% yield.

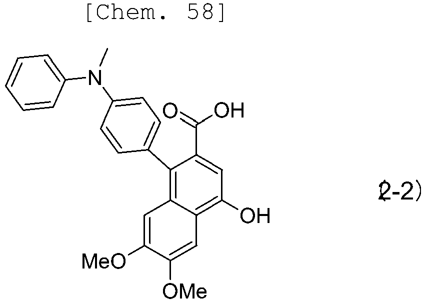

### Third Step

Charged were 7.6 g (40.0 mmol) of p-toluenesulfonic acid monohydrate and 400 mL of toluene into a reaction vessel, and azeotropic dehydration was performed until a water content in the toluene was 100 ppm or less. After the azeotropic dehydration, the compound according to the formula (2-2) was added at an internal temperature of 80°C, the mixture was heated to the azeotropic temperature, to allow the reaction to proceed while performing the azeotropic dehydration. After the confirmation of the consumption of the starting material, the resulting mixture was cooled to room temperature, and the resulting solid was filtered, to give a carbonyl compound represented by the following formula (2-3) in 96% yield.

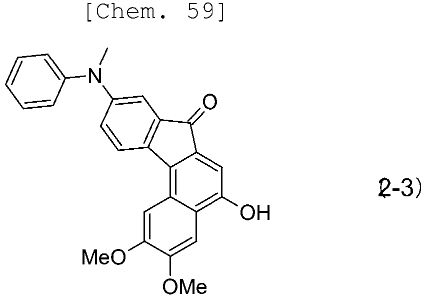

### Fourth Step

A reaction was carried out using the carbonyl compound represented by the formula (2-3) with reference to the method described in Patent Document 9, to give a phenol derivative represented by the following formula (2-4) in 91% yield.

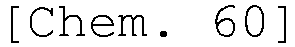

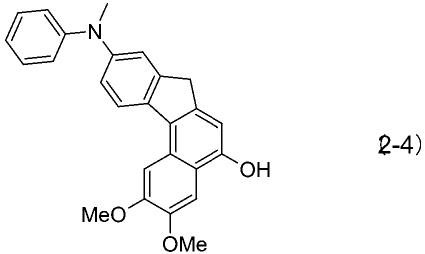

### Fifth Step

Dissolved were 6.9 g (17.4 mmol) of the phenol derivative represented by the formula (2-4) and 2.7 g (21.3 mmol) of benzyl chloride in 100 ml of DMF. To this solution 3.6 g (26.1 mmol) of potassium carbonate was added, and the mixture was stirred at 80°C. After the consumption of the starting material, the mixture was cooled to room temperature, and water was added thereto and washed to be neutral. The solvent was removed from the resulting organic layer, and purification by chromatography on silica gel was carried out, to give a benzyl compound represented by the following formula (2-5) in 97% yield.

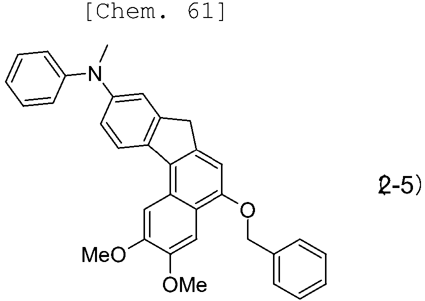

### Sixth Step

Charged were 4.9 g (10.0 mmol) of the benzyl compound according to the formula (2-5), 8.4 g (50.0 mmol) of 1-bromo-4-methoxybutane, and 60 mL of THF into a reaction vessel, and the mixture was cooled with an ice bath. Added was 3.4 g (30.0 mmol) of tBuOK in four portions while maintaining 0-5°C. After the consumption of the starting material, the mixture was neutralized with 10% hydrochloric acid, then 30 mL of toluene was added thereto, and liquid-liquid separation was performed. The solvent was removed from the resulting organic layer, then the debenzylation reaction was carried out similarly to the second step, and purification by chromatography on silica gel was carried out, to give a phenol derivative represented by the following formula (2-6) in 88% yield.

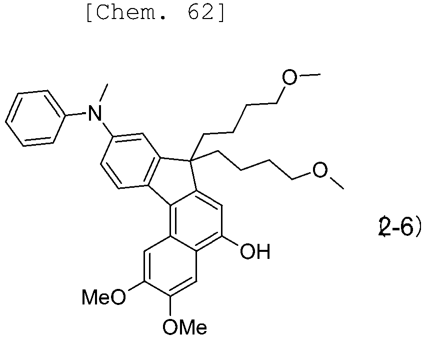

### Seventh Step

The reaction was carried out similarly to the fifth step in Example 1, except that the compound according to the formula (2-6) was used in place of the compound according to the formula (1-5), to give a photochromic compound represented by the following formula (2-7) in 76% yield.

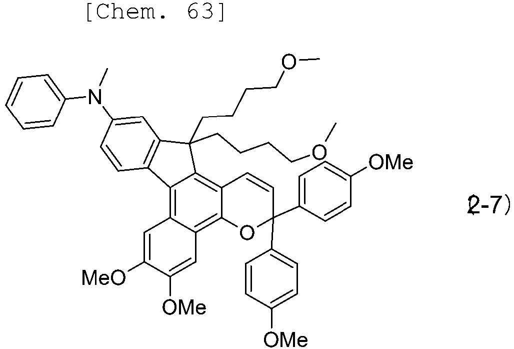

Elemental analysis values for the photochromic compound represented by the formula (2-7) were determined to be C: 77.62%, H: 7.00%, N: 1.72%, which are in good agreement with the calculated values for C₅₃H₅₇NO₇ of C: 77.63%, H: 7.01%, N: 1.71%.

A proton nuclear magnetic resonance spectrum was measured, and showed 15H peaks assigned to the methyl group and the 4-methoxybutyl groups at around δ 0.5 to 3.5 ppm, 22H peaks assigned to the methoxy groups and the 4-methoxybutyl groups at around δ 3.5 to 5.0 ppm, and 20H peaks assigned to the aromatic protons and alkene protons at around δ 5.0 to 9.0 ppm.

Furthermore, a ¹³C-nuclear magnetic resonance spectrum was measured, and showed peaks assigned to the carbons of the aromatic rings around at δ 110 to 160 ppm, peaks assigned to the carbons of the alkene around at δ 80 to 140 ppm, and peaks assigned to the carbons of the alkyls at δ 20 to 60 ppm.

### (Example 3)

### First Step

The reaction was carried out similarly to Example 2, except that 4-bromo-4'-methylbenzophenone was used in place of 4-bromo-3',4'-dimethoxybenzophenone, and benzomorpholine was used in place of N-methylaniline in the first step, and 1,1,1-trifluoro-4-iodobutane was used in place of 1-bromo-4-methoxybutane in the sixth step, to synthesize a phenol derivative represented by the following formula (3-1).

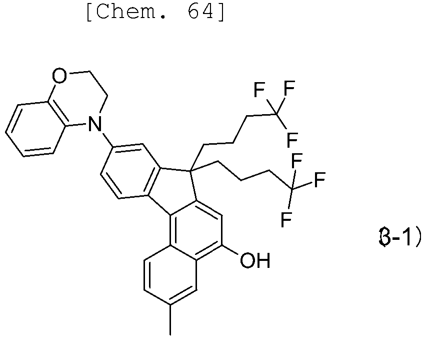

### Second Step

The reaction was carried out similarly to the fifth step in Example 1, except that the compound according to the formula (3-1) was used in place of the compound according to the formula (1-5), and a compound according to the following formula (3-2) was used in place of the compound according to the formula (1-6), to give a photochromic compound represented by the following formula (3-3) in 75% yield.

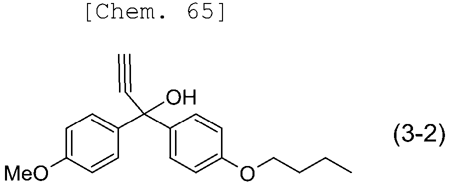

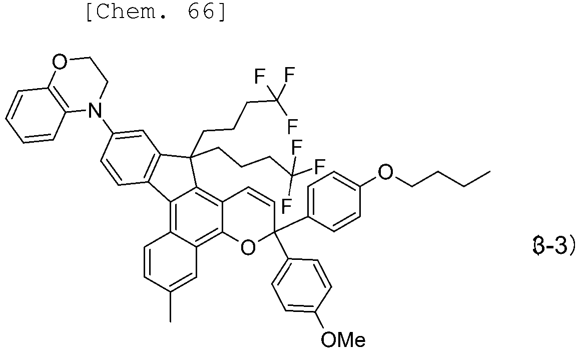

Elemental analysis values for the photochromic compound represented by the formula (3-3) were determined to be C: 72.73%, H: 5.75%, N: 1.55%, which are in good agreement with the calculated values for C₅₄H₅₁F₆NO₄ of C: 72.71%, H: 5.76%, N: 1.57%.

A proton nuclear magnetic resonance spectrum was measured, and showed 24H peaks assigned to the methyl group, the 1,1,1-trifluorobutyl groups, the butoxy group, and the benzomorpholino group at around δ 0.5 to 3.5 ppm, 7H peaks assigned to the methoxy group, the butoxy group, and the benzomorpholino group at around δ 3.5 to 5.0 ppm, and 20H peaks assigned to the aromatic protons and alkene protons at around δ 5.0 to 9.0 ppm.

Furthermore, a ¹³C-nuclear magnetic resonance spectrum was measured, and showed peaks assigned to the carbons of the aromatic rings around at δ 110 to 160 ppm, peaks assigned to the carbons of the alkene around at δ 80 to 140 ppm, and peaks assigned to the carbons of the alkyls at δ 20 to 60 ppm.

### (Example 4)

### First Step

The reaction was carried out similarly to the third step in Example 1, except that cyclooctanone was used in place of acetone, to give a naphthol according to the following formula (4-1) in 42% yield.

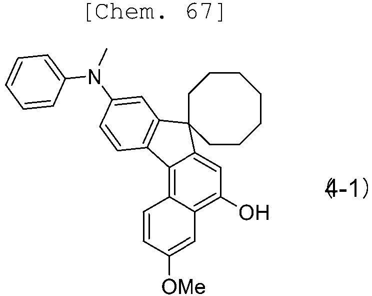

### Second Step

The reaction was carried out similarly to the fifth step in Example 1, except that the compound according to the formula (4-1) was used in place of the compound according to the formula (1-5), and the compound according to the following formula (4-2) was used in place of the compound according to the formula (1-6), to give a photochromic compound represented by the following formula (4-3) in 75% yield.

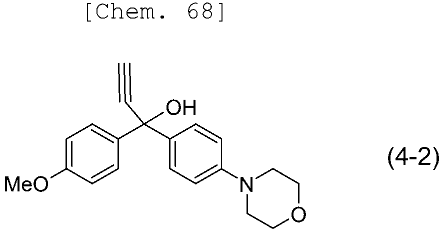

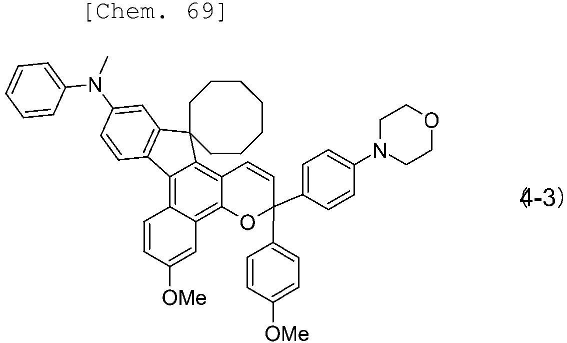

Elemental analysis values for the photochromic compound represented by the formula (4-3) were determined to be C: 81.24%, H: 6.81%, N: 3.64%, which are in good agreement with the calculated values for C₅₂H₅₂N₂O₄ of C: 81.22%, H: 6.82%, N: 3.64%.

A proton nuclear magnetic resonance spectrum was measured, and showed 21H peaks assigned to the methyl group, the cyclooctyl group, and the morpholino group at around δ 0.5 to 3.5 ppm, 10H peaks assigned to the methoxy groups and the morpholino group at around δ 3.5 to 5.0 ppm, and 21H peaks assigned to the aromatic protons and alkene protons at around δ 5.0 to 9.0 ppm.

Furthermore, a ¹³C-nuclear magnetic resonance spectrum was measured, and showed peaks assigned to the carbons of the aromatic rings around at δ 110 to 160 ppm, peaks assigned to the carbons of the alkene around at δ 80 to 140 ppm, and peaks assigned to the carbons of the alkyls at δ 20 to 60 ppm.

### (Example 5)

### First Step

4-Bromobenzoyl chloride was reacted with 2,4-dimethoxy-1,1-biphenyl with reference to the method described in Patent Document 5, to give a compound represented by the following formula (5-1) in 83% yield.

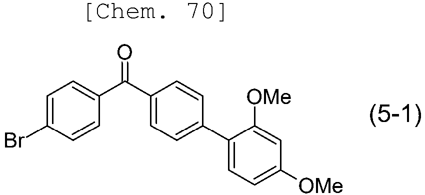

### Second Step

The reaction was carried out similarly to Example 1, except that the compound according to the formula (5-1) was used in place of 4-bromo-4'-methoxybenzophenone, and 4-fluoro-N-methylaniline was used in place of N-methylaniline in the first step, and diethyl ketone was used in place of acetone in the third step, to synthesize a phenol derivative represented by the following formula (5-2).

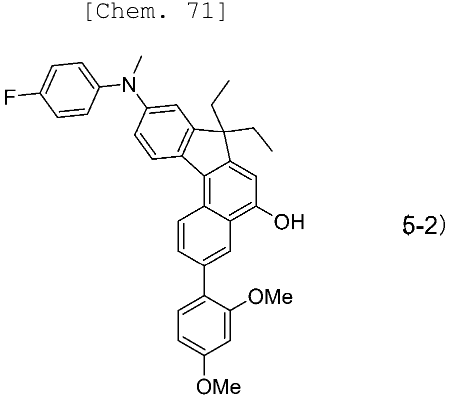

### Third Step

The reaction was carried out similarly to the fifth step in Example 1, except that the compound according to the formula (5-2) was used in place of the compound according to the formula (1-5), and a compound according to the following formula (5-3) was used in place of the compound according to the formula (1-6), to give a photochromic compound represented by the following formula (5-4) in 71% yield.

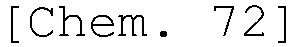

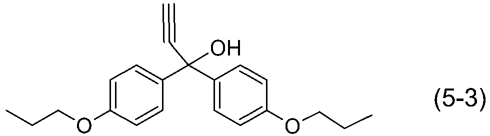

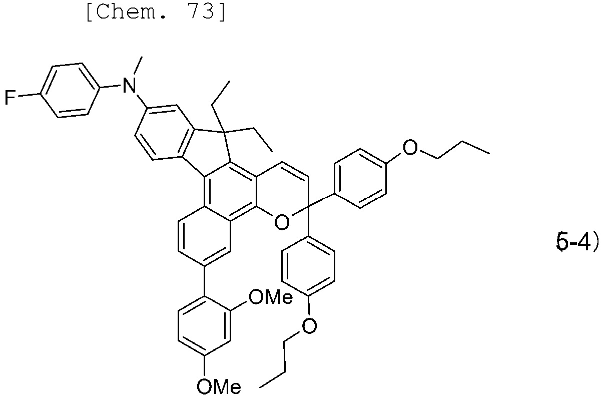

Elemental analysis values for the photochromic compound represented by the formula (5-4) were determined to be C: 80.14%, H: 6.59%, N: 1.65%, which are in good agreement with the calculated values for C₅₇H₅₆FNO₅ of C: 80.16%, H: 6.61%, N: 1.64%.

A proton nuclear magnetic resonance spectrum was measured, and showed 23H peaks assigned to the methyl group, the ethyl groups, and the propyloxy groups at around δ 0.5 to 3.5 ppm, 10H peaks assigned to the methoxy groups and the propyloxy groups at around δ 3.5 to 5.0 ppm, and 23H peaks assigned to the aromatic protons and alkene protons at around δ 5.0 to 9.0 ppm.

Furthermore, a ¹³C-nuclear magnetic resonance spectrum was measured, and showed peaks assigned to the carbons of the aromatic rings around at δ 110 to 160 ppm, peaks assigned to the carbons of the alkene around at δ 80 to 140 ppm, and peaks assigned to the carbons of the alkyls at δ 20 to 60 ppm.

### (Example 6)

### First Step

The reaction was carried out similarly to Example 1, except that 4-bromo-4'-trifluoromethoxybenzophenone was used in place of 4-bromo-4'-methoxybenzophenone, and 4-(trifluoromethyl)-N-methylaniline was used in place of N-methylaniline in the first step, and 4,4-diethylcyclohexanone was used in place of acetone in the third step, to synthesize a phenol derivative represented by the following formula (6-1).

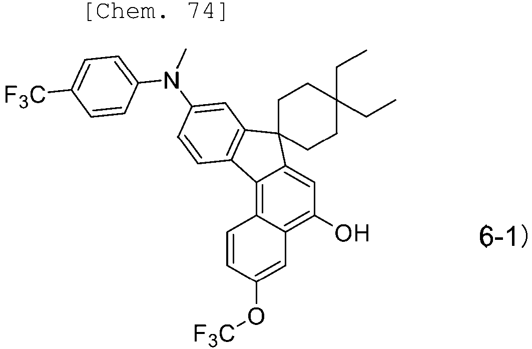

### Second Step

The reaction was carried out similarly to the fifth step in Example 1, except that the compound according to the formula (6-1) was used in place of the compound according to the formula (1-5), and a compound according to the following formula (6-2) was used in place of the compound according to the formula (1-6), to give a photochromic compound represented by the following formula (6-3) in 67% yield.

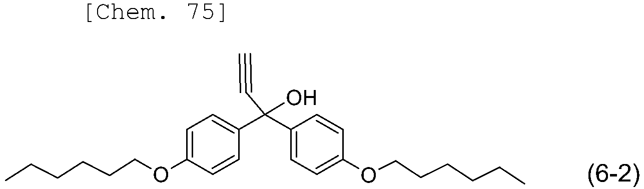

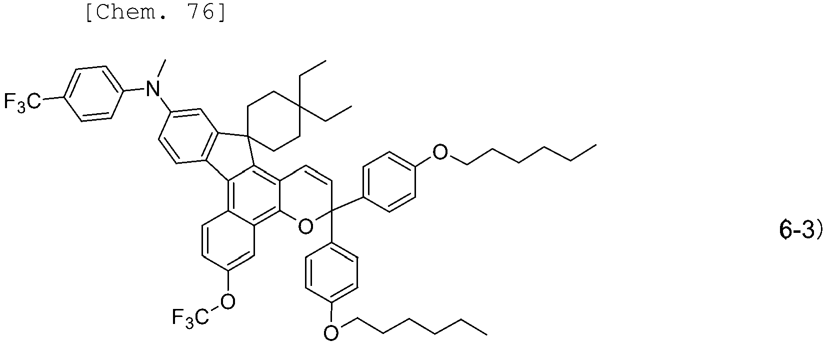

Elemental analysis values for the photochromic compound represented by the formula (6-3) were determined to be C: 74.13%, H: 6.72%, N: 1.40%, which are in good agreement with the calculated values for C₆₂H₆₇F₆NO₄ of C: 74.16%, H: 6.73%, N: 1.39%.

A proton nuclear magnetic resonance spectrum was measured, and showed 43H peaks assigned to the methyl group, the diethylcyclohexyl group, and the hexyloxy groups at around δ 0.5 to 3.5 ppm, 4H peaks assigned to the hexyloxy groups at around δ 3.5 to 5.0 ppm, and 20H peaks assigned to the aromatic protons and alkene protons at around δ 5.0 to 9.0 ppm.

Furthermore, a ¹³C-nuclear magnetic resonance spectrum was measured, and showed peaks assigned to the carbons of the aromatic rings around at δ 110 to 160 ppm, peaks assigned to the carbons of the alkene around at δ 80 to 140 ppm, and peaks assigned to the carbons of the alkyls at δ 20 to 60 ppm.

### (Example 7)

### First Step

The reaction was carried out similarly to the first step in Example 5, except that 2,2-dimethyl-1,3-benzoxathiole was used in place of 2,4-dimethoxy-1,1-biphenyl, to give a compound represented by the following formula (7-1) in 71% yield.

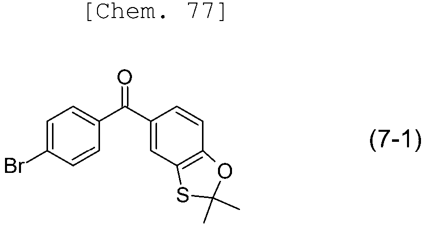

### Second Step

The reaction was carried out similarly to Example 2, except that the compound according to the formula (7-1) was used in place of 4-bromo-3',4'-dimethoxybenzophenone, and 1,2,3,4-tetrahydroquinoline was used in place of N-methylaniline in the first step, and 1-bromo-3-methoxypropane was used in place of 1-bromo-4-methoxybutane in the sixth step, to synthesize a phenol derivative represented by the following formula (7-2).

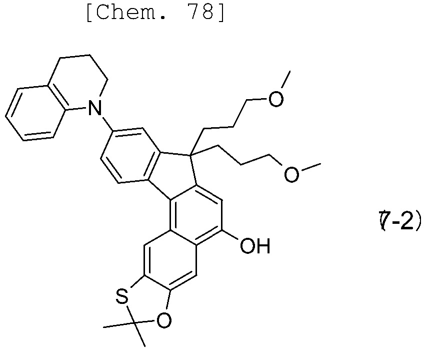

### Third Step

The reaction was carried out similarly to the fifth step in Example 1, except that the compound according to the formula (7-2) was used in place of the compound according to the formula (1-5), and a compound according to the following formula (7-3) was used in place of the compound according to the formula (1-6), to give a photochromic compound represented by the following formula (7-4) in 77% yield.

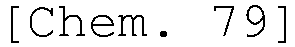

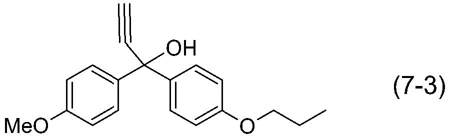

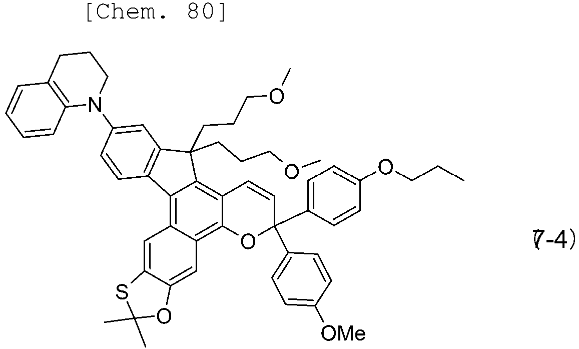

Elemental analysis values for the photochromic compound represented by the formula (7-4) were determined to be C: 76.95%, H: 6.81%, N: 1.59%, S: 3.67%, which are in good agreement with the calculated values for C₅₆H₅₉NO₆S of C: 76.94%, H: 6.80%, N: 1.60%, S: 3.67%.

A proton nuclear magnetic resonance spectrum was measured, and showed 25H peaks assigned to the methyl groups, the 3-methoxypropyl groups, the propoxy group, and the 1,2,3,4-tetrahydroquinoline ring group at around δ 0.5 to 3.5 ppm, 15H peaks assigned to the methoxy group, the 3-methoxypropyl groups, and the propoxy group at around δ 3.5 to 5.0 ppm, and 19H peaks assigned to the aromatic protons and alkene protons at around δ 5.0 to 9.0 ppm.

Furthermore, a ¹³C-nuclear magnetic resonance spectrum was measured, and showed peaks assigned to the carbons of the aromatic rings around at δ 110 to 160 ppm, peaks assigned to the carbons of the alkene around at δ 80 to 140 ppm, and peaks assigned to the carbons of the alkyls at δ 20 to 60 ppm.

### (Example 8)

### First Step

The reaction was carried out similarly to Example 2, except that 4-bromo-4'-phenoxy benzophenone was used in place of 4-bromo-3',4'-dimethoxybenzophenone, and diphenylamine was used in place of N-methylaniline in the first step, and 1-bromo-4-fluorobutane was used in place of 1-bromo-4-methoxybutane in the sixth step, to synthesize a phenol derivative represented by the following formula (8-1).

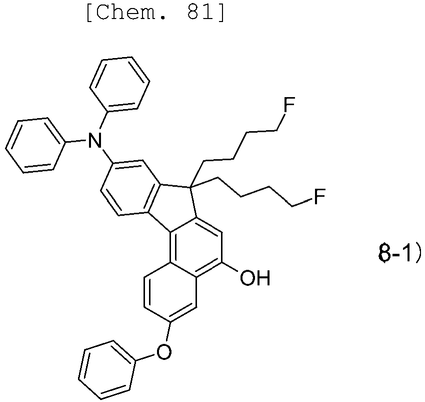

### Second Step

The reaction was carried out similarly to the fifth step in Example 1, except that the compound according to the formula (8-1) was used in place of the compound according to the formula (1-5), to give a photochromic compound represented by the following formula (8-2) in 71% yield.

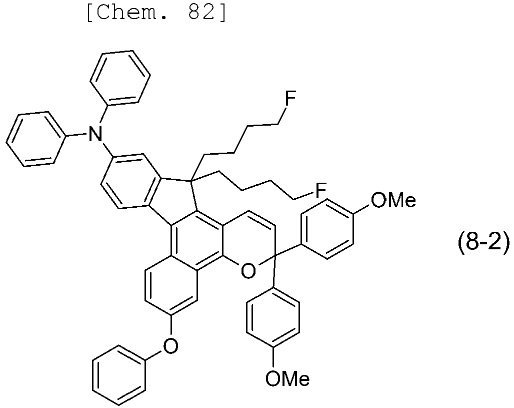

Elemental analysis values for the photochromic compound represented by the formula (8-2) were determined to be C: 80.95%, H: 5.98%, N: 1.57%, which are in good agreement with the calculated values for C₆₀H₅₃F₂NO₄ of C: 80.97%, H: 6.00%, N: 1.57%.

A proton nuclear magnetic resonance spectrum was measured, and showed 16H peaks assigned to the 4-fluorobutyl groups at around δ 0.5 to 3.5 ppm, 6H peaks assigned to the methoxy groups at around δ 3.5 to 5.0 ppm, and 31H peaks assigned to the aromatic protons and alkene protons at around δ 5.0 to 9.0 ppm.

Furthermore, a ¹³C-nuclear magnetic resonance spectrum was measured, and showed peaks assigned to the carbons of the aromatic rings around at δ 110 to 160 ppm, peaks assigned to the carbons of the alkene around at δ 80 to 140 ppm, and peaks assigned to the carbons of the alkyls at δ 20 to 60 ppm.

### (Example 9)

### First Step

The reaction was carried out similarly to Example 2, except that 4-bromo-4'-methoxy-3'-methylbenzophenone was used in place of 4-bromo-3',4'-dimethoxybenzophenone, and benzothiomorpholine was used in place of N-methylaniline in the first step, and iodopropane was used in place of 1-bromo-4-methoxybutane in the sixth step, to synthesize a phenol derivative represented by the following formula (9-1).

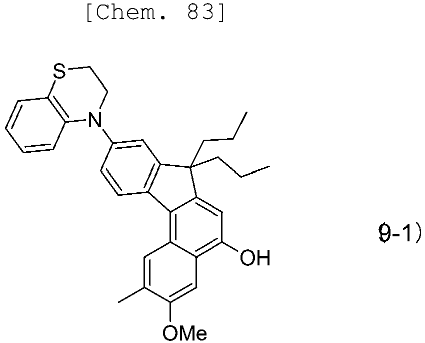

### Second Step

The reaction was carried out similarly to the third step in Example 7, except that the compound according to the formula (9-1) was used in place of the compound according to the formula (7-2), to give a photochromic compound represented by the following formula (9-2) in 76% yield.

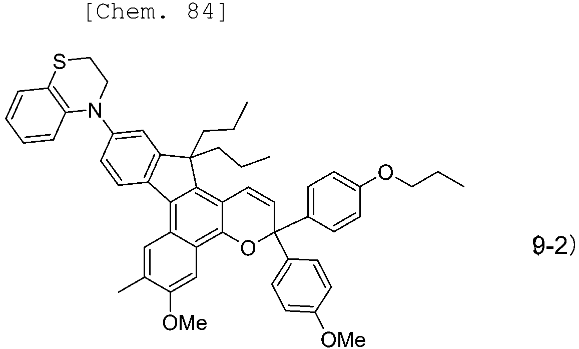

Elemental analysis values for the photochromic compound represented by the formula (9-2) were determined to be C: 79.26%, H: 6.80%, N: 1.78%, S: 4.09%, which are in good agreement with the calculated values for C₅₂H₅₃NO₄S of C: 79.25%, H: 6.78%, N: 1.78%, S: 4.07%.

A proton nuclear magnetic resonance spectrum was measured, and showed 24H peaks assigned to the methyl group, the propyl groups, the propoxy group, and the benzothiomorpholine ring group at around δ 0.5 to 3.5 ppm, 10H peaks assigned to the methoxy groups, the propoxy group, and the benzothiomorpholine ring group at around δ 3.5 to 5.0 ppm, and 19H peaks assigned to the aromatic protons and alkene protons at around δ 5.0 to 9.0 ppm.

Furthermore, a ¹³C-nuclear magnetic resonance spectrum was measured, and showed peaks assigned to the carbons of the aromatic rings around at δ 110 to 160 ppm, peaks assigned to the carbons of the alkene around at δ 80 to 140 ppm, and peaks assigned to the carbons of the alkyls at δ 20 to 60 ppm.

### (Example 10)

### First Step

The reaction was carried out using 4-bromo-3'-fluoro-4'-methylbenzophenone with reference to the method described in Patent Document 10, to give a compound represented by the following formula (10-1) in 83% yield.

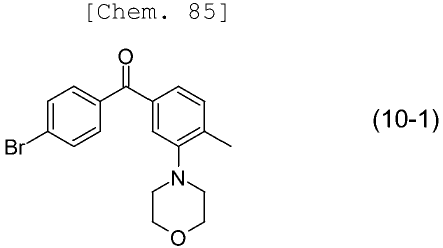

### Second Step

The reaction was carried out similarly to Example 2, except that the compound represented by the formula (10-1) was used in place of 4-bromo-3',4'-dimethoxybenzophenone, and N-(2-methoxyethyl)aniline was used in place of N-methylaniline in the first step, and 1-bromo-2-methoxyethane was used in place of 1-bromo-4-methoxybutane in the sixth step, to synthesize a phenol derivative represented by the following formula (10-2).

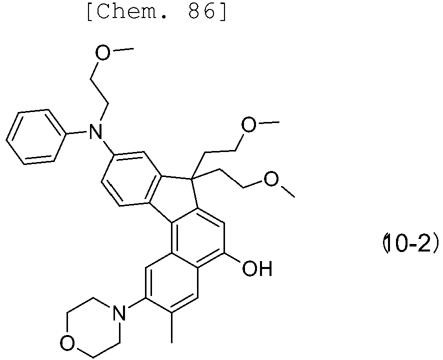

### Third Step

The reaction was carried out similarly to the fifth step in Example 1, except that the compound according to the formula (10-2) was used in place of the compound according to the formula (1-5), to give a photochromic compound represented by the following formula (10-3) in 65% yield.

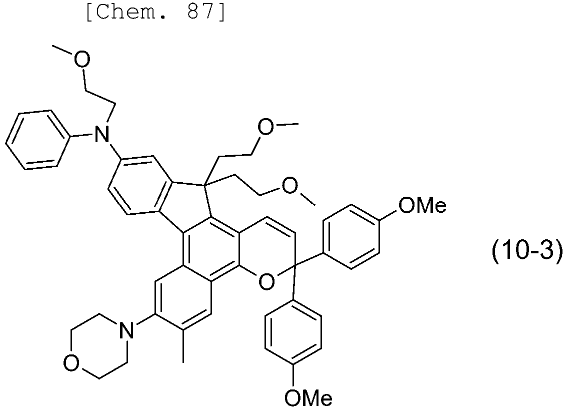

Elemental analysis values for the photochromic compound represented by the formula (10-3) were determined to be C: 76.59%, H: 6.89%, N: 3.32%, which are in good agreement with the calculated values for C₅₄H₅₈N₂O₇ of C: 76.57%, H: 6.90%, N: 3.31%.

A proton nuclear magnetic resonance spectrum was measured, and showed 13H peaks assigned to the methyl group, the 2-methoxyethyl groups, and the morpholino group at around δ 0.5 to 3.5 ppm, 25H peaks assigned to the methoxy groups, the 2-methoxyethyl groups, and the morpholino group at around δ 3.5 to 5.0 ppm, and 20H peaks assigned to the aromatic protons and alkene protons at around δ 5.0 to 9.0 ppm.

Furthermore, a ¹³C-nuclear magnetic resonance spectrum was measured, and showed peaks assigned to the carbons of the aromatic rings around at δ 110 to 160 ppm, peaks assigned to the carbons of the alkene around at δ 80 to 140 ppm, and peaks assigned to the carbons of the alkyls at δ 20 to 60 ppm.

### (Example 11)

### First Step

2-Bromoanisole was reacted with 2,6-dimethylbenzenethiol with reference to the method described in Patent Document 5, to give a compound represented by the following formula (11-1) in 89% yield.

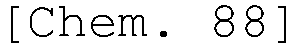

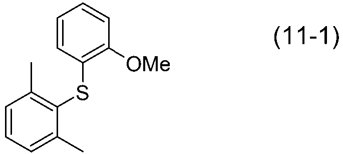

### Second Step

The compound according to the formula (11-1) was reacted with 4-bromobenzoyl chloride with reference to the method described in Patent Document 5, to give a compound represented by the following formula (11-2) in 91% yield.

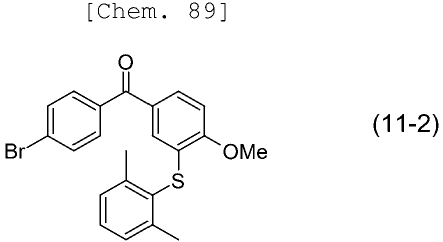

### Third Step

The reaction was carried out similarly to Example 1, except that the compound according to the formula (11-2) was used in in place of 4-bromo-4'-methoxybenzophenone, and 3,4-dihydro-7-fluoro-2H-1,4-benzoxazine was used in place of N-methylaniline in the first step, and 3,3,5,5-tetramethylcyclohexanone was used in place of acetone in the third step, to synthesize a naphthol represented by the following formula (11-3).

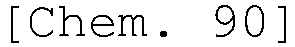

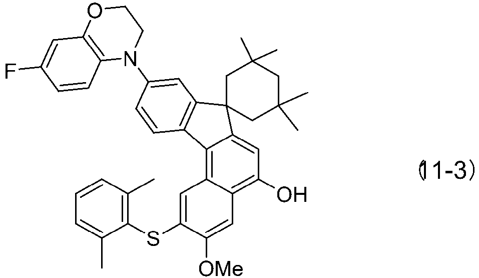

### Fourth Step

The reaction was carried out similarly to the second step in Example 4, except that the compound represented by the formula (11-3) was used in place of the compound according to the formula (4-1), to give a photochromic compound represented by the following formula (11-4) in 71% yield.

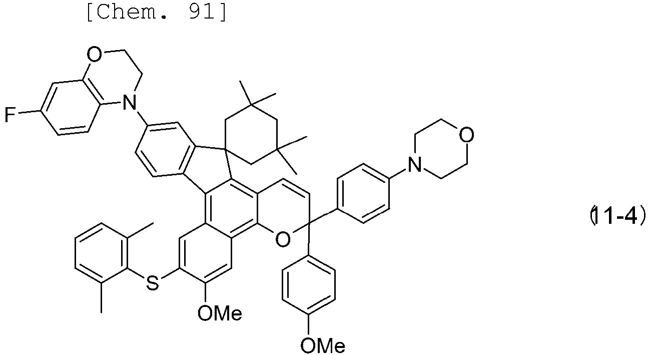

Elemental analysis values for the photochromic compound represented by the formula (11-4) were determined to be C: 77.25%, H: 6.48%, N: 2.85%, S: 3.25%, which are in good agreement with the calculated values for C₆₃H₆₃FN₂O₅S of C: 77.27%, H: 6.48%, N: 2.86%, S: 3.27%.

A proton nuclear magnetic resonance spectrum was measured, and showed 30H peaks assigned to the methyl groups, the tetramethylcyclohexyl group, the morpholino group, and the benzomorpholino group at around δ 0.5 to 3.5 ppm, 12H peaks assigned to the methoxy groups, the morpholino group, and the benzomorpholino group at around δ 3.5 to 5.0 ppm, and 21H peaks assigned to the aromatic protons and alkene protons at around δ 5.0 to 9.0 ppm.

Furthermore, a ¹³C-nuclear magnetic resonance spectrum was measured, and showed peaks assigned to the carbons of the aromatic rings around at δ 110 to 160 ppm, peaks assigned to the carbons of the alkene around at δ 80 to 140 ppm, and peaks assigned to the carbons of the alkyls at δ 20 to 60 ppm.

### (Example 12)

### First Step

Charged were 7.2 g (15.0 mmol) of a compound according to the following formula (12-1), which was synthesized from 3-bromo-4-methyl-4'-chlorobenzophenone, 40 mL of 1,2-dimethoxyethane with reference to the method described in Patent Document 8, 4 mL of ethanol, 3.5 g (33.0 mmol) of sodium carbonate, 66 mL of water, and 2.6 g (17.3 mmol) of 4-methoxyphenylboronic acid into a reaction vessel, and the mixture was purged with nitrogen under reduced pressure. To the mixture, 86.6 mg of tetrakis(triphenylphosphine)palladium was added, and the reaction was allowed to proceed under reflux. After the consumption of the starting material, 100 mL of THF and 50 mL of toluene were added, then 10% hydrochloric acid was added until the pH of the aqueous layer reached 1, and liquid-liquid separation was performed. Twenty mL of water was added, liquid-liquid separation was performed, then the solvent of the organic layer was evaporated, and then 50 mL of methanol was added, and the mixture was heated. The heating was carried out for 1 hour under reflux, and then the mixture was cooled. The mixture was stirred at 0 to 5°C for 3 hours, and then the resulting solid was filtered, to give a carboxylic acid represented by the following formula (12-2) in 95% yield.

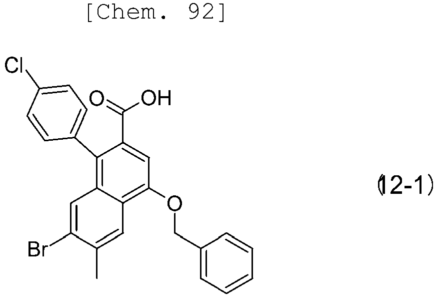

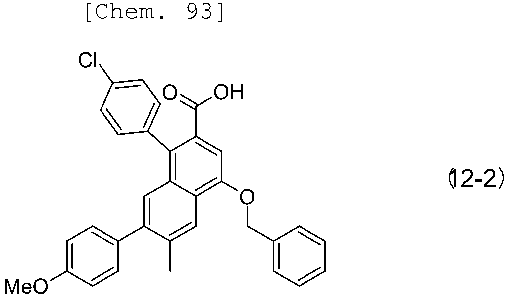

### Second Step

The reaction was carried out similarly to Example 1, except that the compound represented by the formula (12-2) was used in place of the compound represented by the formula (1-1), and N-ethylaniline was used in place of N-methylaniline in the first step, and dibutyl ketone was used in place of acetone in the third step, to synthesize a phenol derivative represented by the following formula (12-3).

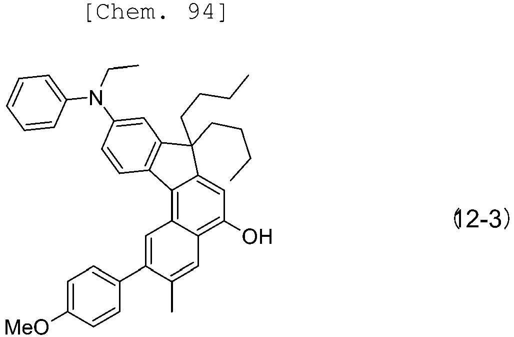

### Third Step

The reaction was carried out similarly to the fifth step in Example 1, except that the compound represented by the formula (12-3) was used in place of the compound according to the formula (1-5), and a compound according to the following formula (12-4) was used in place of the compound according to the formula (1-6), to give a photochromic compound represented by the following formula (12-5) in 67% yield.

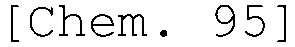

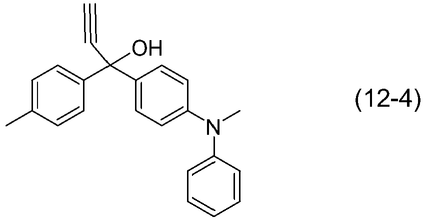

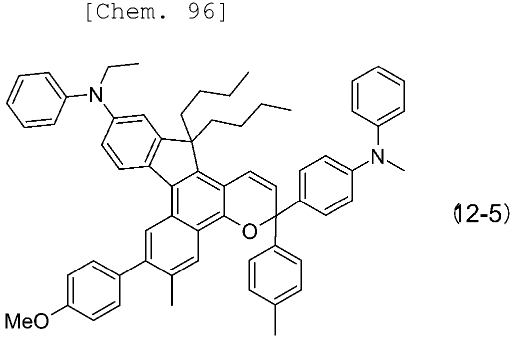

Elemental analysis values for the photochromic compound represented by the formula (12-5) were determined to be C: 86.08%, H: 7.21%, N: 3.15%, which are in good agreement with the calculated values for C₆₄H₆₄N₂O₂ of C: 86.06%, H: 7.22%, N: 3.14%.

A proton nuclear magnetic resonance spectrum was measured, and showed 32H peaks assigned to the methyl groups, the ethyl group, and the butyl groups at around δ 0.5 to 3.5 ppm, 3H peaks assigned to the methoxy group at around δ 3.5 to 5.0 ppm, and 29H peaks assigned to the aromatic protons and alkene protons at around δ 5.0 to 9.0 ppm.

Furthermore, a ¹³C-nuclear magnetic resonance spectrum was measured, and showed peaks assigned to the carbons of the aromatic rings around at δ 110 to 160 ppm, peaks assigned to the carbons of the alkene around at δ 80 to 140 ppm, and peaks assigned to the carbons of the alkyls at δ 20 to 60 ppm.

### (Example 13)

### First Step

The reaction was carried out similarly to Example 1, except that benzomorpholine was used in place of N-methylaniline, to synthesize a phenol derivative represented by the following formula (13-1).

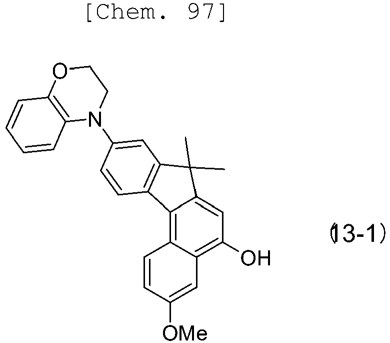

### Second Step

The reaction was carried out similarly to the fifth step in Example 1, except that the compound according to the formula (13-1) was used in place of the compound according to the formula (1-5), to give a photochromic compound represented by the following formula (13-2) in 76% yield.

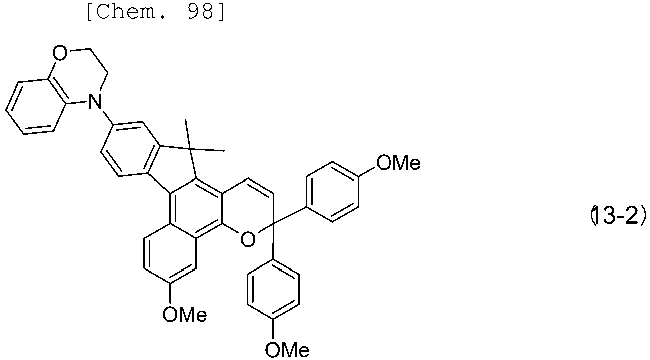

Elemental analysis values for the photochromic compound represented by the formula (13-2) were determined to be C: 80.21%, H: 5.85%, N: 2.06%, which are in good agreement with the calculated values for C₄₅H₃₉NO₅ of C: 80.21%, H: 5.83%, N: 2.08%.

A proton nuclear magnetic resonance spectrum was measured, and showed 8H peaks assigned to the methyl groups and the benzomorpholino group at around δ 0.5 to 3.5 ppm, 11H peaks assigned to the methoxy groups and the benzomorpholino group at around δ 3.5 to 5.0 ppm, and 20H peaks assigned to the aromatic protons and alkene protons at around δ 5.0 to 9.0 ppm.

Furthermore, a ¹³C-nuclear magnetic resonance spectrum was measured, and showed peaks assigned to the carbons of the aromatic rings around at δ 110 to 160 ppm, peaks assigned to the carbons of the alkene around at δ 80 to 140 ppm, and peaks assigned to the carbons of the alkyls at δ 20 to 60 ppm.

### (Example 14)

### First Step

The reaction was carried out similarly to Example 2, except that 4-bromo-4'-methoxybenzophenone was used in place of 4-bromo-3',4'-dimethoxybenzophenone, and N-methyl-p-toluidine was used in place of N-methylaniline in the first step, and 1,1,1-trifluoromethyl-3-iodopropane was used in place of 1-bromo-4-methoxybutane in the sixth step, to synthesize a phenol derivative represented by the following formula (14-1).

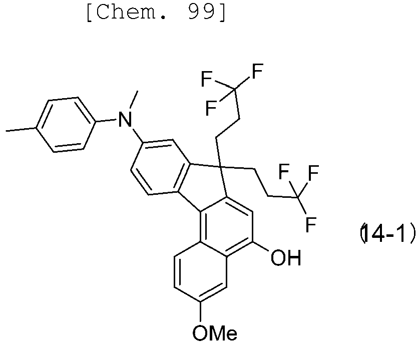

### Second Step

The reaction was carried out similarly to the fifth step in Example 1, except that the compound according to the formula (14-1) was used in place of the compound according to the formula (1-5), and a compound according to the following formula (14-2) was used in place of the compound according to the formula (1-6), to give a photochromic compound represented by the following formula (14-3) in 68% yield.

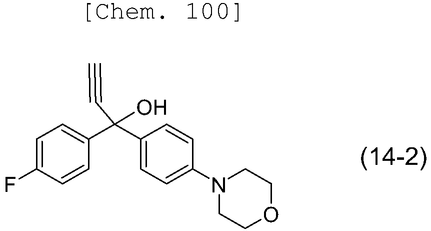

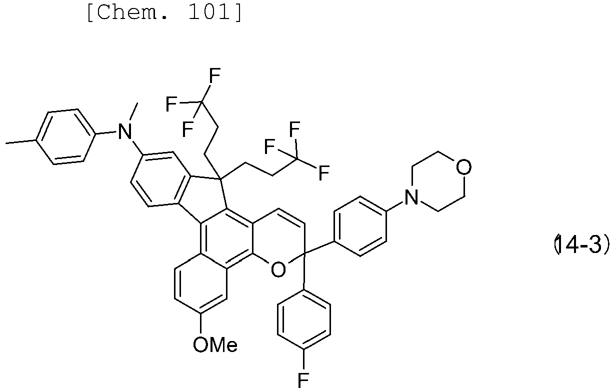

Elemental analysis values for the photochromic compound represented by the formula (14-3) were determined to be C: 70.63%, H: 5.21%, N: 3.22%, which are in good agreement with the calculated values for C₅₁H₄₅F₇N₂O₃ of C: 70.66%, H: 5.23%, N: 3.23%.

A proton nuclear magnetic resonance spectrum was measured, and showed 18H peaks assigned to the methyl group, the 1,1,1-trifluoropropyl groups, and the morpholino group at around δ 0.5 to 3.5 ppm, 7H peaks assigned to the methoxy group and the morpholino group at around δ 3.5 to 5.0 ppm, and 20H peaks assigned to the aromatic protons and alkene protons at around δ 5.0 to 9.0 ppm.

Furthermore, a ¹³C-nuclear magnetic resonance spectrum was measured, and showed peaks assigned to the carbons of the aromatic rings around at δ 110 to 160 ppm, peaks assigned to the carbons of the alkene around at δ 80 to 140 ppm, and peaks assigned to the carbons of the alkyls at δ 20 to 60 ppm.

### (Example 15)

### First Step

The reaction was carried out similarly to Example 1, except that diphenylamine was used in place of N-methylaniline, to synthesize a phenol derivative represented by the following formula (15-1).

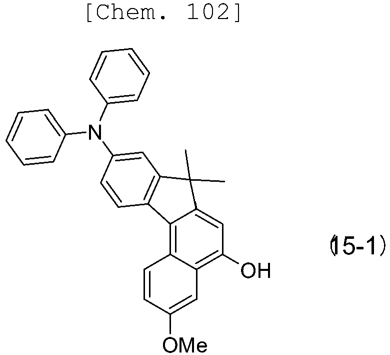

### Second Step

The reaction was carried out similarly to the fifth step in Example 1, except that the compound according to the formula (15-1) was used in place of the compound according to the formula (1-5), to give a photochromic compound represented by the following formula (15-2) in 82% yield.

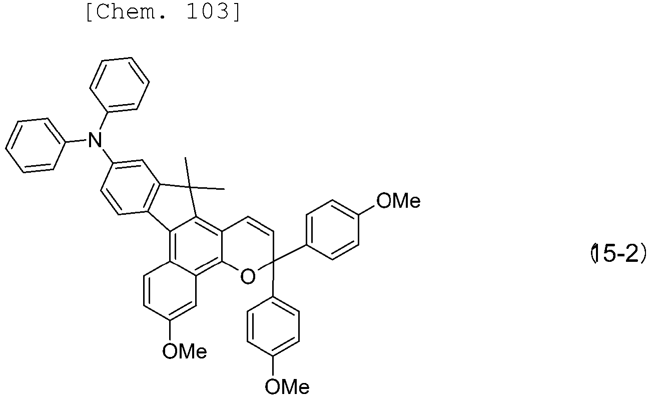

Elemental analysis values for the photochromic compound represented by the formula (15-2) were determined to be C: 83.15%, H: 5.85%, N: 2.00%, which are in good agreement with the calculated values for C₄₉H₄₁NO₄ of C: 83.14%, H: 5.84%, N: 1.98%.

A proton nuclear magnetic resonance spectrum was measured, and showed 6H peaks assigned to the methyl groups at around δ 0.5 to 3.5 ppm, 9H peaks assigned to the methoxy groups at around δ 3.5 to 5.0 ppm, and 26H peaks assigned to the aromatic protons and alkene protons at around δ 5.0 to 9.0 ppm.

Furthermore, a ¹³C-nuclear magnetic resonance spectrum was measured, and showed peaks assigned to the carbons of the aromatic rings around at δ 110 to 160 ppm, peaks assigned to the carbons of the alkene around at δ 80 to 140 ppm, and peaks assigned to the carbons of the alkyls at δ 20 to 60 ppm.

### (Example 16)

### First Step

The reaction was carried out similarly to Example 1, except that 4-bromobenzophenone was used in place of 4-bromo-4'-methoxybenzophenone in the first step, and dimethoxyacetone was used in place of acetone in the second step, to synthesize a phenol derivative represented by the following formula (16-1).

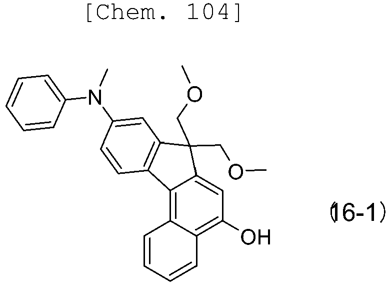

### Second Step

The reaction was carried out similarly to the fifth step in Example 1, except that the compound according to the formula (16-1) was used in place of the compound according to the formula (1-5), and a compound according to the following formula (16-2) was used in place of the compound according to the formula (1-6), to give a photochromic compound represented by the following formula (16-3) in 69% yield.

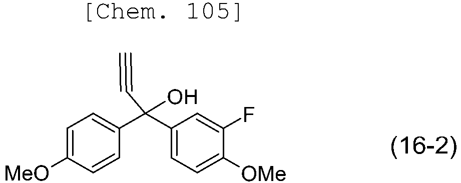

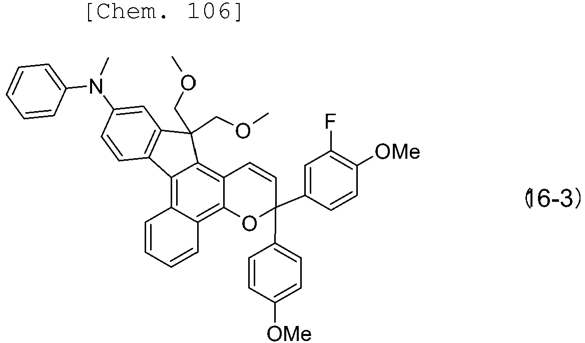

Elemental analysis values for the photochromic compound represented by the formula (16-3) were determined to be C: 77.92%, H: 5.82%, N: 2.04%, which are in good agreement with the calculated values for C₄₅H₄₀FNO₅ of C: 77.90%, H: 5.81%, N: 2.02%.

A proton nuclear magnetic resonance spectrum was measured, and showed 3H peaks assigned to the methyl group at around δ 0.5 to 3.5 ppm, 16H peaks assigned to the methoxy groups and the methoxymethyl groups at around δ 3.5 to 5.0 ppm, and 21H peaks assigned to the aromatic protons and alkene protons at around δ 5.0 to 9.0 ppm.

Furthermore, a ¹³C-nuclear magnetic resonance spectrum was measured, and showed peaks assigned to the carbons of the aromatic rings around at δ 110 to 160 ppm, peaks assigned to the carbons of the alkene around at δ 80 to 140 ppm, and peaks assigned to the carbons of the alkyls at δ 20 to 60 ppm.

### (Example 17)

### First Step

The reaction was carried out similarly to Example 1, except that 1,2,3,4-tetrahydroquinoline was used in place of N-methylaniline in the first step, and dipropyl ketone was used in place of acetone in the second step, to synthesize a phenol derivative represented by the following formula (17-1).

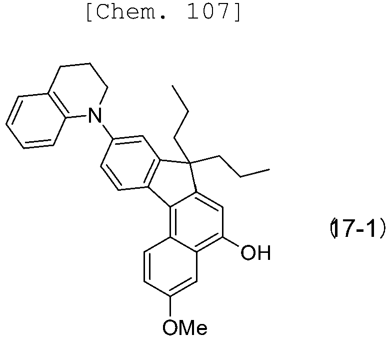

### Second Step

The reaction was carried out similarly to the fifth step in Example 1, except that the compound according to the formula (17-1) was used in place of the compound according to the formula (1-5), and a compound according to the following formula (17-2) was used in place of the compound according to the formula (1-6), to give a photochromic compound represented by the following formula (17-3) in 66% yield.

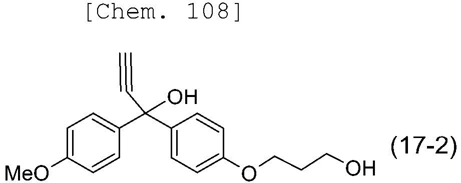

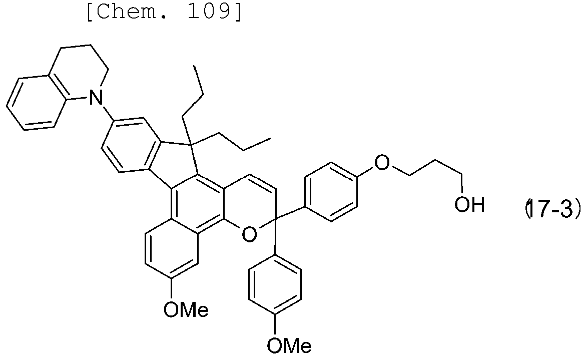

### Third Step

Dissolved were 3.8 g (5.0 mmol) of the photochromic compound according to the formula (17-3), and 0.75 g (11.0 mmol) of imidazole in 50 mL of dimethylformamide. After the cooling to achieve an internal temperature of 5°C or lower, a solution of 2.3 g (5.5 mmol) of [tris(trimethylsiloxy)silylethyl]dimethylchlorosilane in 10 mL of dimethylformamide was slowly added dropwise while maintaining the internal temperature of 5°C or lower. After the dropwise addition, the mixture was stirred for 12 hours during which the mixture was slowly warmed to room temperature. After the completion of the reaction, 50 mL of water and 100 mL of toluene were added, and liquid-liquid separation was performed. The solvent of the resulting organic layer was removed, and purification by chromatography on silica gel was carried out, to give a photochromic compound represented by the following formula (17-4) in 91% yield.

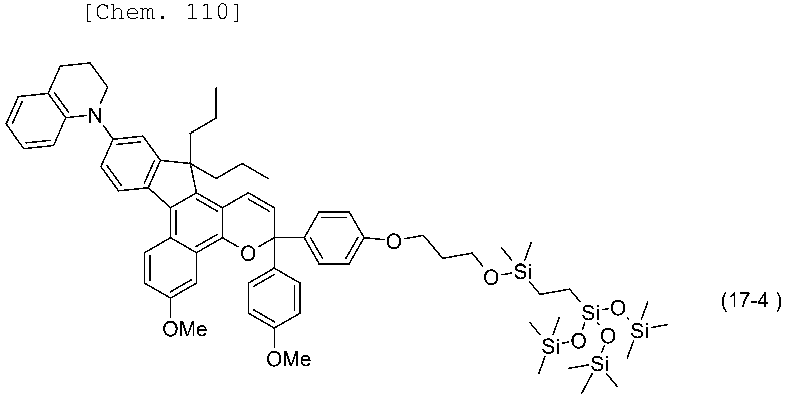

Elemental analysis values for the photochromic compound represented by the formula (17-4) were determined to be C: 67.74%, H: 7.77%, N: 1.22%, which are in good agreement with the calculated values for C₆₅H₈₉NO₈Si₅ of C: 67.72%, H: 7.78%, N: 1.21%.

A proton nuclear magnetic resonance spectrum was measured, and showed 59H peaks assigned to the propyl groups, the 1,2,3,4-tetrahydroquinoline ring group, the propyloxy group, and the [tris(trimethylsiloxy)silylethyl]silyl group at around δ 0.5 to 3.5 ppm, 10H peaks assigned to the methoxy groups and the propyloxy group at around δ 3.5 to 5.0 ppm, and 20H peaks assigned to the aromatic protons and alkene protons at around δ 5.0 to 9.0 ppm.

Furthermore, a ¹³C-nuclear magnetic resonance spectrum was measured, and showed peaks assigned to the carbons of the aromatic rings around at δ 110 to 160 ppm, peaks assigned to the carbons of the alkene around at δ 80 to 140 ppm, and peaks assigned to the carbons of the alkyls at δ 20 to 60 ppm.

### (Example 18)

### First Step

The reaction was carried out similarly to the first step in Example 1, except that benzothiomorpholine was used in place of N-methylaniline, and cyclododecanone was used in place of acetone, to synthesize a phenol derivative represented by the following formula (18-1).

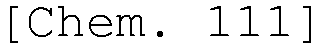

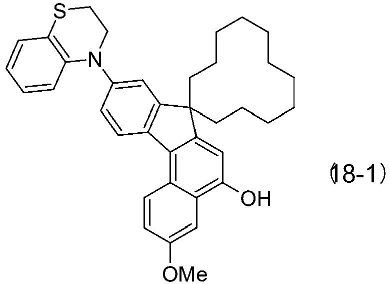

### Second Step

The reaction was carried out similarly to the fifth step in Example 1, except that the compound according to the formula (18-1) was used in place of the compound according to the formula (1-5), and a compound according to the following formula (18-2) was used in place of the compound according to the formula (1-6), to give a photochromic compound represented by the following formula (18-3) in 61% yield.

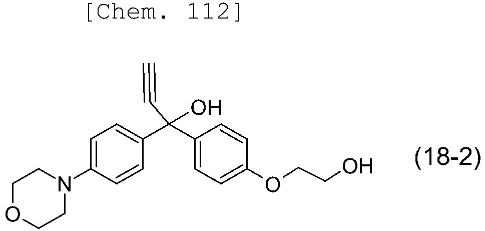

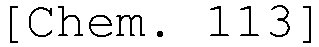

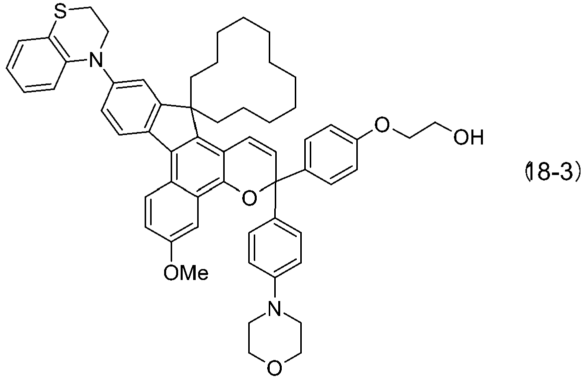

### Third Step

A compound represented by the following formula (18-4) synthesized from polyethylene glycol monomethyl ether having a number average molecular weight of 1000 with reference to the method described in Patent Document 11 was reacted with the photochromic compound according to the formula (18-3), to give a photochromic compound represented by the following formula (18-5) in 67% yield.

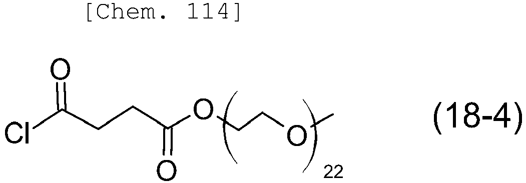

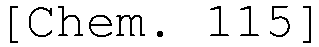

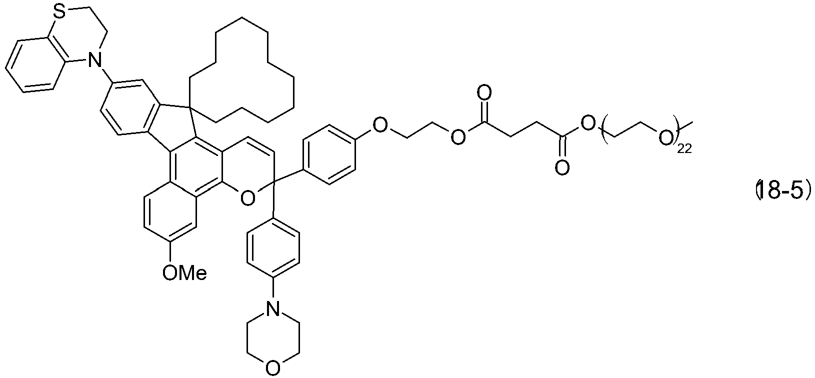

A proton nuclear magnetic resonance spectrum of the photochromic compound represented by the formula (18-5) was measured, and showed 32H peaks assigned to the cyclododecyl group, the succinic acid moiety, the morpholine group, and the benzothiomorpholino group at around δ 0.5 to 3.5 ppm, 104H peaks assigned to the methoxy group, the ethylenedioxy group, the polyethylene glycol chain group, the morpholino group, and the thiomorpholino group at around δ 3.5 to 5.0 ppm, and 20H peaks assigned to the aromatic protons and alkene protons at around δ 5.0 to 9.0 ppm.

### (Example 19)

### First Step

The reaction was carried out similarly to the first step in Example 17, except that benzomorpholine was used in place of 1,2,3,4-tetrahydroquinoline, and diethyl ketone was used in place of dipropyl ketone, to synthesize a phenol derivative represented by the following formula (19-1).

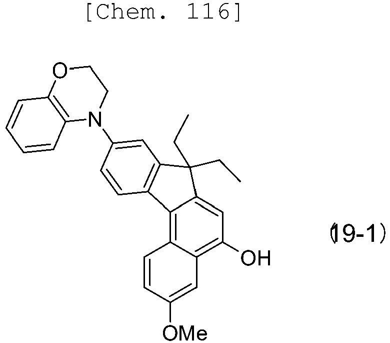

### Second Step

The reaction was carried out similarly to the second step in Example 18, except that the formula (19-1) was used in place of the compound according to the formula (18-1), to give a photochromic compound represented by the following formula (19-2) in 67% yield.

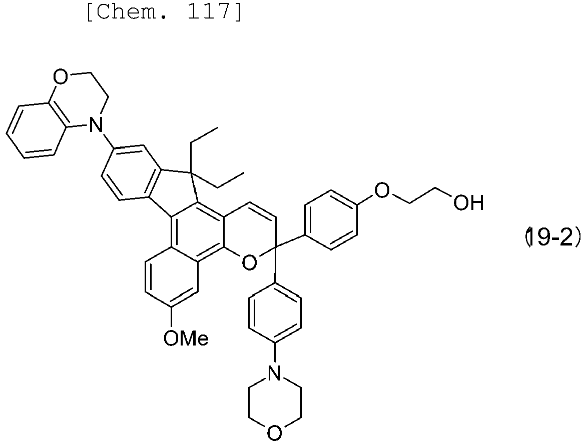

### Third Step

The reaction was carried out similarly to the third step in Example 18, except that sebacoyl dichloride was used in place of the compound according to the formula (18-4), to give a photochromic compound represented by the following formula (19-3) in 85% yield.

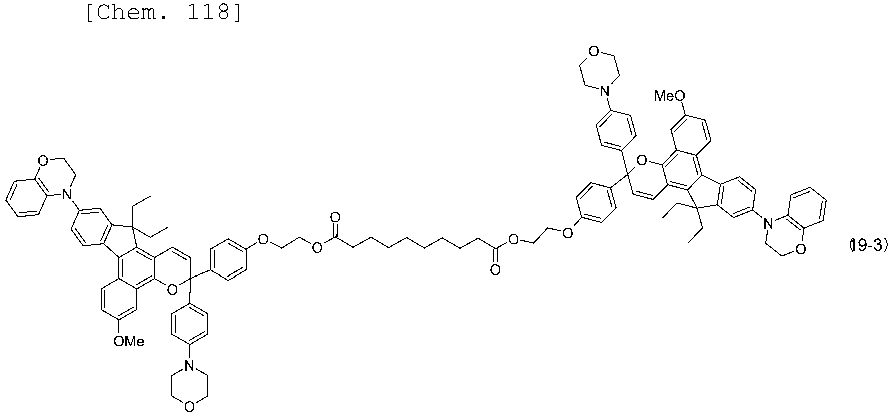

Elemental analysis values for the photochromic compound represented by the formula (19-3) were determined to be C: 77.33%, H: 6.59%, N: 3.22%, which are in good agreement with the calculated values for C₁₁₂H₁₁₄N₄O₁₄ of C: 77.30%, H: 6.60%, N: 3.22%.

A proton nuclear magnetic resonance spectrum was measured, and showed 48H peaks assigned to the ethyl groups, the sebacic acid group, the morpholino groups, and the benzomorpholino groups at around δ 0.5 to 3.5 ppm, 26H peaks assigned to the methoxy groups, the morpholino groups, the benzomorpholino groups, and ethylenedioxy groups at around δ 3.5 to 5.0 ppm, and 40H peaks assigned to the aromatic protons and alkene protons at around δ 5.0 to 9.0 ppm.

Furthermore, a ¹³C-nuclear magnetic resonance spectrum was measured, and showed peaks assigned to the carbons of the aromatic rings around at δ 110 to 160 ppm, peaks assigned to the carbons of the alkene around at δ 80 to 140 ppm, and peaks assigned to the carbons of the alkyls at δ 20 to 60 ppm.

### (Analytical Results of Phenol Derivatives)

Tables 1 and 2 summarize the analytical results of the phenol derivatives used in Examples 1 to 19.

### [Table 1]

**table 1**

| Examples | Calculated value | | | | Measured value | | | | 1H-NMR | |
|---|---|---|---|---|---|---|---|---|---|---|
| | C | H | N | S | C | H | N | S | | |
| 1 | 82.00 | 6.37 | 3.54 | 0.00 | 82.01 | 6.35 | 3.55 | 0.00 | δ0.5-5.0ppm | 12H |
| | | | | | | | | | δ5.0-9.0ppm | 13H |
| 2 | 75.89 | 7. 61 | 2.46 | 0.00 | 75.87 | 7.62 | 2.44 | 0.00 | δ0.5-5.0ppm | 31H |
| | | | | | | | | | δ5.0-9.0ppm | 12H |
| 3 | 68.11 | 5.21 | 2.34 | 0.00 | 68.10 | 5.23 | 2.34 | 0.00 | δ0.5-5.0ppm | 19H |
| | | | | | | | | | δ5.0-9.0ppm | 12H |
| 4 | 82.90 | 7.17 | 3.02 | 0.00 | 82.88 | 7.18 | 3.03 | 0.00 | δ0.5-5.0ppm | 20H |
| | | | | | | | | | δ5.0-9.0ppm | 12H |
| 5 | 78.95 | 6.26 | 3.47 | 0.00 | 78.93 | 6.25 | 3.47 | 0.00 | δ0.5-5.0ppm | 19H |
| | | | | | | | | | δ5.0-9.0ppm | 15H |
| 6 | 68.51 | 5.42 | 2.28 | 0.00 | 68.50 | 5.43 | 2.26 | 0.00 | δ0.5-5.0ppm | 21H |
| | | | | | | | | | δ5.0-9.0ppm | 12H |
| 7 | 74.59 | 6.94 | 2.35 | 5.38 | 74.61 | 6.93 | 2.35 | 5.40 | δ0.5-5.0ppm | 30H |
| | | | | | | | | | δ5.0-9.0ppm | 11H |
| 8 | 80.73 | 6.14 | 2.19 | 0.00 | 80.72 | 6.14 | 2.21 | 0.00 | δ0.5-5.0ppm | 16H |
| | | | | | | | | | δ5.0-9.0ppm | 23H |
| 9 | 77.76 | 6.92 | 2.75 | 6.29 | 77.77 | 6.92 | 2.73 | 6.30 | δ0.5-5.0ppm | 24H |
| | | | | | | | | | δ5.0-9.0ppm | 11H |
| 10 | 74.47 | 7.43 | 4.69 | 0.00 | 74.48 | 7.44 | 4.70 | 0.00 | δ0.5-5.0ppm | 32H |
| | | | | | | | | | δ5.0-9.0ppm | 12H |

### [Table 2]

**table 2**

| Examples | Calculated value | | | | Measured value | | | | 1H-NMR | |
|---|---|---|---|---|---|---|---|---|---|---|
| | C | H | N | S | C | H | N | S | | |
| 11 | 76.64 | 6.58 | 2.08 | 4.76 | 76.62 | 6.57 | 2.07 | 4.77 | δ0.5-5.0ppm | 31H |
| | | | | | | | | | δ5.0-9.0ppm | 13H |
| 12 | 84.35 | 7.77 | 2.40 | 0.00 | 84.36 | 7.78 | 2.40 | 0.00 | δ0.5-5.0ppm | 29H |
| | | | | | | | | | δ5.0-9.0ppm | 16H |
| 13 | 79.41 | 5.95 | 3.31 | 0.00 | 79.43 | 5.94 | 3.32 | 0.00 | δ0.5-5.0ppm | 13H |
| | | | | | | | | | δ5.0-9.0ppm | 12H |
| 14 | 67.01 | 5.10 | 2.44 | 0.00 | 66.99 | 5.09 | 2.42 | 0.00 | δ0.5-5.0ppm | 17H |
| | | | | | | | | | δ5.0-9.0ppm | 12H |
| 15 | 84.00 | 5.95 | 3.06 | 0.00 | 84.01 | 5.97 | 3.05 | 0.00 | δ0.5-5.0ppm | 9H |
| | | | | | | | | | δ5.0-9.0ppm | 18H |
| 16 | 79.03 | 6. 40 | 3.29 | 0.00 | 79.02 | 6.38 | 3.29 | 0.00 | δ0.5-5.0ppm | 13H |
| | | | | | | | | | δ5.0-9.0ppm | 14H |
| 17 | 82.98 | 7.39 | 2.93 | 0.00 | 83.00 | 7.37 | 2.93 | 0.00 | δ0.5-5.0ppm | 23H |
| | | | | | | | | | δ5.0-9.0ppm | 12H |
| 18 | 78.82 | 7.33 | 2.48 | 5. 69 | 78.83 | 7.33 | 2.39 | 5.68 | δ0.5-5.0ppm | 29H |
| | | | | | | | | | δ5.0-9.0ppm | 12H |
| 19 | 79.80 | 6.47 | 3.10 | 0.00 | 79.78 | 6.48 | 3.10 | 0.00 | δ0.5-5.0ppm | 17H |
| | | | | | | | | | δ5.0-9.0ppm | 12H |

### (Evaluation of Photochromic Plastic Lens Made by Coating Method for Physical Properties)

### (Example 20)

### (Preparation of Curable Composition)

First, the photochromic compound obtained in Example 1 above, a photoinitiator, and polymerizable compounds were mixed to obtain a curable composition.

As the polymerizable compounds, a blend of the following radically-polymerizable monomers in combination was used.

Polyethylene glycol dimethacrylate (average molecular weight: 736): 42 parts by mass
Polyethylene glycol dimethacrylate (average molecular weight: 536): 12 parts by mass
Trimethylolpropane trimethacrylate: 38 parts by mass
γ-Methacryloyloxypropyltrimethoxysilane: 2 parts by mass
Glycidyl methacrylate: 1 part by mass.

Note that in the curable composition, the photochromic compound obtained in Example 1 above was added to be 0.25 mmol taking the total amount of the radically-polymerizable monomers as 100 g. The following were used as additives.

Phenylbis(2,4,6-trimethylbenzoyl)phosphine oxide (photoinitiator: Omnirad 819): 0.3 parts by mass Ethylenebis(oxyethylene)bis[3-(5-tert-butyl-4-hydroxy-m-tolyl)propionate] (stabilizer, Irganox 245): 1 part by mass Bis(1,2,2,6,6-pentamethyl-4-piperidyl) sebacate: 3 parts by mass
Leveling agent from DuPont Toray Specialty Materials K.K. (L7001): 0.1 parts by mass.

Note that the above-mentioned additives are described with blending ratios taking a total of the radically-polymerizable monomers as 100 parts by mass.

### (Production of Optical Article)

Using this curable composition, a photochromic laminate was obtained by a lamination process described below with the polymerization described below being carried out.

First, a thiourethane plastic lens with a center thickness of 2 mm and a refractive index of 1.60 was prepared as an optical substrate. Note that the thiourethane plastic lens had been alkaline-etched beforehand in a 10% aqueous sodium hydroxide solution at 50°C for 5 minutes, and then thoroughly washed with distilled water.

A spin coater (1H-DX2, manufactured by MIKASA CO., LTD) was used to coat a surface of the above-mentioned plastic lens with a moisture-curable primer (product name: TR-SC-P, manufactured by Tokuyama Corporation) at a rotation speed of 70 rpm for 15 seconds, followed by at 1000 rpm for 10 seconds. Then, about 2 g of the photochromic curable composition obtained as above was spin-coated at a rotation speed of 60 rpm for 40 seconds, followed by at 600 rpm for 10 to 20 seconds so as to achieve a photochromic coating layer with a film thickness of 40 µm.

The lens having the surface coated with the photochromic curable composition (photochromic coating layer) was irradiated with light for 90 seconds in a nitrogen gas atmosphere using a metal halide lamp with an output of 200 mW/cm² to cure the coating film. The coating was then further heated at 110°C for 1 hour to produce a photochromic laminate with a photochromic layer.

### (Examples 21 to 38)

Photochromic laminates were produced using the photochromic compounds obtained in Examples 2 to 19 in the same manner as in Example 20.

Note that in Example 38, in which the compound 19-3 was used, the photochromic compound was added to be 0.25 mmol taking the total amount of the radically-polymerizable monomer as 200 g.

### (Comparative Examples 1 to 6)

Photochromic laminates were obtained in the same manner as in Example 20 using photochromic compounds represented by the following formulas (A) to (F).

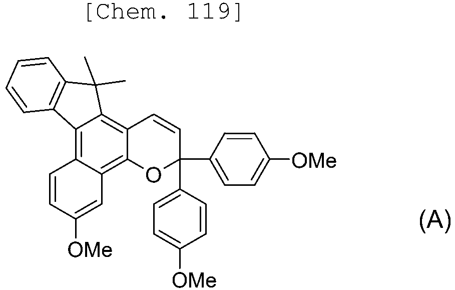

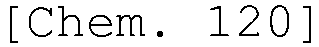

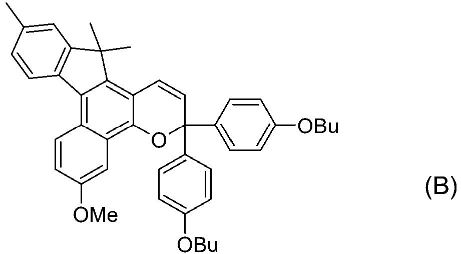

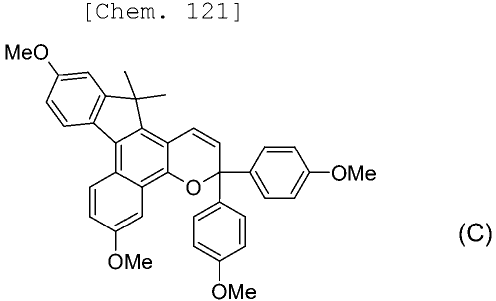

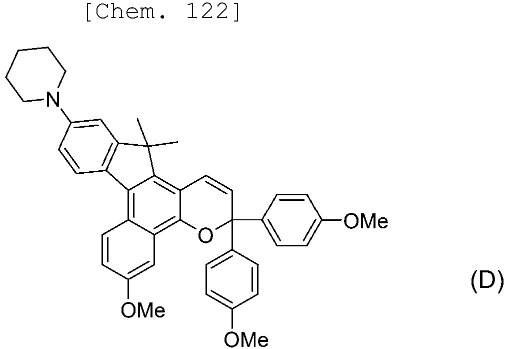

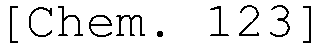

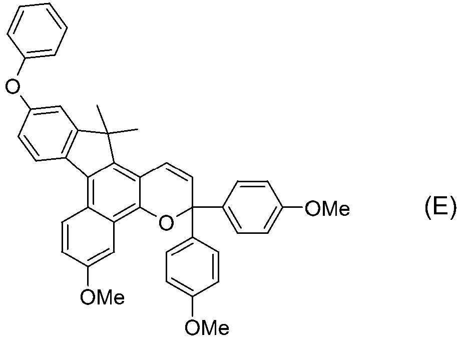

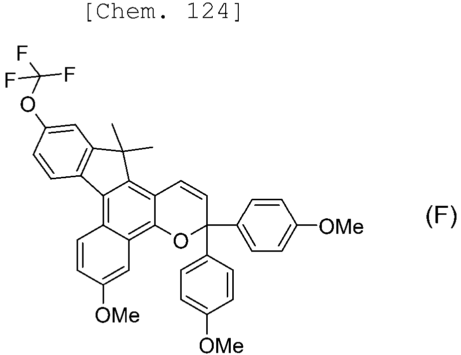

### <Evaluation Method>

The resulting photochromic laminates were evaluated by the methods described below.

### ·Photochromic Property

### [1] Maximum Absorption Wavelength (λmax):

This is the maximum absorption wavelength after color development determined by a spectrophotometer manufactured by Otsuka Electronics Co., Ltd. (instantaneous multichannel photo detector MCPD3000) and was used as an index of a color tone upon color development.

### [2] Color Developing Density at 23°C (A₂₃):

This is a difference between an absorbance {ε(240)} after irradiation with light at the above-mentioned maximum absorption wavelength at 23°C for 240 seconds and an absorbance ε(0) when not irradiated with light, and was used as an index of a color developing density. The higher this value is, the better photochromic property is.

### [3] Color Developing Density at 35°C (A₃₅):

This is a difference between an absorbance {ε(240)} after irradiation with light at first and second absorption wavelengths at 35°C for 240 seconds and an absorbance ε(0) when not irradiated with light, and was used as an index of a color developing density. The higher this value is, the better photochromic property is.

### [4] Color Development Ratio at High Temperature (A₃₅/A₂₃ × 100):

Color developing density at 35°C (A₃₅) /color developing density at 23°C (A₂₃) × 100(%). The higher this value is, the smaller the difference in color developing density between the higher temperature and the lower temperature is, and the smaller the temperature dependence is.

### [5] Fading Half-Life at 23°C [τ1/2(sec.)]:

This is the period of time required for the absorbance at a maximum absorption wavelength of a sample to decrease to 1/2 of {ε(240)-ε(0)}, when light irradiation is stopped after the sample had been irradiated with light at 23°C for 240 seconds, and was used as an index of a color fading rate. The shorter this period of time is, the higher the color fading rate is.

Table 3 summarizes the results of Examples 20, 32, and 34, and Comparative Examples 1 to 6, and Table 4 summarizes the results of Examples 21 to 31, 33, and 35 to 38. The relationship between the fading half-life at 23°C and the color development ratio at the higher temperature of the photochromic laminates according to Examples and Comparative Examples are shown in a graph in FIG. 1.

### [Table 3]

**table 3**

| | Compound No. | Maximum absorption wavelength (nm) | Color developing density at 23°C (-) | Color developing density at 35°C (-) | Color development ratio at high temperature (%) | Fading half-life at 23°C (sec) |
|---|---|---|---|---|---|---|
| Example 20 | Formula(1-7) | 453 | 0.86 | 0.64 | 76% | 144 |
| | | 616 | 1.06 | 0.81 | | 145 |
| Example 32 | Formula(13-2) | 453 | 0.87 | 0.63 | 73% | 112 |
| | | 608 | 1.02 | 0.75 | | 113 |
| Example 34 | Formula(15-2) | 456 | 0.92 | 0.67 | 73% | 119 |
| | | 619 | 1.07 | 0.79 | | 120 |
| Comparative Example 1 | Formula(A) | 449 | 0.69 | 0.44 | 63% | 95 |
| | | 569 | 0.87 | 0.55 | | 95 |
| Comparative Example 2 | Formula(B) | 429 | 0.69 | 0.48 | 68% | 120 |
| | | 579 | 0.96 | 0.65 | | 120 |
| Comparative Example 3 | Formula(C) | 433 | 0.71 | 0.48 | 71% | 157 |
| | | 593 | 1.04 | 0.74 | | 158 |
| Comparative Example 4 | Formula(D) | 444 | 0.78 | 0.57 | 75% | 209 |
| | | 617 | 1.14 | 0.86 | | 210 |
| Comparative Example 5 | Formula(E) | 431 | 0.71 | 0.46 | 65% | 97 |
| | | 579 | 0.96 | 0.63 | | 97 |
| Comparative Example 6 | Formula(F) | 449 | 0.62 | 0.37 | 60% | 58 |
| | | 565 | 0.75 | 0.45 | | 59 |

### [Table 4]

**table 4**

| | Compound No. | Maximum absorption wavelength (nm) | Color developing density at 23°C (-) | Color developing density at 35°C (-) | Color development ratio at high temperature (%) | Fading half-life at 23°C (sec) |
|---|---|---|---|---|---|---|
| Example 21 | Formula(2-7) | 475 | 1.43 | 1.06 | 75% | 144 |
| | | 608 | 0.83 | 0.62 | | 144 |
| Example 22 | Formula(3-3) | 456 | 0.91 | 0.69 | 77 | 173 |
| | | 581 | 1.33 | 1.02 | | 173 |
| Example 23 | Formula(4-3) | 482 | 0.68 | 0.48 | 71% | 103 |
| | | 626 | 1.14 | 0.81 | | 104 |
| Example 24 | Formula (5-4) | 463 | 0.72 | 0.53 | 75% | 99 |
| | | 596 | 1.18 | 0.88 | | 101 |
| Example 25 | Formula (6-3) | 456 | 0.37 | 0.22 | 59% | 20 |
| | | 598 | 0.77 | 0.46 | | 20 |
| Example 26 | Formula (7-4) | 494 | 1.30 | 0.90 | 69% | 70 |
| | | 622 | 0.71 | 0.49 | | 70 |
| Example 27 | Formula (8-2) | 452 | 0.75 | 0.52 | 69% | 50 |
| | | 610 | 0.99 | 0.69 | | 50 |
| Example 28 | Formula (9-2) | 450 | 1.01 | 0.74 | 73% | 125 |
| | | 609 | 0.90 | 0.66 | | 125 |
| Example 29 | Formula (10-3) | 475 | 1.24 | 0.83 | 67% | 71 |
| | | 594 | 1.01 | 0.68 | | 71 |
| Example 30 | Formula (11-4) | 505 | 0.51 | 0.31 | 59% | 24 |
| | | 620 | 0.51 | 0.30 | | 24 |
| Example 31 | Formula (12-5) | 508 | 0.86 | 0.64 | 74% | 118 |
| | | 624 | 1.49 | 1.11 | | 117 |
| Example 33 | Formula (14-3) | 499 | 0.73 | 0.51 | 71% | 73 |
| | | 628 | 1.13 | 0.80 | | 71 |
| Example 35 | Formula (16-3) | 458 | 0.53 | 0.36 | 68% | 80 |
| | | 593 | 0.89 | 0.61 | | 80 |
| Example 36 | Formula (17-3) | 451 | 0.85 | 0.61 | 73% | 88 |
| | | 613 | 0.96 | 0.70 | | 89 |
| Example 37 | Formula(18-4) | 485 | 0.57 | 0.37 | 65% | 69 |
| | | 621 | 0.98 | 0.64 | | 69 |
| Example 38 | Formula (19-3) | 483 | 0.55 | 0.34 | 64% | 50 |
| | | 629 | 0.88 | 0.57 | | 50 |

### <Evaluation of Photochromic Layer Made by Binder Method for Physical Properties>

### (Example 39)

A binder sheet was made according to the following method. The binder sheet had a first optical sheet, a first adhesion layer (first coating film), a photochromic layer (third coating film), a second adhesion layer (second coating film), and a second optical sheet laminated in this order. As the first and second optical sheets, polycarbonate sheets with a thickness of 400 µm were used.

### (Preparation of Composition for Photochromic Layer Formation)

Into a 2 L 4-necked flask equipped with a stirring blade, a condenser, a thermometer, and a nitrogen gas inlet tube were charged 315 parts by mass of polycarbonate diol with a number average molecular weight of 1000, 100 parts by mass of isophorone diisocyanate, and 72 parts by mass of toluene, and reacted under a nitrogen atmosphere at 100°C for 7 hours. Thus, a urethane prepolymer with a terminal isocyanate group was synthesized. After the urethane prepolymer reaction was completed, the resulting reaction solution was cooled to about 0°C and dissolved into 205 parts by mass of tertiary butyl alcohol and 382 parts by mass of diethyl ketone, and then the temperature of the resulting liquid was kept at 0°C. Next, a mixed solution of 21.3 parts by mass of bis-(4-aminocyclohexyl)methane serving as a chain extender and 20 parts by mass of diethyl ketone was added dropwise thereto within 30 minutes and reacted at 0°C for 1 hour. Then, 8.1 parts by mass of 1,2,2,6,6-pentamethyl-4-aminopiperidine was further added dropwise thereto and reacted at 0°C for 1 hour to obtain a solution of a terminally non-reactive urethaneurea resin in diethyl ketone.

One hundred parts by mass of the resulting solution of a terminally non-reactive urethaneurea resin, the photochromic compound according to Example 1 (the formula (1-7)), 6.3 parts by mass of an isomer mixture of 4,4'-methylenebis(cyclohexylisocyanate) (polyisocyanate compound), and 0.4 parts by mass of ethylenebis(oxyethylene)bis[3-(5-tert-butyl-4-hydroxy-m-tolyl)propionate] serving as an antioxidant and 0.06 parts by mass of L-7001 from DuPont Toray Specialty Materials K.K. serving as a surfactant were added, and stirred and mixed at room temperature to obtain a composition for photochromic layer formation.

Note that the photochromic compound was added to be 0.25 mmol relative to 100 parts by mass of the terminally non-reactive urethaneurea resin.

### (Preparation of Composition for Adhesion Layer)

A 5 L separable flask (4-necked) equipped with a stirring blade, a condenser, a thermometer, and a nitrogen gas inlet tube was provided, and 400 parts by mass of polycarbonate diol with a number average molecular weight of 1000, 175 parts by mass of isophorone diisocyanate, and 120 parts by mass of toluene were charged into the flask and reacted under a nitrogen atmosphere at 110°C for 7 hours. Thus, a urethane prepolymer with a terminal isocyanate group was synthesized. After the urethane prepolymer reaction was completed, the resulting reaction solution was cooled to about 20°C and dissolved into 2500 parts by mass of propylene glycol monomethyl ether, and then the temperature of the resulting liquid was kept at 20°C. Next, 60 parts by mass of isophoronediamine serving as a chain extender was added dropwise to the liquid and allowed to react at 20°C for 1 hour. Then, 3 parts by mass of n-butylamine was further added dropwise to the liquid and allowed to react at 20°C for 1 hour, to obtain a solution of a terminally non-reactive urethaneurea resin in propylene glycol-monomethyl ether.

To 500 parts by mass of the resulting solution of a terminally non-reactive urethane urea resin was added 0.2 parts by mass of L-7001 from DuPont Toray Specialty Materials K.K. serving as a surfactant, and the resulting mixture was stirred and mixed at room temperature to obtain a composition for adhesive layer.

### (Production of Binder Sheet)

The composition for adhesive layer was applied to one main surface of a first optical sheet using a coater (from Tester Sangyo Co., Ltd.) at a coating rate of 0.5 m/min and dried at a dry temperature of 110°C for 3 minutes, to obtain the first optical sheet having a first coating film with a thickness of 5 µm. The composition for adhesive layer was applied to one main surface of a second optical sheet in the same manner, to obtain the second optical sheet having a second coating film.

Then, the composition for photochromic layer formation was applied to an OPP film (oriented polypropylene film) having a thickness of 50 µm using a coater (manufactured by TESTER SANGYO CO. LTD.) at a coating rate of 0.3 m/min and dried at a drying temperature of 100°C for 5 minutes. Thus, a third coating film was obtained. Then, the third coating film and the first optical sheet were laminated together so that the third coating film was in contact with the first coating film. The OPP film was peeled off from this structure, and the second optical sheet and the third coating film were laminated together so that the other main surface exposed of the third coating film was in contact with the second coating film. Subsequently, the resulting laminate was allowed to stand at 40°C for 24 hours under vacuum, then heat-treated at 110°C for 60 minutes, then subjected a humidification treatment at 60°C and 100% RH for 24 hours, and finally allowed to stand at 40°C for 24 hours under vacuum, to obtain a binder sheet. The same evaluation as in Example 23 was carried out on the resulting binder sheet. The results are shown in Table 5.

### (Example 40 to Example 49, and Comparative Examples 7 to 10)

Binder sheets were formed using the photochromic compounds shown in Table 5 in the same manner as in Example 39.

### [Table 5]

**table 5**

| | Compound No. | Maximum absorption wavelength (nm) | Color developing density at 23°C (-) | Color developing density at 35°C (-) | Color development ratio at high temperature (%) | Fading half-life at 23°C (sec) |
|---|---|---|---|---|---|---|
| Example 39 | Formula (1-7) | 453 | 0.88 | 0.63 | 74% | 162 |
| | | 613 | 1.08 | 0.80 | | 162 |
| Example 40 | Formula (2-7) | 475 | 1.47 | 1.06 | 72% | 164 |
| | | 609 | 0.85 | 0.61 | | 165 |
| Example 41 | Formula (3-3) | 456 | 0.94 | 0.68 | 75% | 192 |
| | | 580 | 1.34 | 1.00 | | 194 |
| Example 42 | Formula (4-3) | 482 | 0.68 | 0.47 | 69% | 112 |
| | | 627 | 1.16 | 0.80 | | 113 |
| Example 43 | Formula (7-4) | 492 | 1.33 | 0.89 | 67% | 74 |
| | | 622 | 0.72 | 0.49 | | 74 |
| Example 44 | Formula (8-2) | 452 | 0.77 | 0.51 | 68% | 58 |
| | | 609 | 1.01 | 0.69 | | 59 |
| Example 45 | Formula (9-2) | 450 | 1.04 | 0.73 | 70% | 135 |
| | | 607 | 0.91 | 0.64 | | 135 |
| Example 46 | Formula (12-5) | 510 | 0.87 | 0.61 | 72% | 129 |
| | | 625 | 1.51 | 1.08 | | 128 |
| Example 47 | Formula (13-2) | 456 | 0.90 | 0.63 | 71% | 122 |
| | | 608 | 1.04 | 0.74 | | 123 |
| Example 48 | Formula (15-2) | 456 | 0.93 | 0.67 | 72% | 130 |
| | | 616 | 1.08 | 078 | | 131 |
| Example 49 | Formula (16-3) | 459 | 0.55 | 0.37 | 68% | 90 |
| | | 595 | 0.90 | 0.61 | | 90 |
| Comparative Example 7 | Formula(A) | 427 | 0.71 | 0.46 | 64% | 100 |
| | | 569 | 0.90 | 0.58 | | 101 |
| Comparative Example 8 | Formula(B) | 429 | 0.75 | 0.50 | 68% | 131 |
| | | 580 | 0.98 | 0.67 | | 132 |
| Comparative Example 9 | Formula(C) | 434 | 0.73 | 0.51 | 71% | 175 |
| | | 594 | 1.06 | 0.75 | | 176 |
| Comparative Example 10 | Formula(D) | 445 | 0.80 | 0.58 | 75% | 235 |
| | | 617 | 1.17 | 0.88 | | 235 |

### <Evaluation of Photochromic Cured Product Made by Kneading Method for Physical Properties>

### (Example 50)

### (Preparation of Curable Composition)

First, the photochromic compound obtained in Example 1, additives, and polymerizable compounds were mixed to obtain a curable composition. As the polymerizable compounds, a blend of the following polymerizable monomers in combination was used.
1,3-Bis(isocyanatomethyl)cyclohexane: 36.7 parts by mass Pentaerythritol tetrakis(3-mercaptopropionate): 39.4 parts by mass
Polyoxyethylene polyoxypropylene lauryl ether (WONDERSURF 140 from Aoki Oil Industrial Co., Ltd.): 17.4 parts by mass 1-Decanethiol: 2.8 parts by mass
RX-1 prepared according to the method described in Patent Document 12: 3.8 parts by mass
Note that in the curable composition, the photochromic compound was added to be 0.106 mmol taking the total amount of the polymerizable monomers as 100 g.

The following additives were used. Dimethyltin dichloride: 0.05 parts by mass
Irganox 245 from BASF Japan Ltd.: 0.1 parts by mass
2-Ethylhexyl 4-methoxycinnamate: 0.6 parts by mass

### (Production of Cured Product)

The prepared curable composition was sufficiently degassed, and then injected into a glass mold provided with a gap of 1 mm, and the curable composition was polymerized via casting polymerization. The polymerization was carried out using an air furnace over a period of 18 hours, with the temperature being gradually increased from 27°C to 120°C. After the polymerization, the cured product was removed from the glass mold, to obtain photochromic cured product having a thickness of 1 mm. The resulting photochromic cured product was evaluated in the same manner as in Example 23. The results are shown in Table 6.

(Examples 51 to 60, and Comparative Examples 11 to 14) Photochromic cured products were formed using the photochromic compound shown in Table 6 in the same manner as in Example 50. Table 6 summarizes the results.

### [Table 6]

**table 6**

| | Compound No. | Maximum absorption wavelength (nm) | Color developing density at 23°C (-) | Color developing density at 35°C (-) | Color development ratio at high temperature (%) | Fading half-life at 23°C (sec) |
|---|---|---|---|---|---|---|
| Example 50 | Formula (1-7) | 455 | 0.89 | 0.67 | 77% | 200 |
| | | 621 | 1.09 | 0.84 | | 200 |
| Example 51 | Formula (2-7) | 478 | 1.50 | 1.12 | 77% | 212 |
| | | 615 | 0.86 | 0.66 | | 211 |
| Example 52 | Formula (3-3) | 458 | 0.94 | 0.73 | 78% | 245 |
| | | 588 | 1.36 | 1.06 | | 244 |
| Example 53 | Formula (4-3) | 484 | 0.69 | 0.49 | 72% | 143 |
| | | 631 | 1.17 | 0.84 | | 143 |
| Example 54 | Formula (7-4) | 497 | 1.35 | 0.95 | 70% | 100 |
| | | 625 | 0.72 | 0.50 | | 99 |
| Example 55 | Formula (8-2) | 455 | 0.78 | 0.55 | 69% | 70 |
| | | 618 | 1.02 | 0.70 | | 70 |
| Example 56 | Formula (9-2) | 453 | 1.05 | 0.78 | 74% | 177 |
| | | 614 | 0.92 | 0.69 | | 178 |
| Example 57 | Formula (12-5) | 510 | 0.88 | 0.67 | 76% | 170 |
| | | 632 | 1.52 | 1.15 | | 171 |
| Example 58 | Formula (13-2) | 455 | 0.90 | 0.66 | 75% | 159 |
| | | 616 | 1.04 | 0.78 | | 160 |
| Example 59 | Formula (15-2) | 458 | 0.95 | 0.70 | 75% | 170 |
| | | 622 | 1.10 | 0.83 | | 170 |
| Example 60 | Formula (16-3) | 460 | 0.56 | 0.38 | 71% | 120 |
| | | 599 | 0.92 | 0.65 | | 119 |
| Comparative Example 11 | Formula (A) | 432 | 0.72 | 0.48 | 67% | 127 |
| | | 578 | 0.91 | 0.61 | | 128 |
| Comparative Example 12 | Formula (B) | 432 | 0.76 | 0.53 | 70% | 169 |
| | | 586 | 0.98 | 0.69 | | 168 |
| Comparative Example 13 | Formula (C) | 436 | 0.74 | 0.54 | 76% | 220 |
| | | 600 | 1.07 | 0.81 | | 220 |
| Comparative Example 14 | Formula (D) | 446 | 0.80 | 0.62 | 80% | 302 |
| | | 625 | 1.18 | 0.94 | | 303 |

### Example 61

### First Step

To a 1 L 4-necked flask were charged 86 g (645.0 mmol) of aluminum chloride and 500 mL of dichloromethane, and cooled on ice. Slowly added dropwise was 121.5 g (663.0 mmol) of N-methyldiphenylamine dissolved in 100 mL of dichloromethane, and the mixture was stirred for 2 hours. To the mixture, 80.6 g (323.0 mmol) of 3-bromo-4-methoxybenzoyl chloride was slowly added dropwise while maintaining an internal temperature of 5°C or lower. The mixture was stirred for 3 hours with the internal temperature being 0-5°C, and then poured into 2000 mL of ice water, and the total mixture was warmed to room temperature. Liquid-liquid separation was performed, then 2000 mL of water was added, and washing with water was carried out. The washing with water was repeated until the pH of the aqueous layer reached 6-7. The resulting organic layer was concentrated, and the residue was subjected to recrystallization in 780 mL of methanol, to give a compound represented by the following formula (61-1) in 70% yield.

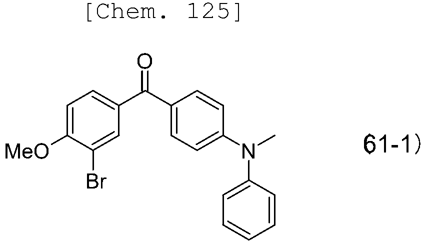

### Second Step

The reaction was carried out similarly to the first step in Example 1, except that the compound according to the formula (61-1) was used in place of 4-bromo-4'-methoxybenzophenone, to give a carboxylic acid compound represented by the following formula (61-2).

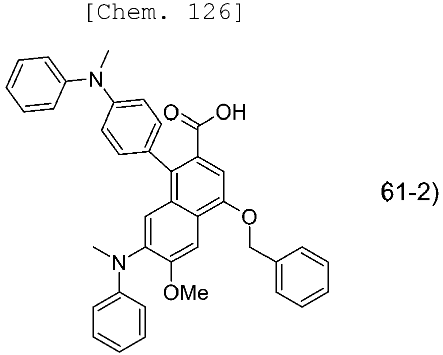

### Third Step

The reaction was carried out similarly to the second step in Example 2, except that the compound according to the formula (61-2) was used in place of the compound according to the formula (2-1), and the reaction was carried out similarly to the sixth step in Example 2, except that iodopropane was used, in place of 1-bromo-4-methoxybutane, to give a compound represented by the following formula (61-3).

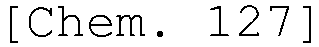

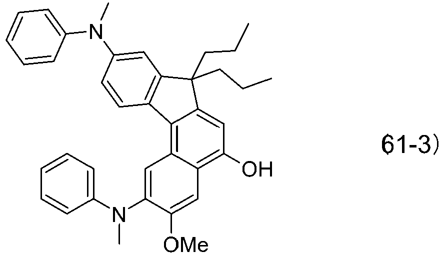

### Fourth Step

The reaction was carried out similarly to the fifth step in Example 1, except that the compound according to the formula (61-3) was used in place of the compound according to the formula (1-5), and the compound according to the formula (5-3) was used in place of the compound according to the formula (1-6), to give a photochromic compound represented by the following formula (61-4) in 72% yield.

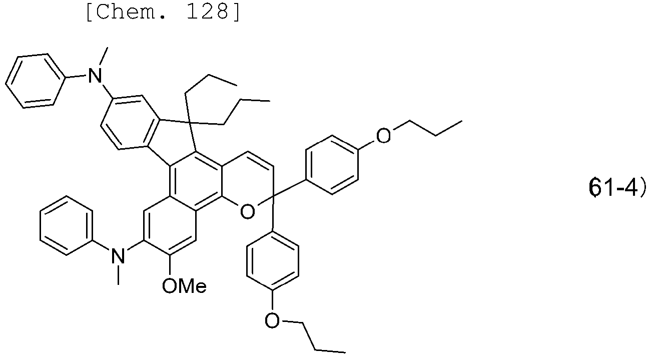

Elemental analysis values for the photochromic compound represented by the formula (61-4) were determined to be C: 82.12%, H: 7.26%, N: 3.23%, which are in good agreement with the calculated values for C₅₉H₆₂N₂O₄ of C: 82.10%, H: 7.24%, N: 3.25%. A proton nuclear magnetic resonance spectrum was measured, and showed 30H peaks assigned to the methyl groups, the propyl groups, and the propyloxy groups at around δ 0.5 to 3.5 ppm, 7H peaks assigned to the propyloxy groups and the methoxy group at around δ 3.5 to 5.0 ppm, and 25H peaks assigned to the aromatic protons and alkene protons at around δ 5.0 to 9.0 ppm. Furthermore, a ¹³C-nuclear magnetic resonance spectrum was measured, and showed peaks assigned to the carbons of the aromatic rings around at δ 110 to 160 ppm, peaks assigned to the carbons of the alkene around at δ 80 to 140 ppm, and peaks assigned to the carbons of the alkyls at δ 20 to 60 ppm.

### Example 62

### First Step

The reaction was carried out similarly to the first step in Example 61, except that triphenylamine was used in place of N-methyldiphenylamine, to give a compound represented by the following formula (62-1).

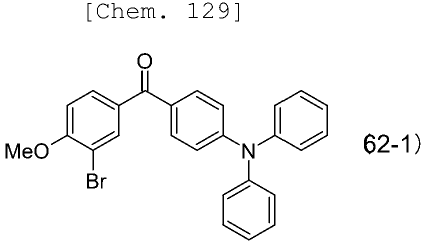

### Second Step

The reaction was carried out similarly to the second step in Example 61, except that the compound according to the formula (62-1) was used in place of the compound according to the formula (61-1), to give a carboxylic acid compound represented by the following formula (62-2).

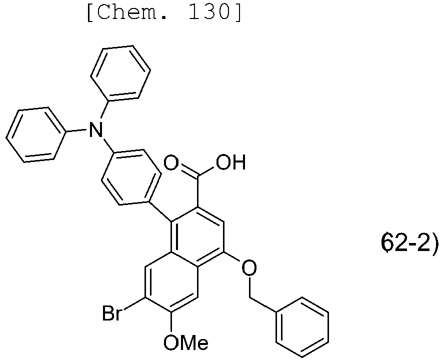

### Third Step

The reaction was carried out similarly to the first step in Example 12, except that the compound according to the formula (62-2) was used in place of the compound according to the formula (12-1), and 2,4,6-trimethoxyphenylboronic acid was used in place of 4-methoxyphenylboronic acid, to give a compound according to the following formula (62-3).

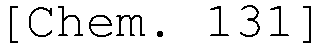

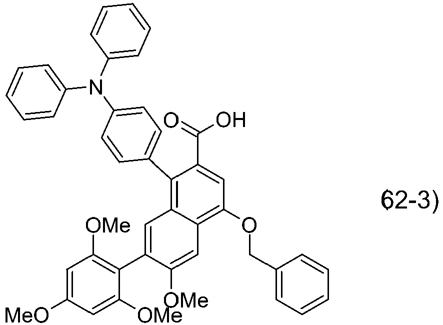

### Fourth Step

The reaction was carried out similarly to Example 2, except that the compound according to the formula (62-3) was used in place of the compound according to the formula (2-1) in the first step, and 1-bromo-2-[2-(2-methoxyethoxy)ethoxy]ethane was used in place of 1-bromo-4-methoxybutane in the sixth step, to give a phenol derivative represented by the following formula (62-4).

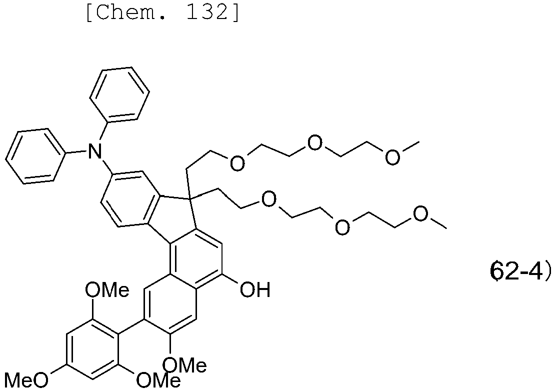

### Fifth Step

The reaction was carried out similarly to the fifth step in Example 1, except that the compound according to the formula (62-4) was used in place of the compound according to the formula (1-5), to give a photochromic compound represented by the following formula (62-5) in 62% yield.

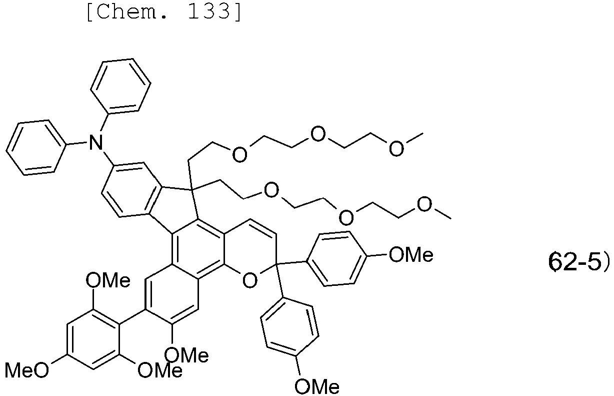

Elemental analysis values for the photochromic compound represented by the formula (62-5) were determined to be C: 73.88%, H: 6.62%, N: 1.22%, which are in good agreement with the calculated values for C₇₁H₇₇NO₁₃ of C: 73.86%, H: 6.64%, N: 1.23%. A proton nuclear magnetic resonance spectrum was measured, and showed 4H peaks assigned to the 2-[2-(2-methoxyethoxy)ethoxy]ethyloxy group at around δ 0.5 to 3.5 ppm, 44H peaks assigned to the 2-[2-(2-methoxyethoxy)ethoxy]ethyloxy group and the methoxy groups at around δ 3.5 to 5.0 ppm, and 27H peaks assigned to the aromatic protons and alkene protons at around δ 5.0 to 9.0 ppm. Furthermore, a ¹³C-nuclear magnetic resonance spectrum was measured, and showed peaks assigned to the carbons of the aromatic rings around at δ 110 to 160 ppm, peaks assigned to the carbons of the alkene around at δ 80 to 140 ppm, and peaks assigned to the carbons of the alkyls at δ 20 to 60 ppm.

### Example 63

### First Step

The reaction was carried out similarly to Example 62, except that the compound according to the formula (61-1) was used in place of the compound according to the formula (62-1) in the second step, 4-trifluoromethylphenylboronic acid was used in place of 2,4,6-trimethoxyphenylboronic acid in the third step, and iodopropane was used in place of 1-bromo-2-[2-(2-methoxyethoxy)ethoxy]ethane in the fourth step, to give a phenol derivative represented by the following formula (63-1).

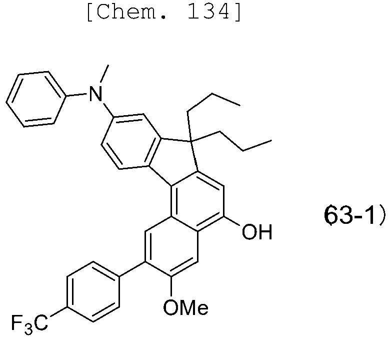

### Second Step

The reaction was carried out similarly to the fifth step in Example 1, except that the compound according to the formula (63-1) was used in place of the compound according to the formula (1-5), and the compound according to the formula (3-2) was used in place of the compound according to the formula (1-6), to give a photochromic compound represented by the following formula (63-2) in 69% yield.

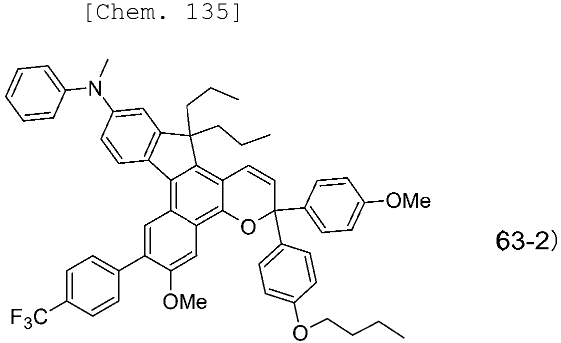

Elemental analysis values for the photochromic compound represented by the formula (63-2) were determined to be C: 78.41%, H: 6.34%, N: 1.59%, which are in good agreement with the calculated values for C₅₈H₅₆F₃NO₄ of C: 78.44%, H: 6.36%, N: 1.58%. A proton nuclear magnetic resonance spectrum was measured, and showed 24H peaks assigned to the propyl groups, the butyloxy group, and the methyl group at around δ 0.5 to 3.5 ppm, 8H peaks assigned to the butyloxy group and the methoxy groups at around δ 3.5 to 5.0 ppm, and 24H peaks assigned to the aromatic protons and alkene protons at around δ 5.0 to 9.0 ppm. Furthermore, a ¹³C-nuclear magnetic resonance spectrum was measured, and showed peaks assigned to the carbons of the aromatic rings around at δ 110 to 160 ppm, peaks assigned to the carbons of the alkene around at δ 80 to 140 ppm, and peaks assigned to the carbons of the alkyls at δ 20 to 60 ppm.

### Example 64

### First Step

The reaction was carried out similarly to the first step in Example 1, except that the compound according to the formula (62-2) was used in place of the compound according to the formula (1-1), and 4-trifluoromethylpiperidine was used in place of N-methylaniline, to synthesize a compound represented by the following formula (64-1).

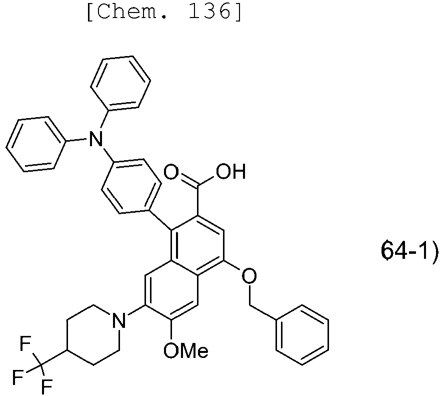

### Second Step

The reaction was carried out similarly to Example, except that the compound according to the formula (64-1) was used in place of the compound according to the formula (1-2) in the second step, and spiro[5.5]undecan-3-one was used in place of acetone in the third step, to synthesize a compound represented by the following formula (64-2).

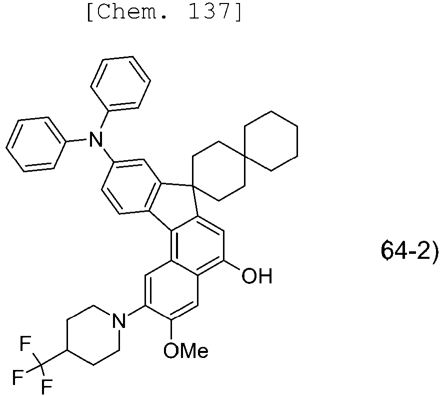

### Third Step

The reaction was carried out similarly to the fifth step in Example 1, except that the compound according to the formula (64-2) was used in place of the compound according to the formula (1-5), and the compound according to the formula (6-2) was used in place of the compound according to the formula (1-6), to give a photochromic compound represented by the following formula (64-3) in 61% yield.

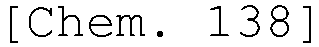

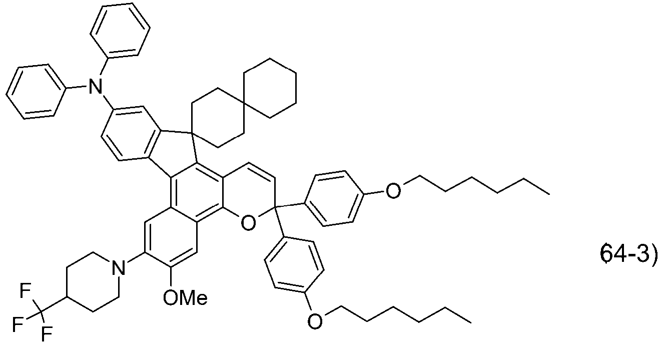

Elemental analysis values for the photochromic compound represented by the formula (64-3) were determined to be C: 79.16%, H: 7.34%, N: 2.52%, which are in good agreement with the calculated values for C₇₃H₈₁F₃N₂O₄ of C: 79.17%, H: 7.37%, N: 2.53%. A proton nuclear magnetic resonance spectrum was measured, and showed 49H peaks assigned to the spiro[5.5]undecane ring group, the hexyloxy groups, and the 4-trifluoropiperidine group at around δ 0.5 to 3.5 ppm, 7H peaks assigned to the hexyloxy groups and the methoxy group at around δ 3.5 to 5.0 ppm, and 25H peaks assigned to the aromatic protons and alkene protons at around δ 5.0 to 9.0 ppm. Furthermore, a ¹³C-nuclear magnetic resonance spectrum was measured, and showed peaks assigned to the carbons of the aromatic rings around at δ 110 to 160 ppm, peaks assigned to the carbons of the alkene around at δ 80 to 140 ppm, and peaks assigned to the carbons of the alkyls at δ 20 to 60 ppm.

### Example 65

### First Step

The reaction was carried out similarly to Example 61, except that the compound according to the formula (62-1) was used in place of the compound according to the formula (61-1) in the first step, N-methyl-4-(trifluoromethyl)aniline was used in place of N-methylaniline, and 1-butoxy-2-bromoethane was used in place of iodopropane in the third step, to give a phenol derivative represented by the following formula (65-1).

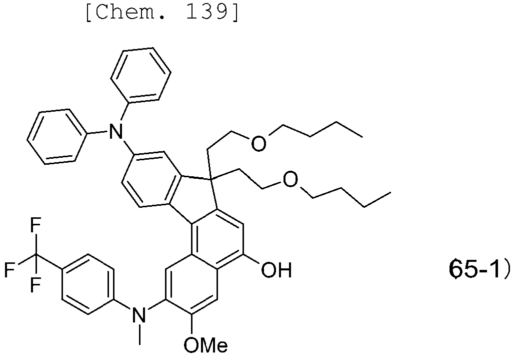

### Second Step

The reaction was carried out similarly to the fifth step in Example 1, except that the compound according to the formula (65-1) was used in place of the compound according to the formula (1-5), and the compound according to the formula (7-3) was used in place of the compound according to the formula (1-6), to give a photochromic compound represented by the following formula (65-2) in 68% yield.

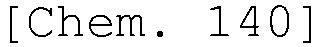

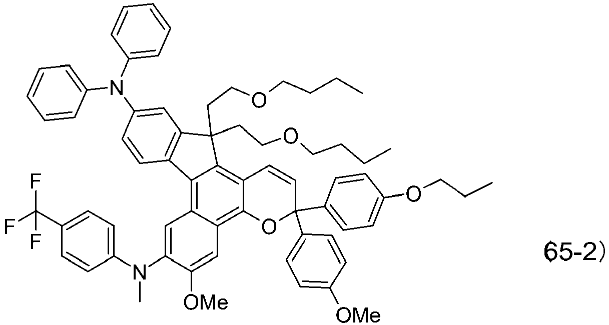

Elemental analysis values for the photochromic compound represented by the formula (65-2) were determined to be C: 76.61%, H: 6.58%, N: 2.57%, which are in good agreement with the calculated values for C₆₉H₇₁F₃N₂O₆ of C: 76.64%, H: 6.62%, N: 2.59%. A proton nuclear magnetic resonance spectrum was measured, and showed 26H peaks assigned to the 2-butoxyethyl groups, the propyloxy group, and the methyl group at around δ 0.5 to 3.5 ppm, 16H peaks assigned to the 2-butoxyethyl groups, the propyloxy group, and the methoxy groups at around δ 3.5 to 5.0 ppm, and 29H peaks assigned to the aromatic protons and alkene protons at around δ 5.0 to 9.0 ppm. Furthermore, a ¹³C-nuclear magnetic resonance spectrum was measured, and showed peaks assigned to the carbons of the aromatic rings around at δ 110 to 160 ppm, peaks assigned to the carbons of the alkene around at δ 80 to 140 ppm, and peaks assigned to the carbons of the alkyls at δ 20 to 60 ppm.

### Example 66

### First Step

In the first step in Example 5, 1-bromo-4-(2-methoxyphenyl)benzene was used in place of 2,4-dimethoxy-1,1-biphenyl, to give a compound represented by the following formula (66-1) in 68% yield.

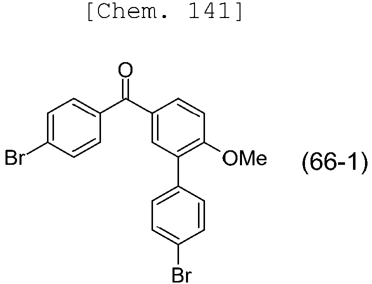

### Second Step

The reaction was carried out similarly to the first step in Example 1, except that the formula (66-1) was used in place of 4-bromo-4'-methoxybenzophenone, to synthesize a compound represented by the following formula (66-2).

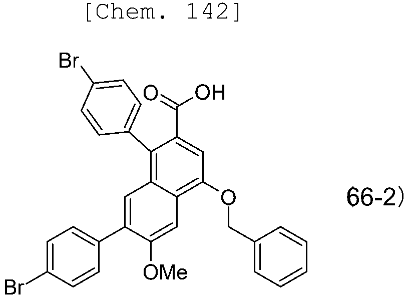

The reaction was carried out similarly to that described above except that the compound according to the formula (66-2) was used in place of the compound according to the formula (1-1), benzomorpholine was used in place of N-methylaniline, and additionally, the mole equivalents of benzomorpholine, sodium tertiary butoxide, Pd₂(dba)₃, and X-phos were doubled, to synthesize a compound represented by the following formula (66-3).

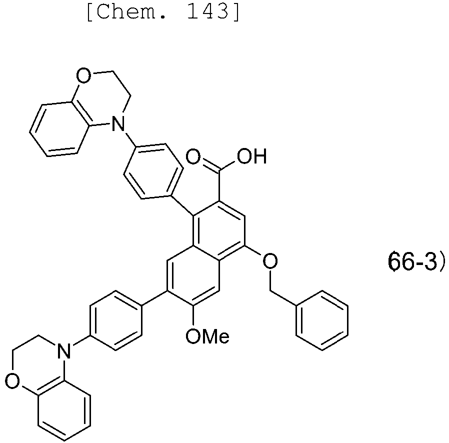

### Third Step

The reaction was carried out similarly to Example 1, except that the compound according to the formula (66-3) was used in place of the compound according to the formula (1-2) in the second step, and 6-undecanone was used in place of acetone in the third step, to give a phenol derivative represented by the following formula (66-4).

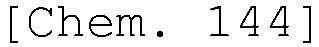

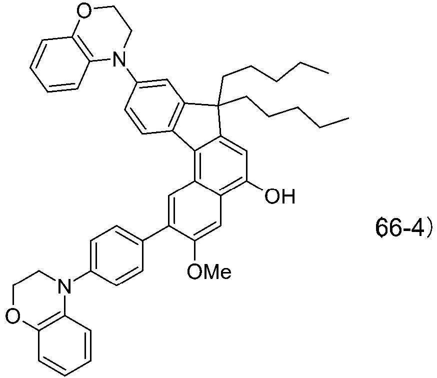

### Fourth Step

The reaction was carried out similarly to the fifth step in Example 1, except that the compound according to the formula (66-4) was used in place of the compound according to the formula (1-5), to give a photochromic compound represented by the following formula (66-5) in 65% yield.

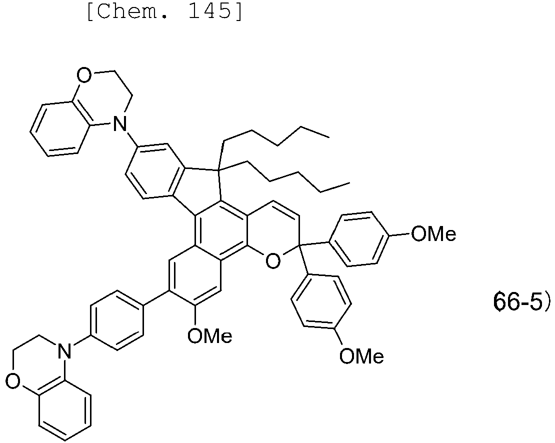

Elemental analysis values for the photochromic compound represented by the formula (66-5) were determined to be C: 80.85%, H: 6.70%, N: 2.82%, which are in good agreement with the calculated values for C₆₇H₆₆N₂O₆ of C: 80.86%, H: 6.68%, N: 2.81%. A proton nuclear magnetic resonance spectrum was measured, and showed 26H peaks assigned to the pentyl groups and the benzomorpholino groups at around δ 0.5 to 3.5 ppm, 13H peaks assigned to the benzomorpholino groups and the methoxy groups at around δ 3.5 to 5.0 ppm, and 27H peaks assigned to the aromatic protons and alkene protons at around δ 5.0 to 9.0 ppm. Furthermore, a ¹³C-nuclear magnetic resonance spectrum was measured, and showed peaks assigned to the carbons of the aromatic rings around at δ 110 to 160 ppm, peaks assigned to the carbons of the alkene around at δ 80 to 140 ppm, and peaks assigned to the carbons of the alkyls at δ 20 to 60 ppm.

### Example 67

### First Step

The reaction was carried out similarly to Example 62, except that the formula (61-1) was used in place of the compound according to the formula (62-1) in the second step, 2,4-dimethoxyphenylboronic acid was used in place of 2,4,6-trimethoxyphenylboronic acid in the third step, and iodoethane was used in place of 1-bromo-2-[2-(2-methoxyethoxy)ethoxy]ethane in the fourth step, to give a phenol derivative represented by the following formula (67-1).

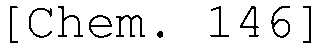

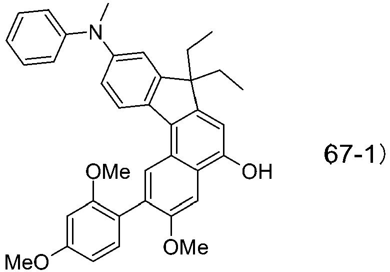

### Second Step

The reaction was carried out similarly to the fifth step in Example 1, except that the compound according to the formula (67-1) was used in place of the compound according to the formula (1-5), and the compound according to the formula (4-2) was used in place of the compound according to the formula (1-6), to give a photochromic compound represented by the following formula (67-2) in 71% yield.

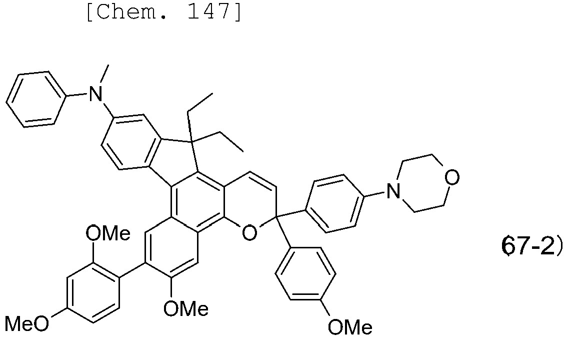

Elemental analysis values for the photochromic compound represented by the formula (67-2) were determined to be C: 79.16%, H: 6.52%, N: 3.23%, which are in good agreement with the calculated values for C₅₇H₅₆N₂O₆ of C: 79.14%, H: 6.52%, N: 3.24%. A proton nuclear magnetic resonance spectrum was measured, and showed 17H peaks assigned to the methyl group, the ethyl groups, and the morpholino group at around δ 0.5 to 3.5 ppm, 16H peaks assigned to the morpholino group and the methoxy groups at around δ 3.5 to 5.0 ppm, and 23H peaks assigned to the aromatic protons and alkene protons at around δ 5.0 to 9.0 ppm. Furthermore, a ¹³C-nuclear magnetic resonance spectrum was measured, and showed peaks assigned to the carbons of the aromatic rings around at δ 110 to 160 ppm, peaks assigned to the carbons of the alkene around at δ 80 to 140 ppm, and peaks assigned to the carbons of the alkyls at δ 20 to 60 ppm.

### Example 68

### First Step

The reaction was carried out similarly to Example 2, except that 4-methyldiphenylamine was used in place of N-methylaniline in the first step, and 1-butoxy-2-bromoethane was used in place of 1-bromo-4-methoxybutane in the sixth step, to give a phenol derivative represented by the following formula (68-1).

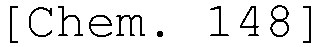

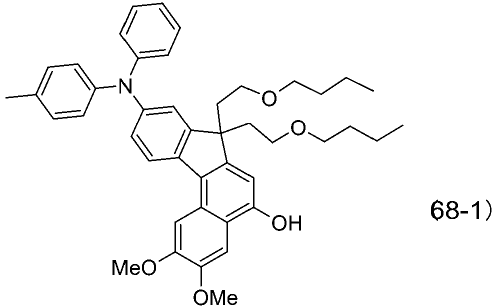

### Second Step

The reaction was carried out similarly to the fifth step in Example 1, except that the compound according to the formula (68-1) was used in place of the compound according to the formula (1-5), and the compound according to the formula (4-2) was used in place of the compound according to the formula (1-6), to give a photochromic compound represented by the following formula (68-2) in 75% yield.

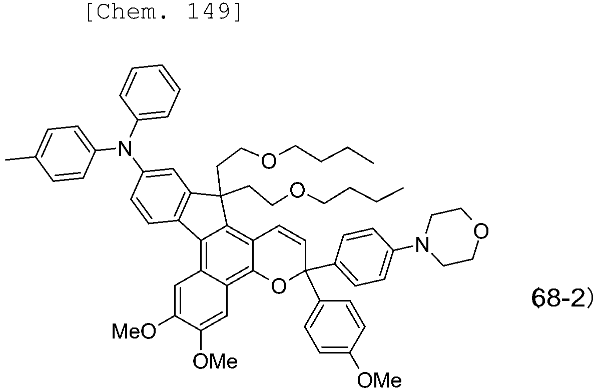

Elemental analysis values for the photochromic compound represented by the formula (68-2) were determined to be C: 78.51%, H: 7.19%, N: 2.86%, which are in good agreement with the calculated values for C₆₄H₇₀N₂O₇ of C: 78.50%, H: 7.21%, N: 2.86%. A proton nuclear magnetic resonance spectrum was measured, and showed 25H peaks assigned to the methyl group, the 2-butoxyethyl groups, and the morpholino group at around δ 0.5 to 3.5 ppm, 21H peaks assigned to the 2-butoxyethyl groups, the morpholino group, and the methoxy groups at around δ 3.5 to 5.0 ppm, and 24H peaks assigned to the aromatic protons and alkene protons at around δ 5.0 to 9.0 ppm. Furthermore, a ¹³C-nuclear magnetic resonance spectrum was measured, and showed peaks assigned to the carbons of the aromatic rings around at δ 110 to 160 ppm, peaks assigned to the carbons of the alkene around at δ 80 to 140 ppm, and peaks assigned to the carbons of the alkyls at δ 20 to 60 ppm.

### Example 69

### First Step

The reaction was carried out similarly to Example 65, except that morpholine was used in place of N-methyl-4-(trifluoromethyl)aniline in the first step, and 1-bromo-3-methoxypropane was used in place of 1-butoxy-2-bromoethane in the second step, to synthesize a phenol derivative represented by the following formula (69-1).

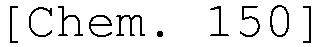

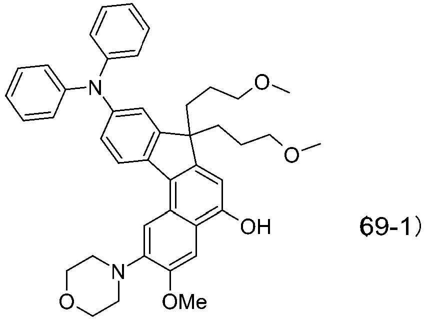

### Second Step

The reaction was carried out similarly to the fifth step in Example 1, except that the compound according to the formula (69-1) was used in place of the compound according to the formula (1-5), and the compound according to the formula (12-4) was used in place of the compound according to the formula (1-6), to a photochromic compound give represented by the following formula (69-2) in 75% yield.

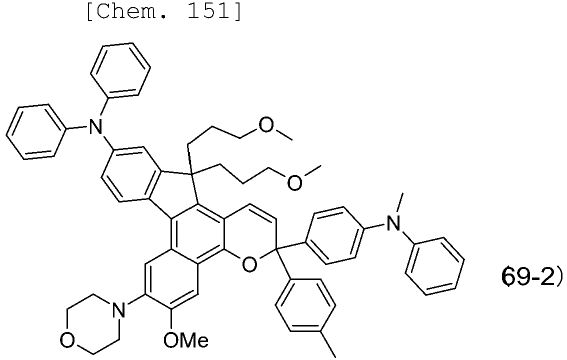

Elemental analysis values for the photochromic compound represented by the formula (69-2) were determined to be C: 80.61%, H: 6.76%, N: 4.35%, which are in good agreement with the calculated values for C₆₅H₆₅N₃O₅ of C: 80.63%, H: 6.77%, N: 4.34%. A proton nuclear magnetic resonance spectrum was measured, and showed 18H peaks assigned to the methyl groups, the methoxypropyl groups, and the morpholino group at around δ 0.5 to 3.5 ppm, 17H peaks assigned to the methoxypropyl groups, the morpholino group, and the methoxy group at around δ 3.5 to 5.0 ppm, and 30H peaks assigned to the aromatic protons and alkene protons at around δ 5.0 to 9.0 ppm. Furthermore, a ¹³C-nuclear magnetic resonance spectrum was measured, and showed peaks assigned to the carbons of the aromatic rings around at δ 110 to 160 ppm, peaks assigned to the carbons of the alkene around at δ 80 to 140 ppm, and peaks assigned to the carbons of the alkyls at δ 20 to 60 ppm.

### Example 70

### First Step

The reaction was carried out similarly to the first step in Example 12, except that the compound according to the formula (62-2) was used in place of the compound according to the formula (12-1), and 2-chlorophenylboronic acid was used in place of 4-methoxyphenylboronic acid, to give a compound represented by the following formula (70-1).

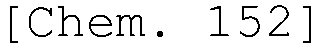

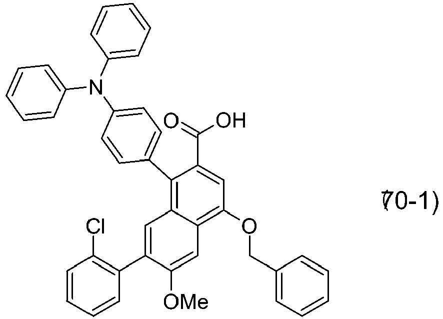

### Second Step

The reaction was carried out similarly to the first step in Example 1, except that the compound according to the formula (70-1) was used in in place of the compound according to the formula (1-1), and morpholine was used in place of N-methylaniline, to give a compound represented by the following formula (70-2).

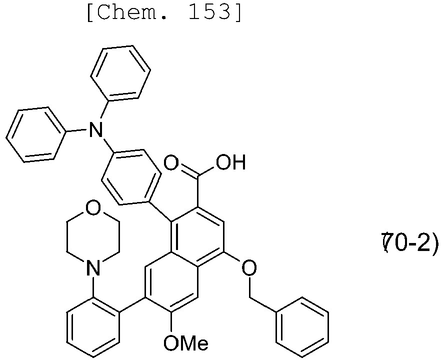

### Third Step

The reaction was carried out similarly to Example 1, except that the compound according to the formula (70-2) was used in place of the compound according to the formula (1-3) in the third step, and dihexyl ketone was used in place of acetone in the fourth step, to synthesize a phenol derivative represented by the following formula (70-3).

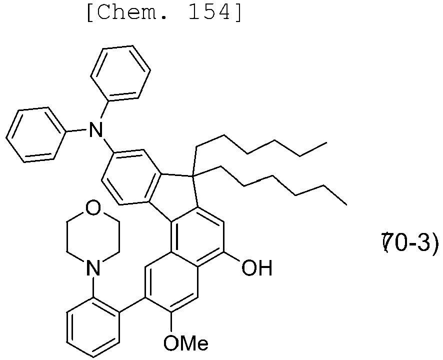

### Fourth Step

The reaction was carried out similarly to the fifth step in Example 1, except that the compound according to the formula (70-3) was used in place of the compound according to the formula (1-5), and the compound according to the formula (16-2) was used in place of the compound according to the formula (1-6), to give a photochromic compound represented by the following formula (70-4) in 68% yield.

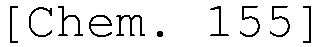

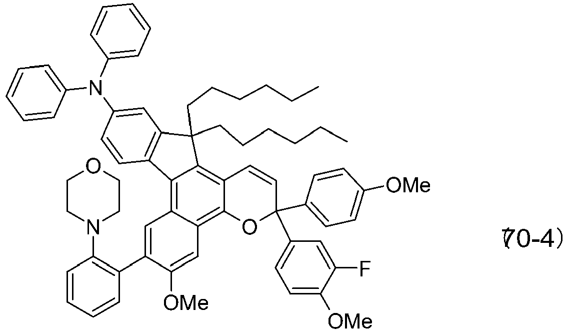

Elemental analysis values for the photochromic compound represented by the formula (70-4) were determined to be C: 80.64%, H: 6.96%, N: 2.72%, which are in good agreement with the calculated values for C₆₉H₇₁FN₂O₅ of C: 80.67%, H: 6.97%, N: 2.73%. A proton nuclear magnetic resonance spectrum was measured, and showed 30H peaks assigned to the hexyl groups and the morpholino group at around δ 0.5 to 3.5 ppm, 13H peaks assigned to the morpholino group and the methoxy groups at around δ 3.5 to 5.0 ppm, and 28H peaks assigned to the aromatic protons and alkene protons at around δ 5.0 to 9.0 ppm. Furthermore, a ¹³C-nuclear magnetic resonance spectrum was measured, and showed peaks assigned to the carbons of the aromatic rings around at δ 110 to 160 ppm, peaks assigned to the carbons of the alkene around at δ 80 to 140 ppm, and peaks assigned to the carbons of the alkyls at δ 20 to 60 ppm.

### Example 71

### First Step

The reaction was carried out similarly to Example 62, except that the formula (61-1) was used in place of the compound according to the formula (62-1) in the second step, and 4-chlorophenylboronic acid was used in place of 2,4,6-trimethoxyphenylboronic acid in the third step, to give a compound represented by the following formula (71-1).

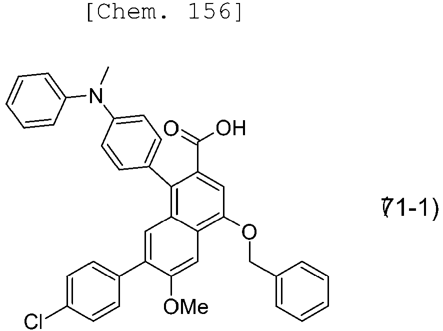

### Second Step

The reaction was carried out similarly to the first step in Example 1, except that the compound according to the formula (71-1) was used in in place of the compound according to the formula (1-1), and 3,5-dimethylmorpholine was used in place of N-methylaniline, to give a compound represented by the following formula (71-2).

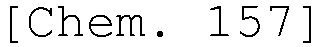

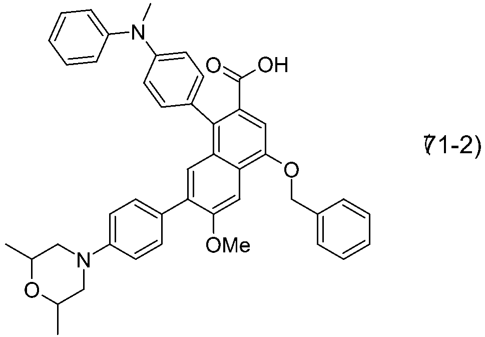

### Third Step

The reaction was carried out similarly to Example 1, except that the compound according to the formula (71-2) was used in place of the compound according to the formula (1-3) in the third step, and dipropyl ketone was used in place of acetone in the fourth step, to synthesize a phenol derivative represented by the following formula (71-3).

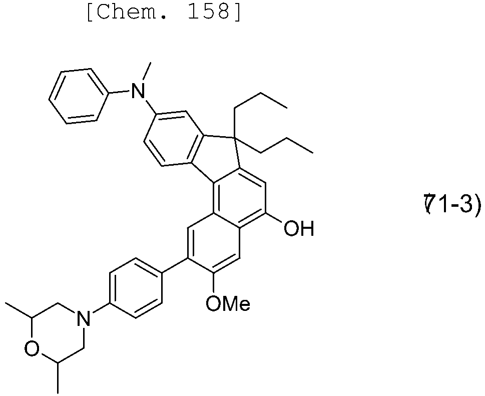

### Fourth Step

The reaction was carried out similarly to the fifth step in Example 1, except that the compound according to the formula (71-3) was used in place of the compound according to the formula (1-5), and a compound according to the following formula (71-4) was used in place of the compound according to the formula (1-6), to give a photochromic compound represented by the following formula (71-5) in 71% yield.

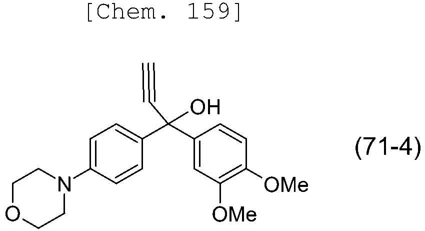

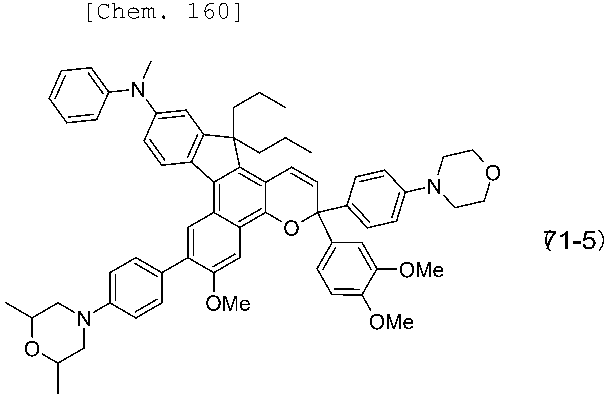

Elemental analysis values for the photochromic compound represented by the formula (71-5) were determined to be C: 78.72%, H: 7.13%, N: 4.30%, which are in good agreement with the calculated values for C₆₄H₆₉N₃O₆ of C: 78.74%, H: 7.12%, N: 4.30%. A proton nuclear magnetic resonance spectrum was measured, and showed 31H peaks assigned to the propyl groups, the morpholino groups, and the methyl groups at around δ 0.5 to 3.5 ppm, 15H peaks assigned to the morpholino groups and the methoxy groups at around δ 3.5 to 5.0 ppm, and 23H peaks assigned to the aromatic protons and alkene protons at around δ 5.0 to 9.0 ppm. Furthermore, a ¹³C-nuclear magnetic resonance spectrum was measured, and showed peaks assigned to the carbons of the aromatic rings around at δ 110 to 160 ppm, peaks assigned to the carbons of the alkene around at δ 80 to 140 ppm, and peaks assigned to the carbons of the alkyls at δ 20 to 60 ppm.

The analytical results of the phenol derivatives synthesized in Examples 61 to 71 are shown in Table 7.

### [Table 7]

**table 7**

| Examples | Calculated value | | | | Measured value | | | | 1H-NMR | |
|---|---|---|---|---|---|---|---|---|---|---|
| | C | H | N | S | C | H | N | S | | |
| 61 | 81.98 | 7.24 | 5.03 | 0.00 | 81.99 | 7.26 | 5.04 | 0.00 | δ0.5-5.0ppm | 23H |
| | | | | | | | | | δ5.0-9.0ppm | 17H |
| 62 | 71.68 | 6.92 | 1.58 | 0.00 | 71.69 | 6.91 | 1.56 | 0.00 | δ0.5-5.0ppm | 42H |
| | | | | | | | | | δ5.0-9.0ppm | 19H |
| 63 | 76.62 | 6.09 | 2.35 | 0.00 | 76.60 | 6.08 | 2.34 | 0.00 | δ0.5-5.0ppm | 20H |
| | | | | | | | | | δ5.0-9.0ppm | 16H |
| 64 | 77.07 | 6.61 | 3.91 | 0.00 | 77.04 | 6.59 | 3.90 | 0.00 | δ0.5-5.0ppm | 30H |
| | | | | | | | | | δ5.0-9.0ppm | 17H |
| 65 | 74.79 | 6.65 | 3.49 | 0.00 | 74.77 | 6.62 | 3.48 | 0.00 | δ0.5-5.0ppm | 32H |
| | | | | | | | | | δ5.0-9.0ppm | 21H |
| 66 | 80.61 | 7.04 | 3.76 | 0.00 | 80.63 | 7.04 | 3.74 | 0.00 | δ0.5-5.0ppm | 33H |
| | | | | | | | | | δ5.0-9.0ppm | 19H |
| 67 | 79.40 | 6.66 | 2.50 | 0.00 | 79.41 | 6.65 | 2.52 | 0.00 | δ0.5-5.0ppm | 22H |
| | | | | | | | | | δ5.0-9.0ppm | 15H |
| 68 | 78.42 | 7.63 | 2.08 | 0.00 | 78.44 | 7.62 | 2.05 | 0.00 | δ0.5-5.0ppm | 35H |
| | | | | | | | | | δ5.0-9.0ppm | 16H |
| 69 | 76.57 | 7.04 | 4.25 | 0.00 | 76.58 | 7.05 | 4.24 | 0.00 | δ0.5-5.0ppm | 29H |
| | | | | | | | | | δ5.0-9.0ppm | 17H |
| 70 | 82.28 | 7.70 | 3.69 | 0.00 | 82.30 | 7.69 | 3.67 | 0.00 | δ0.5-5.0ppm | 37H |
| | | | | | | | | | δ5.0-9.0ppm | 21H |
| 71 | 80.59 | 7.55 | 4.37 | 0.00 | 80.61 | 7.54 | 4.39 | 0.00 | δ0.5-5.0ppm | 32H |
| | | | | | | | | | δ5.0-9.0ppm | 16H |

### (Examples 72 to 81)

Photochromic laminates were formed using the photochromic compound obtained in Examples 61 to 71 in the same manner as in Example 20. The results are shown in Table 8.

### [Table 8]

**table 8**

| | Compound No. | Maximum absorption wavelength (nm) | Color developing density at 23°C (-) | Color developing density at 35°C (-) | Color development ratio at high temperature (%) | Fading half-life at 23°C (sec) |
|---|---|---|---|---|---|---|
| Example 72 | Formula (61-4) | 502 | 1.37 | 1.03 | 75% | 108 |
| | | 619 | 0.88 | 0.64 | | 110 |
| Example 73 | Formula (62-5) | 456 | 1.20 | 0.88 | 73% | 102 |
| | | 625 | 0.90 | 0.66 | | 103 |
| Example 74 | Formula (63-2) | 460 | 0.71 | 0.50 | 70% | 82 |
| | | 626 | 0.80 | 0.57 | | 82 |
| Example 75 | Formula (64-3) | 473 | 1.94 | 1.38 | 71% | 167 |
| | | 611 | 0.92 | 0.67 | | 168 |
| Example 76 | Formula (65-2) | 462 | 0.85 | 0.57 | 67% | 43 |
| | | 623 | 0.77 | 0.53 | | 43 |
| Example 77 | Formula (66-5) | 469 | 1.37 | 1.09 | 80% | 119 |
| | | 602 | 1.03 | 0.82 | | 120 |
| Example 78 | Formula (67-2) | 472 | 0.84 | 0.60 | 71% | 83 |
| | | 639 | 1.00 | 0.72 | | 83 |
| Example 79 | Formula (68-2) | 477 | 0.79 | 0.51 | 65% | 64 |
| | | 636 | 0.73 | 0.47 | | 64 |
| Example 80 | Formula (69-2) | 514 | 1.76 | 1.32 | 75% | 147 |
| | | 640 | 1.28 | 0.96 | | 145 |
| Example 81 | Formula (70-4) | 456 | 1.44 | 1.16 | 81% | 205 |
| | | 621 | 1.24 | 1.01 | | 205 |
| Example 82 | Formula (71-5) | 494 | 1.43 | 1.17 | 82% | 225 |
| | | 643 | 1.36 | 1.10 | | 228 |

Preferred embodiments of the present invention will be additionally described below.

[1] A photochromic compound represented by the following formula (1): 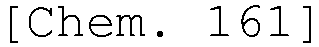 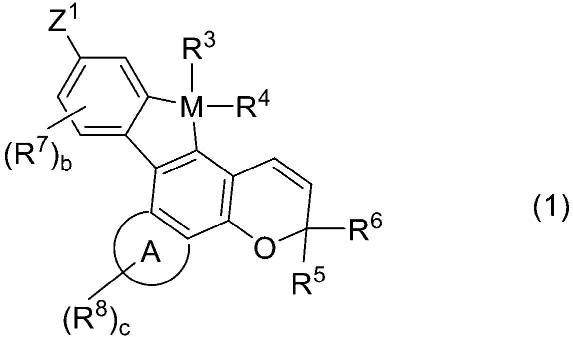 wherein in the formula (1),
   M is C, Si, or Ge,
   the ring A is an aromatic ring, an aromatic heterocyclic ring, or a substituted or unsubstituted fused polycyclic ring in which an aromatic ring or an aromatic heterocyclic ring is fused to the aromatic ring or the aromatic heterocyclic ring, Z¹ is a group represented by the following formula (1a), or a group represented by the following formula (1b), 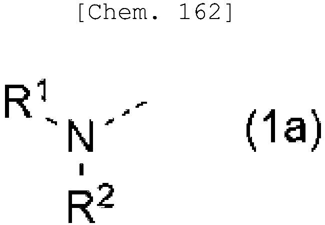 wherein in the formula (1a),
   R¹ is a substituted or unsubstituted aryl group, a substituted or unsubstituted heteroaryl group, or a substituted or unsubstituted fused polycyclic ring in which an aromatic ring or an aromatic heterocyclic ring is fused to the aryl group or the heteroaryl group, and
   R² is a hydrogen atom, a substituted or unsubstituted alkyl group, a substituted or unsubstituted haloalkyl group, a substituted or unsubstituted cycloalkyl group, a substituted or unsubstituted alkoxy group, a substituted or unsubstituted aryl group, a substituted or unsubstituted heteroaryl group, or a group represented by the following formula (2a),

      -Q¹-(X¹Q²) a-X²Q³ (2a)
   wherein in the formula (2a),
   Q¹ is an alkylene group or a haloalkylene group,
   Q² is an alkylene group or a haloalkylene group,
   Q³ is an alkyl group or a haloalkyl group,
   X¹ and X² are each independently O, S, NR⁷⁰⁰, PR⁷⁰¹, or P(=O), R⁷⁰⁰ and R⁷⁰¹ are each independently a hydrogen atom, a substituted or unsubstituted alkyl group, a substituted or unsubstituted cycloalkyl group, a substituted or unsubstituted aryl group, or a substituted or unsubstituted heteroaryl group, and
   a is 0, or 1 or more and 3 or less, 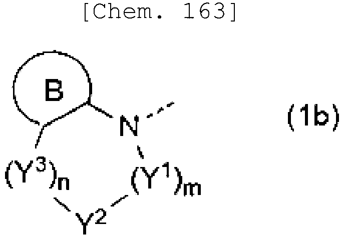 wherein in the formula (1b),
   the ring B is a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to the aromatic ring or the aromatic heterocyclic ring,
   Y¹ is a substituted or unsubstituted methylene group,
   Y² is a substituted or unsubstituted methylene group, O, S, SO₂, NR⁶⁰⁰, R⁶⁰¹C=CR⁶⁰², or CC,
   R⁶⁰⁰, R⁶⁰¹, and R⁶⁰² are each independently a hydrogen atom, a substituted or unsubstituted alkyl group, a substituted or unsubstituted alkoxy group, a substituted or unsubstituted haloalkyl group, a substituted or unsubstituted haloalkoxy group, a substituted or unsubstituted cycloalkyl group, a substituted or unsubstituted aryl group, or a substituted or unsubstituted heteroaryl group,
   Y³ is a substituted or unsubstituted methylene group,
   m is an integer of 1 to 4, n is an integer of 0 to 4, and a sum of m and n is an integer of 1 or more,
   R³ and R⁴ are each independently a hydrogen atom, a hydroxyl group, a substituted or unsubstituted alkyl group, a haloalkyl group, the group represented by the formula (2a), a substituted or unsubstituted cycloalkyl group, a substituted or unsubstituted alkoxy group, a formyl group, a hydroxycarbonyl group, an alkylcarbonyl group, an alkoxycarbonyl group, a halogen atom, a substituted or unsubstituted aralkyl group, a substituted or unsubstituted aralkoxy group, a substituted or unsubstituted aryloxy group, a substituted or unsubstituted heteroaryl group, or a group represented by the following formula (X3),

      L¹-R⁴⁰⁰ (X3)
   wherein in the formula (X3),
   R⁴⁰⁰ is a hydrogen atom, an alkyl group, an aryl group, a polymerizable group, a photochromic group, or a silyl group having an alkyl group, an alkoxy group or an aryl group as a substituent, and
   L¹ is a group represented by the following formula (X2), 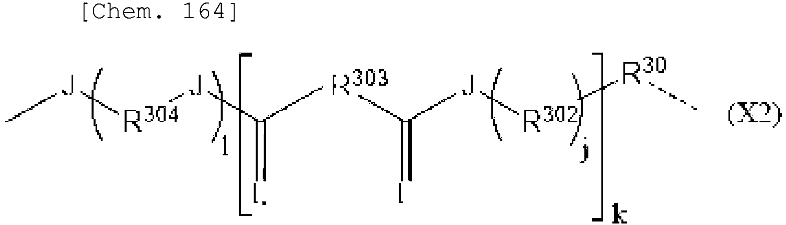
      wherein in the formula (X2), R³⁰ is a group represented by the following formula (X2a), 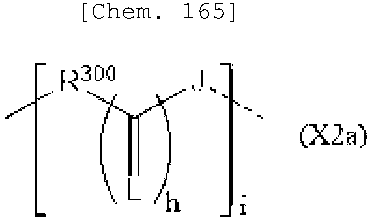
      wherein in the formula (X2) and the formula (X2a),
   J is a divalent group, and is each independently a direct bond, a substituted or unsubstituted methylene group, an oxygen atom, a sulfur atom, or NR³⁰¹, and R³⁰¹ is a hydrogen atom or an alkyl group,
   L is an oxygen atom or a sulfur atom,
   R³⁰⁰ is an alkylene group, or a silylene group having an alkyl group or an aryl group as a substituent,
   R³⁰², R³⁰³, and R³⁰⁴ are each independently an alkylene group,
   h, j, k and 1 are each independently an integer of 0 or 1, and i is an integer of 1 to 200, and when i is 2 or more, a plurality of R³⁰ may be identical or different, and the dashed line represents a bond to R⁴⁰⁰, and
   R³ and R⁴ are optionally taken together with M to form a substituted or unsubstituted aliphatic ring having 3 to 20 ring member carbon atoms, a substituted or unsubstituted fused polycyclic ring in which an aromatic hydrocarbon ring or an aromatic heterocyclic ring is fused to the aliphatic ring, a substituted or unsubstituted heterocyclic ring having 3 to 20 ring member atoms, or a substituted or unsubstituted fused polycyclic ring in which an aromatic ring or an aromatic heterocyclic ring is fused to the heterocyclic ring,
   R⁵ and R⁶ are each independently a hydrogen atom or a substituent,
   R⁷ and R⁸ are each independently a substituent,
   b is an integer of 0 to 3, and
   c is an integer of 0 to 4.
[2] The photochromic compound according to [1], represented by the following formula (3): 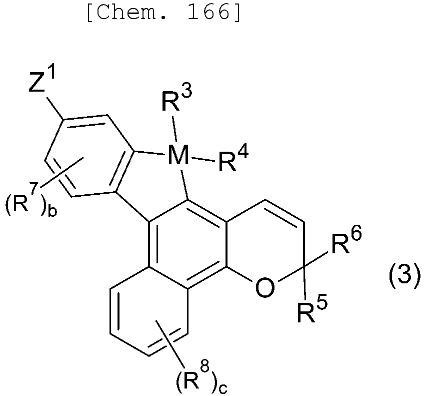
   wherein in the formula (3), R³, R⁴, Z¹, M, b, and c are each as defined in the formula (1),
      R⁵ and R⁶ are each independently a substituted or unsubstituted aryl group, or a substituted or unsubstituted heteroaryl group,
      R⁷ and R⁸ are each independently a hydroxyl group, a substituted or unsubstituted alkyl group, a haloalkyl group, a substituted or unsubstituted cycloalkyl group, a substituted or unsubstituted alkoxy group, a substituted or unsubstituted amino group, a substituted or unsubstituted heterocyclic group, a cyano group, a halogen atom, a substituted or unsubstituted alkylthio group, a substituted or unsubstituted arylthio group, a nitro group, a formyl group, a hydroxycarbonyl group, a substituted or unsubstituted alkylcarbonyl group, a substituted or unsubstituted alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, a substituted or unsubstituted alkoxyalkylthio group, a haloalkylthio group, a substituted or unsubstituted cycloalkylthio group, a substituted or unsubstituted silyl group, a substituted or unsubstituted oxysilyl group, the group represented by the formula (2a), a group represented by the following formula (X), or the group represented by the formula (X3),
      when b is 2 to 3, a plurality of R⁷ may be identical to or different from one another,
      when c is 2 to 4, a plurality of R⁸ may be identical to or different from one another,
      when b is 2 to 3, and adjacent R⁷ are present, two adjacent R⁷ are optionally taken together with the carbon atoms bonded to R⁷ to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to the aliphatic ring, the aliphatic heterocyclic ring, the aromatic ring, or the aromatic heterocyclic ring, and
      when c is 2 to 4, and adjacent R⁸ are present, two adjacent R⁸ are optionally taken together with the carbon atoms bonded to R⁸ to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to the aliphatic ring, the aliphatic heterocyclic ring, the aromatic ring, or the aromatic heterocyclic ring, 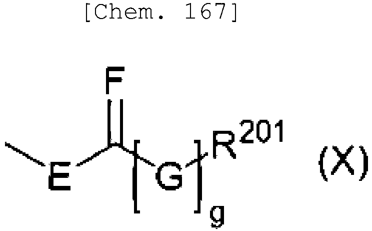
   wherein in the formula (X),
      E is an oxygen atom or NR¹⁰¹, and R¹⁰¹ is a hydrogen atom or an alkyl group,
      F is an oxygen atom or a sulfur atom,
      G is an oxygen atom, a sulfur atom, or NR²⁰², and R²⁰² is a hydrogen atom, an alkyl group, a cycloalkyl group, an aryl group, or a heteroaryl group,
      g is 0 or 1,
      R²⁰¹ is a hydrogen atom, an alkyl group, a cycloalkyl group, an aryl group, or a heteroaryl group, and
      when G is an oxygen atom or a sulfur atom, R²⁰¹ is a group
      other than a hydrogen atom.
[3] The photochromic compound according to [2], represented by the following formula (4): 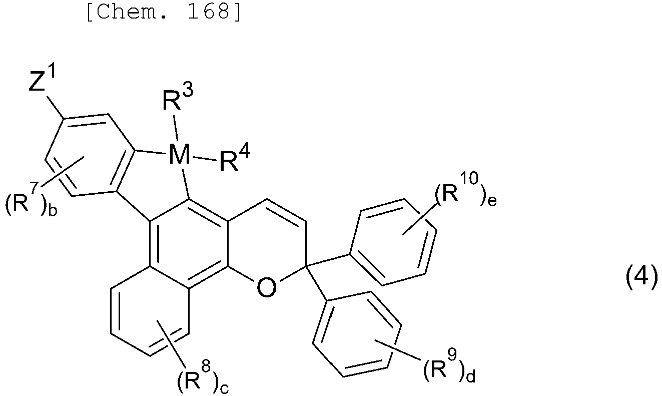 wherein in the formula (4),
   R³, R⁴, R⁷, R⁸, Z¹, M, b, and c are each independently as defined in the formula (3),
   R⁹ and R¹⁰ are each independently a hydroxyl group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, a substituted or unsubstituted amino group, a substituted or unsubstituted heterocyclic group, a cyano group, a halogen atom, an alkylthio group having 1 to 6 carbon atoms, a substituted or unsubstituted arylthio group, a substituted or unsubstituted aryloxy group, a substituted or unsubstituted aryl group, a substituted or unsubstituted heteroaryl group, a nitro group, a substituted or unsubstituted silyl group, a substituted or unsubstituted oxysilyl group, the group represented by the formula (2a), or the group represented by the formula (X3),
   d is an integer of 0 to 5,
   when d is 2 to 5, a plurality of R⁹ may be groups identical to or different from one another,
   when adjacent R⁹ are present, two adjacent R⁹ are optionally taken together with the carbon atoms bonded to R⁹ to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to the aliphatic ring, the aliphatic heterocyclic ring, the aromatic ring, or the aromatic heterocyclic ring,
   e is an integer of 0 to 5,
   when e is 2 to 5, a plurality of R¹⁰ may be groups identical to or different from one another,
   when adjacent R¹⁰ are present, two adjacent R¹⁰ are optionally taken together with the carbon atoms bonded to R¹⁰ to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to the aliphatic ring, the aliphatic heterocyclic ring, the aromatic ring, or the aromatic heterocyclic ring.
[4] The photochromic compound according to any one of [1] to [3], wherein M in the formula (1) is C.
[5] The photochromic compound according to any one of [1] to [4], wherein R³ and R⁴ in the formula (1) are a substituted or unsubstituted alkyl group, a haloalkyl group, the group represented by the formula (2a), a substituted or unsubstituted alkoxy group, the group represented by the formula (X3), A substituted or unsubstituted aliphatic ring having 3 to 20 ring member carbon atoms formed together with M, or a substituted or unsubstituted heterocyclic ring having 3 to 20 ring member atoms formed together with M.
[6] The photochromic compound according to any one of [1] to [5], wherein Z¹ in the formula (1) has the group represented by the formula (1a), and R¹ is a substituted or unsubstituted phenyl group.
[7] The photochromic compound according to any one of [1] to [6], wherein Z¹ in the formula (1) has the group represented by the formula (1a), and R² is a substituted or an unsubstituted alkyl group having 1 or more and 10 or less carbon atoms, a substituted or unsubstituted haloalkyl group having 1 or more and 10 or less carbon atoms, or the group represented by the formula (2a).
[8] The photochromic compound according to any one of [1] to [7], wherein Z¹ in the formula (1) has the group represented by the formula (1b), and the ring B is a substituted or unsubstituted phenyl group.
[9] The photochromic compound according to any one of [1] to [8], wherein Z¹ in the formula (1) has the group represented by the formula (1b), and Y² is a substituted or unsubstituted methylene group, O, or S.
[10] The photochromic compound according to any one of [1] to [9], wherein Z¹ in the formula (1) has the group represented by the formula (1b), and the sum of m and n is an integer of 1 to 3.
[11] A curable composition containing:
   the photochromic compound according to any one of [1] to [10]; and
   at least one selected from the group consisting of a radically-polymerizable monomer, a cationically-polymerizable monomer, a compound having a polymerization-reactive group, and a (thio)urethane(urea) polymer.
[12] A cured product of the curable composition according to [11].
[13] An optical article containing the cured product according to [12].
[14] A lens containing the photochromic compound according to any one of [1] to [10].
[15] Eyeglasses including the lens according to [14].
[16] A phenol derivative represented by the following formula (5): 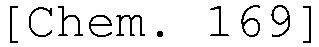 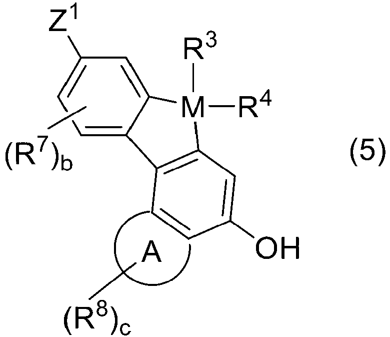 wherein in the formula (5),
   M is C, Si, or Ge,
   the ring A is a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to the aromatic ring or the aromatic heterocyclic ring,
   Z¹ is a group represented by the following formula (1a), or a group represented by the following formula (1b), 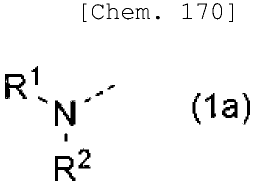 wherein in the formula (1a),
   R¹ is a substituted or unsubstituted aryl group, or a substituted or unsubstituted heteroaryl group, and
   R² is a hydrogen atom, a substituted or unsubstituted alkyl group, a substituted or unsubstituted haloalkyl group, a substituted or unsubstituted cycloalkyl group, a substituted or unsubstituted alkoxy group, a substituted or unsubstituted aryl group, a substituted or unsubstituted heteroaryl group, or a group represented by the following formula (2a),

      -Q¹-(X¹Q²) a-X²Q³ (2a)
   wherein in the formula (2a),
   Q¹ is an alkylene group or a haloalkylene group,
   Q² is an alkylene group or a haloalkylene group,
   Q³ is an alkyl group or a haloalkyl group,
   X¹ and X² are each independently O, S, NR⁷⁰⁰R⁷⁰¹, PR⁷⁰²R⁷⁰³, or P(=O) R⁷⁰⁴,
   R⁷⁰⁰, R⁷⁰¹, R⁷⁰², R⁷⁰³, and R⁷⁰⁴ are each independently a hydrogen atom, a substituted or unsubstituted alkyl group, a substituted or unsubstituted cycloalkyl group, a substituted or unsubstituted aryl group, or a substituted or unsubstituted heteroaryl group, and
   a is an integer of 0 or 1 or more and 3 or less, 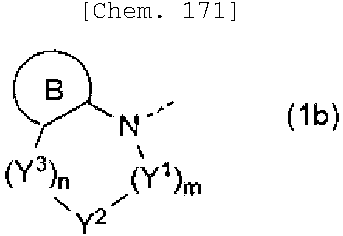 wherein in the formula (1b),
   the ring B is a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to the aromatic ring or the aromatic heterocyclic ring,
   Y¹ is a substituted or unsubstituted methylene group,
   Y² is a substituted or unsubstituted methylene group, O, S, SO₂, NR⁶⁰⁰, R⁶⁰¹C=CR⁶⁰², or CC,
   R⁶⁰⁰, R⁶⁰¹, and R⁶⁰² are each independently a hydrogen atom, a substituted or unsubstituted alkyl group, a substituted or unsubstituted alkoxy group, a substituted or unsubstituted haloalkyl group, a substituted or unsubstituted haloalkoxy group, a substituted or unsubstituted cycloalkyl group, a substituted or unsubstituted aryl group, or a substituted or unsubstituted heteroaryl group,
   Y³ is a substituted or unsubstituted methylene group,
   m is an integer of 1 to 4, n is an integer of 0 to 4, and a sum of m and n is an integer of 1 or more,
   R³ and R⁴ are each independently a hydrogen atom, a hydroxyl group, a substituted or unsubstituted alkyl group, a haloalkyl group, the group represented by the formula (2a), a substituted or unsubstituted cycloalkyl group, a substituted or unsubstituted alkoxy group, a formyl group, a hydroxycarbonyl group, an alkylcarbonyl group, an alkoxycarbonyl group, a halogen atom, a substituted or unsubstituted aralkyl group, a substituted or unsubstituted aralkoxy group, a substituted or unsubstituted aryloxy group, a substituted or unsubstituted heteroaryl group, or a group represented by the following formula (X3),

      L¹- R⁴⁰⁰ (X3)
   wherein in the formula (X3),
   R⁴⁰⁰ is a hydrogen atom, an alkyl group, an aryl group, a polymerizable group, a photochromic group, or a silyl group having an alkyl group, an alkoxy group or an aryl group as a substituent, and
   L¹ is a group represented by the following formula (X2), 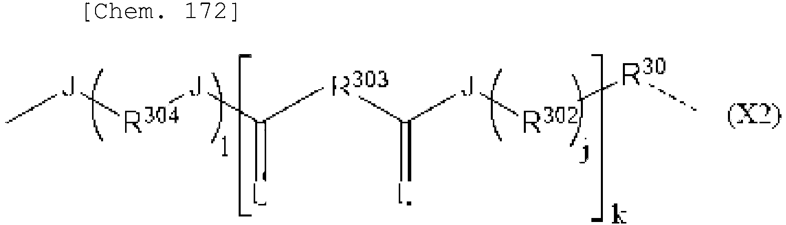 wherein in the formula (X2), R³⁰ is a group represented by the following formula (X2a), 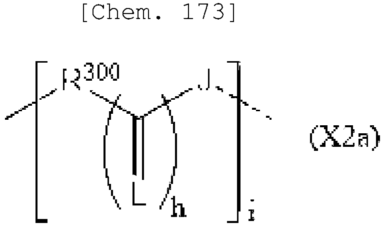
   wherein in the formula (X2) and the formula (X2a),
   J is a divalent group, and is each independently a direct bond, a substituted or unsubstituted methylene group, an oxygen atom, a sulfur atom, or NR³⁰¹, and R³⁰¹ is a hydrogen atom or an alkyl group,
   L is an oxygen atom or a sulfur atom,
   R³⁰⁰ is an alkylene group, or a silylene group having an alkyl group or an aryl group as a substituent,
   R³⁰², R³⁰³, and R³⁰⁴ are each independently an alkylene group,
   h, j, k and l are each independently an integer of 0 or 1, and i is an integer of 1 to 200, and when i is 2 or more, a plurality of R³⁰ may be identical or different, and the dashed line represents a bond to R⁴⁰⁰, and
   R³ and R⁴ are optionally taken together with M to form a substituted or unsubstituted aliphatic ring having 3 to 20 ring member carbon atoms, a substituted or unsubstituted fused polycyclic ring in which an aromatic hydrocarbon ring or an aromatic heterocyclic ring is fused to the aliphatic ring, a substituted or unsubstituted heterocyclic ring having 3 to 20 ring member atoms, or a substituted or unsubstituted fused polycyclic ring in which an aromatic ring or an aromatic heterocyclic ring is fused to the heterocyclic ring,
   R⁷ and R⁸ are each independently a substituent,
   b is an integer of 0 to 3, and
   c is an integer of 0 to 4.
[17] The phenol derivative according to [16], represented by the following formula (7): 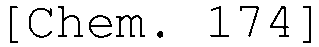 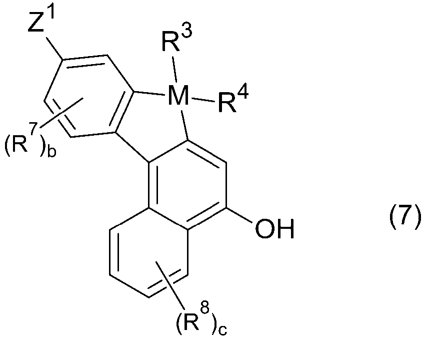 wherein in the formula (7), Z¹, R³, R⁴, M, b, and c are as defined in the formula (6),
   R⁷ and R⁸ are each independently a hydroxyl group, a substituted or unsubstituted alkyl group, a haloalkyl group, a substituted or unsubstituted cycloalkyl group, a substituted or unsubstituted alkoxy group, a substituted or unsubstituted amino group, a substituted or unsubstituted heterocyclic group, a cyano group, a halogen atom, a substituted or unsubstituted alkylthio group, a substituted or unsubstituted arylthio group, a nitro group, a formyl group, a hydroxycarbonyl group, a substituted or unsubstituted alkylcarbonyl group, a substituted or unsubstituted alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, a substituted or unsubstituted alkoxyalkylthio group, a haloalkylthio group, a substituted or unsubstituted cycloalkylthio group, a substituted or unsubstituted silyl group, a substituted or unsubstituted oxysilyl group, the group represented by the formula (2a), a group represented by the following formula (X), or the group represented by the formula (X3),
   when b is 2 to 3, a plurality of R⁷ may be identical to or different from one another,
   when c is 2 to 4, a plurality of R⁸ may be identical to or different from one another,
   when b is 2 to 3, and adjacent R⁷ are present, two adjacent R⁷ are optionally taken together with the carbon atoms bonded to R⁷ to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to the aliphatic ring, the aliphatic heterocyclic ring, the aromatic ring, or the aromatic heterocyclic ring, and
   when c is 2 to 4, and adjacent R⁸ are present, two adjacent R⁸ are optionally taken together with the carbon atoms bonded to R⁸ to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to the aliphatic ring, the aliphatic heterocyclic ring, the aromatic ring, or the aromatic heterocyclic ring, 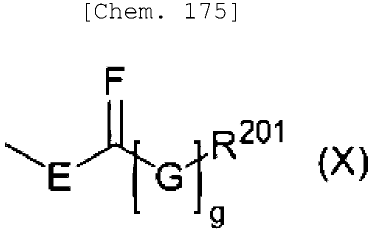 wherein in the formula (X),
   E is an oxygen atom or NR¹⁰¹, and R¹⁰¹ is a hydrogen atom or an alkyl group,
   F is an oxygen atom or a sulfur atom,
   G is an oxygen atom, a sulfur atom, or NR²⁰², and R²⁰² is a hydrogen atom, an alkyl group, a cycloalkyl group, an aryl group, or a heteroaryl group,
   g is 0 or 1,
   R²⁰¹ is a hydrogen atom, an alkyl group, a cycloalkyl group, an aryl group, or a heteroaryl group, and
   when G is an oxygen atom or a sulfur atom, R²⁰¹ is a group other than a hydrogen atom.

## Claims

1. A photochromic compound represented by formula (1): 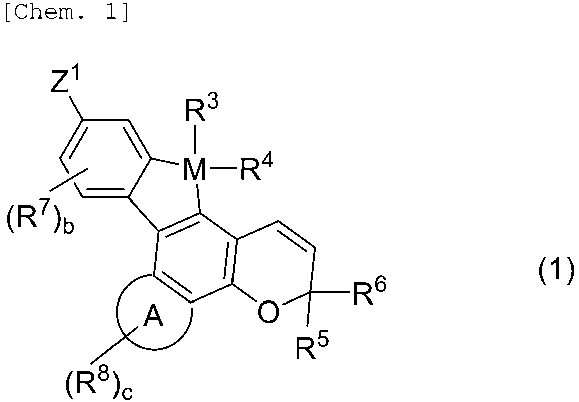 wherein in the formula (1),
M is C, Si, or Ge,
the ring A is an aromatic ring, an aromatic heterocyclic ring, or a substituted or unsubstituted fused polycyclic ring in which an aromatic ring or an aromatic heterocyclic ring is fused to the aromatic ring or the aromatic heterocyclic ring,
Z¹ is a group represented by formula (1a), or a group represented by formula (1b), 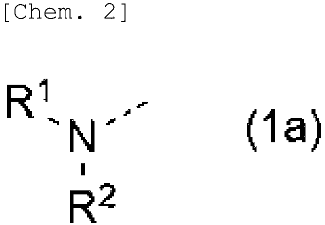
wherein in the formula (1a),
R¹ is a substituted or unsubstituted aryl group, a substituted or unsubstituted heteroaryl group, or a substituted or unsubstituted fused polycyclic ring in which an aromatic ring or an aromatic heterocyclic ring is fused to the aryl group or the heteroaryl group, and
R² is a hydrogen atom, a substituted or unsubstituted alkyl group, a substituted or unsubstituted haloalkyl group, a substituted or unsubstituted cycloalkyl group, a substituted or unsubstituted alkoxy group, a substituted or unsubstituted aryl group, a substituted or unsubstituted heteroaryl group, or a group represented by formula (2a),
-Q¹-(X¹Q²) a-X²Q³ (2a)
wherein in the formula (2a),
Q¹ is an alkylene group or a haloalkylene group,
Q² is an alkylene group or a haloalkylene group,
Q³ is an alkyl group or a haloalkyl group,
X¹ and X² are each independently O, S, NR⁷⁰⁰, PR⁷⁰¹, or P(=O),
R⁷⁰⁰ and R⁷⁰¹ are each independently a hydrogen atom, a substituted or unsubstituted alkyl group, a substituted or unsubstituted cycloalkyl group, a substituted or unsubstituted aryl group, or a substituted or unsubstituted heteroaryl group, and
a is 0, or 1 or more and 3 or less, 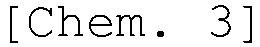 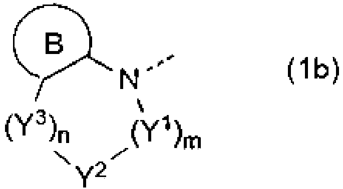
wherein in the formula (1b),
the ring B is a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to the aromatic ring or the aromatic heterocyclic ring,
Y¹ is a substituted or unsubstituted methylene group,
Y² is a substituted or unsubstituted methylene group, O, S, SO₂, NR⁶⁰⁰, R⁶⁰¹C=CR⁶⁰², or CC,
R⁶⁰⁰, R⁶⁰¹, and R⁶⁰² are each independently a hydrogen atom, a substituted or unsubstituted alkyl group, a substituted or unsubstituted alkoxy group, a substituted or unsubstituted haloalkyl group, a substituted or unsubstituted haloalkoxy group, a substituted or unsubstituted cycloalkyl group, a substituted or unsubstituted aryl group, or a substituted or unsubstituted heteroaryl group,
Y³ is a substituted or unsubstituted methylene group,
m is an integer of 1 to 4, n is an integer of 0 to 4, and a sum of m and n is an integer of 1 or more,
R³ and R⁴ are each independently a hydrogen atom, a hydroxyl group, a substituted or unsubstituted alkyl group, a haloalkyl group, the group represented by the formula (2a), a substituted or unsubstituted cycloalkyl group, a substituted or unsubstituted alkoxy group, a formyl group, a hydroxycarbonyl group, an alkylcarbonyl group, an alkoxycarbonyl group, a halogen atom, a substituted or unsubstituted aralkyl group, a substituted or unsubstituted aralkoxy group, a substituted or unsubstituted aryloxy group, a substituted or unsubstituted heteroaryl group, or a group represented by formula (X3),
L¹-R⁴⁰⁰ (X3)
wherein in the formula (X3),
R⁴⁰⁰ is a hydrogen atom, an alkyl group, an aryl group, a polymerizable group, a photochromic group, or a silyl group having an alkyl group, an alkoxy group or an aryl group as a substituent, and
L¹ is a group represented by formula (X2), 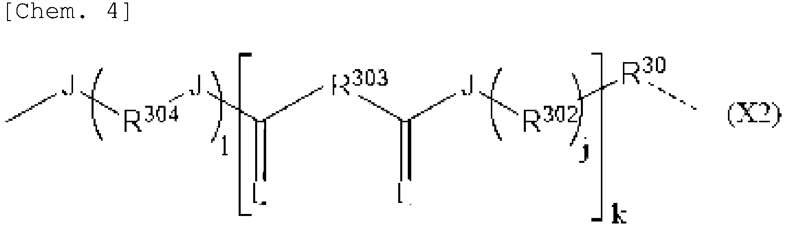
wherein in the formula (X2), R³⁰ is a group represented by formula (X2a), 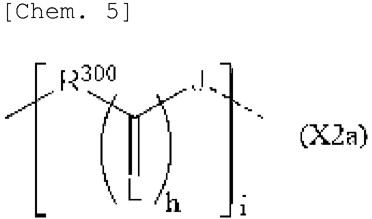
wherein in the formula (X2) and the formula (X2a),
J is a divalent group, and is each independently a direct bond, a substituted or unsubstituted methylene group, an oxygen atom, a sulfur atom, or NR³⁰¹, and R³⁰¹ is a hydrogen atom or an alkyl group,
L is an oxygen atom or a sulfur atom,
R³⁰⁰ is an alkylene group, or a silylene group having an alkyl group or an aryl group as a substituent,
R³⁰², R³⁰³, and R³⁰⁴ are each independently an alkylene group,
h, j, k and 1 are each independently an integer of 0 or 1, and
i is an integer of 1 to 200, and when i is 2 or more, a plurality of R³⁰ are identical or different, and the dashed line represents a bond to R⁴⁰⁰, and
R³ and R⁴ are optionally taken together with M to form a substituted or unsubstituted aliphatic ring having 3 to 20 ring member carbon atoms, a substituted or unsubstituted fused polycyclic ring in which an aromatic hydrocarbon ring or an aromatic heterocyclic ring is fused to the aliphatic ring, a substituted or unsubstituted heterocyclic ring having 3 to 20 ring member atoms, or a substituted or unsubstituted fused polycyclic ring in which an aromatic ring or an aromatic heterocyclic ring is fused to the heterocyclic ring,
R⁵ and R⁶ are each independently a hydrogen atom or a substituent,
R⁷ and R⁸ are each independently a substituent,
b is an integer of 0 to 3, and
c is an integer of 0 to 4.

2. The photochromic compound according to claim 1, represented by formula (3): 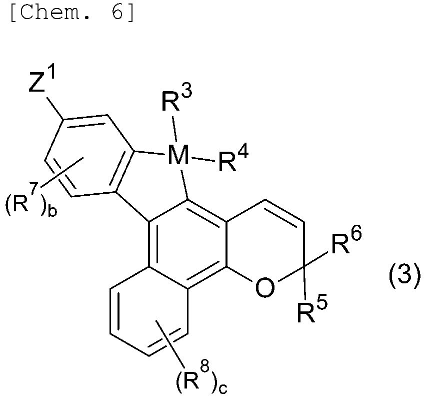
wherein in the formula (3), R³, R⁴, Z¹, M, b, and c are each as defined in the formula (1),
R⁵ and R⁶ are each independently a substituted or unsubstituted aryl group, or a substituted or unsubstituted heteroaryl group,
R⁷ and R⁸ are each independently a hydroxyl group, a substituted or unsubstituted alkyl group, a haloalkyl group, a substituted or unsubstituted cycloalkyl group, a substituted or unsubstituted alkoxy group, a substituted or unsubstituted amino group, a substituted or unsubstituted heterocyclic group, a cyano group, a halogen atom, a substituted or unsubstituted alkylthio group, a substituted or unsubstituted arylthio group, a nitro group, a formyl group, a hydroxycarbonyl group, a substituted or unsubstituted alkylcarbonyl group, a substituted or unsubstituted alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, a substituted or unsubstituted alkoxyalkylthio group, a haloalkylthio group, a substituted or unsubstituted cycloalkylthio group, a substituted or unsubstituted silyl group, a substituted or unsubstituted oxysilyl group, the group represented by the formula (2a), a group represented by formula (X), or the group represented by the formula (X3),
when b is 2 to 3, a plurality of R⁷ are identical to or different from one another,
when c is 2 to 4, a plurality of R⁸ are identical to or different from one another,
when b is 2 to 3, and adjacent R⁷ are present, two adjacent R⁷ are optionally taken together with the carbon atoms bonded to R⁷ to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to the aliphatic ring, the aliphatic heterocyclic ring, the aromatic ring, or the aromatic heterocyclic ring, and
when c is 2 to 4, and adjacent R⁸ are present, two adjacent R⁸ are optionally taken together with the carbon atoms bonded to R⁸ to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to the aliphatic ring, the aliphatic heterocyclic ring, the aromatic ring, or the aromatic heterocyclic ring, 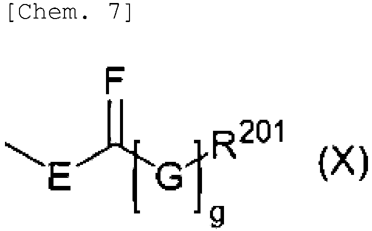
wherein in the formula (X),
E is an oxygen atom or NR¹⁰¹, and R¹⁰¹ is a hydrogen atom or an alkyl group,
F is an oxygen atom or a sulfur atom,
G is an oxygen atom, a sulfur atom, or NR²⁰², and R²⁰² is a hydrogen atom, an alkyl group, a cycloalkyl group, an aryl group, or a heteroaryl group,
g is 0 or 1,
R²⁰¹ is a hydrogen atom, an alkyl group, a cycloalkyl group, an aryl group, or a heteroaryl group, and
when G is an oxygen atom or a sulfur atom, R²⁰¹ is a group other than a hydrogen atom.

3. The photochromic compound according to claim 2, represented by formula (4): 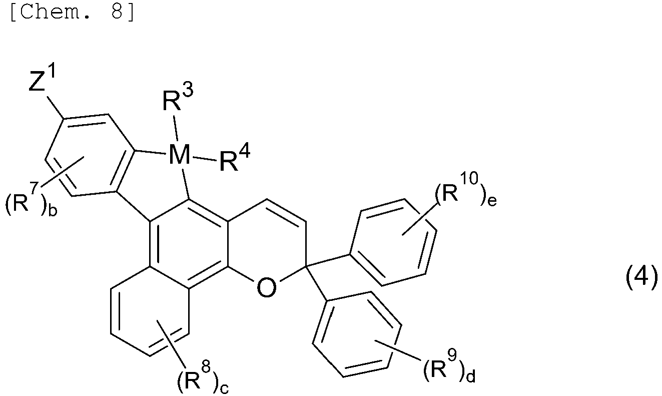 wherein in the formula (4),
R³, R⁴, R⁷, R⁸, Z¹, M, b, and c are each independently as defined in the formula (3),
R⁹ and R¹⁰ are each independently a hydroxyl group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, a substituted or unsubstituted amino group, a substituted or unsubstituted heterocyclic group, a cyano group, a halogen atom, an alkylthio group having 1 to 6 carbon atoms, a substituted or unsubstituted arylthio group, a substituted or unsubstituted aryloxy group, a substituted or unsubstituted aryl group, a substituted or unsubstituted heteroaryl group, a nitro group, a substituted or unsubstituted silyl group, a substituted or unsubstituted oxysilyl group, the group represented by the formula (2a), or the group represented by the formula (X3),
d is an integer of 0 to 5,
when d is 2 to 5, a plurality of R⁹ are groups identical to or different from one another,
when adjacent R⁹ are present, two adjacent R⁹ are optionally taken together with the carbon atoms bonded to R⁹ to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to the aliphatic ring, the aliphatic heterocyclic ring, the aromatic ring, or the aromatic heterocyclic ring,
e is an integer of 0 to 5,
when e is 2 to 5, a plurality of R¹⁰ are groups identical to or different from one another,
when adjacent R¹⁰ are present, two adjacent R¹⁰ are optionally taken together with the carbon atoms bonded to R¹⁰ to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to the aliphatic ring, the aliphatic heterocyclic ring, the aromatic ring, or the aromatic heterocyclic ring.

4. The photochromic compound according to claim 1, wherein M in the formula (1) is C.

5. The photochromic compound according to claim 1, wherein R³ and R⁴ in the formula (1) are a substituted or unsubstituted alkyl group, a haloalkyl group, the group represented by the formula (2a), a substituted or unsubstituted alkoxy group, the group represented by the formula (X3), a substituted or unsubstituted aliphatic ring having 3 to 20 ring member carbon atoms formed together with M, or a substituted or unsubstituted heterocyclic ring having 3 to 20 ring member atoms formed together with M.

6. The photochromic compound according to claim 1, wherein Z¹ in the formula (1) has the group represented by the formula (1a), and R¹ is a substituted or unsubstituted phenyl group.

7. The photochromic compound according to claim 1, wherein Z¹ in the formula (1) has the group represented by the formula (1a), and R² is a substituted or an unsubstituted alkyl group having 1 or more and 10 or less carbon atoms, a substituted or unsubstituted haloalkyl group having 1 or more and 10 or less carbon atoms, or the group represented by the formula (2a).

8. The photochromic compound according to claim 1, wherein Z¹ in the formula (1) has the group represented by the formula (1b), and the ring B is a substituted or unsubstituted phenyl group.

9. The photochromic compound according to claim 1, wherein Z¹ in the formula (1) has the group represented by the formula (1b), and Y² is a substituted or unsubstituted methylene group, O, or S.

10. The photochromic compound according to claim 1, wherein Z¹ in the formula (1) has the group represented by the formula (1b), and the sum of m and n is an integer of 1 to 3.

11. A curable composition, comprising:
the photochromic compound according to claim 1; and
at least one selected from the group consisting of a radically-polymerizable monomer, a cationically-polymerizable monomer, a compound having a polymerization-reactive group, and a (thio)urethane(urea) polymer.

12. A cured product of the curable composition according to claim 11.

13. An optical article comprising the cured product according to claim 12.

14. A lens comprising the photochromic compound according to claim 1.

15. Eyeglasses comprising the lens according to claim 14.

16. A phenol derivative represented by formula (5): 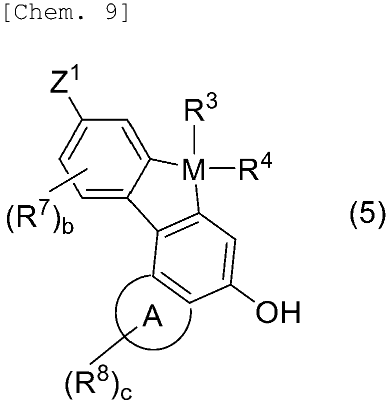 wherein in the formula (5),
M is C, Si, or Ge,
the ring A is a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to the aromatic ring or the aromatic heterocyclic ring,
Z¹ is a group represented by formula (1a), or a group represented by formula (1b), 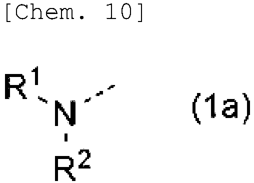
wherein in the formula (1a),
R¹ is a substituted or unsubstituted aryl group, or a substituted or unsubstituted heteroaryl group, and
R² is a hydrogen atom, a substituted or unsubstituted alkyl group, a substituted or unsubstituted haloalkyl group, a substituted or unsubstituted cycloalkyl group, a substituted or unsubstituted alkoxy group, a substituted or unsubstituted aryl group, a substituted or unsubstituted heteroaryl group, or a group represented by formula (2a),
-Q¹-(X¹Q²) a-X²Q³ (2a)
wherein in the formula (2a),
Q¹ is an alkylene group or a haloalkylene group,
Q² is an alkylene group or a haloalkylene group,
Q³ is an alkyl group or a haloalkyl group,
X¹ and X² are each independently O, S, NR⁷⁰⁰R⁷⁰¹, PR⁷⁰²R⁷⁰³, or P(=O) R⁷⁰⁴,
R⁷⁰⁰, R⁷⁰¹, R⁷⁰², R⁷⁰³, and R⁷⁰⁴ are each independently a hydrogen atom, a substituted or unsubstituted alkyl group, a substituted or unsubstituted cycloalkyl group, a substituted or unsubstituted aryl group, or a substituted or unsubstituted heteroaryl group, and
a is an integer of 0 or 1 or more and 3 or less, 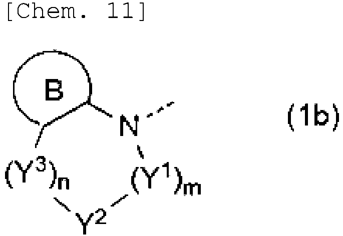
wherein in the formula (1b),
the ring B is a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to the aromatic ring or the aromatic heterocyclic ring,
Y¹ is a substituted or unsubstituted methylene group,
Y² is a substituted or unsubstituted methylene group, O, S, SO₂, NR⁶⁰⁰, R⁶⁰¹C=CR⁶⁰², or CC,
R⁶⁰⁰, R⁶⁰¹, and R⁶⁰² are each independently a hydrogen atom, a substituted or unsubstituted alkyl group, a substituted or unsubstituted alkoxy group, a substituted or unsubstituted haloalkyl group, a substituted or unsubstituted haloalkoxy group, a substituted or unsubstituted cycloalkyl group, a substituted or unsubstituted aryl group, or a substituted or unsubstituted heteroaryl group,
Y³ is a substituted or unsubstituted methylene group, and m is an integer of 1 to 4, n is an integer of 0 to 4, and a sum of m and n is an integer of 1 or more, and
R³ and R⁴ are each independently a hydrogen atom, a hydroxyl group, a substituted or unsubstituted alkyl group, a haloalkyl group, the group represented by the formula (2a), a substituted or unsubstituted cycloalkyl group, a substituted or unsubstituted alkoxy group, a formyl group, a hydroxycarbonyl group, an alkylcarbonyl group, an alkoxycarbonyl group, a halogen atom, a substituted or unsubstituted aralkyl group, a substituted or unsubstituted aralkoxy group, a substituted or unsubstituted aryloxy group, a substituted or unsubstituted heteroaryl group, or a group represented by formula (X3),
L¹-R⁴⁰⁰ (X3)
wherein in the formula (X3),
R⁴⁰⁰ is a hydrogen atom, an alkyl group, an aryl group, a polymerizable group, a photochromic group, or a silyl group having an alkyl group, an alkoxy group or an aryl group as a substituent, and
L¹ is a group represented by formula (X2), 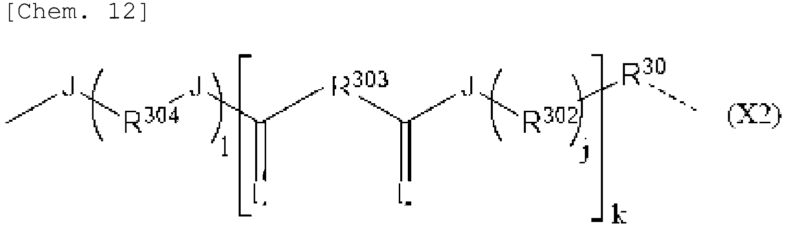
wherein in the formula (X2), R³⁰ is a group represented by formula (X2a), 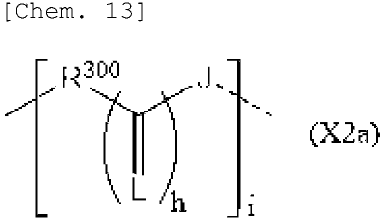
wherein in the formula (X2) and the formula (X2a),
J is a divalent group, and is each independently a direct bond, a substituted or unsubstituted methylene group, an oxygen atom, a sulfur atom, or NR³⁰¹, and R³⁰¹ is a hydrogen atom or an alkyl group,
L is an oxygen atom or a sulfur atom,
R³⁰⁰ is an alkylene group, or a silylene group having an alkyl group or an aryl group as a substituent,
R³⁰², R³⁰³, and R³⁰⁴ are each independently an alkylene group,
h, j, k and l are each independently an integer of 0 or 1, and
i is an integer of 1 to 200, and when i is 2 or more, a plurality of R³⁰ are identical or different, and the dashed line represents a bond to R⁴⁰⁰, and
R³ and R⁴ are optionally taken together with M to form a substituted or unsubstituted aliphatic ring having 3 to 20 ring member carbon atoms, a substituted or unsubstituted fused polycyclic ring in which an aromatic hydrocarbon ring or an aromatic heterocyclic ring is fused to the aliphatic ring, a substituted or unsubstituted heterocyclic ring having 3 to 20 ring member atoms, or a substituted or unsubstituted fused polycyclic ring in which an aromatic ring or an aromatic heterocyclic ring is fused to the heterocyclic ring,
R⁷ and R⁸ are each independently a substituent,
b is an integer of 0 to 3, and
c is an integer of 0 to 4.

17. The phenol derivative according to claim 16, represented by formula (7): 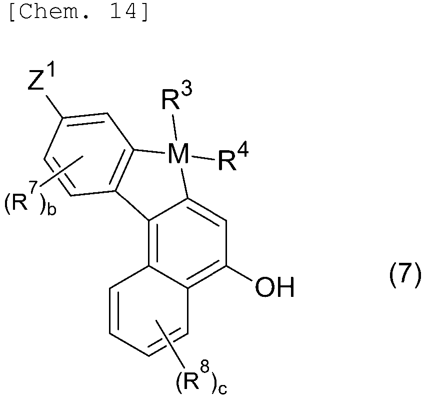
wherein in the formula (7), Z¹, R³, R⁴, M, b, and c are as defined in the formula (6),
R⁷ and R⁸ are each independently a hydroxyl group, a substituted or unsubstituted alkyl group, a haloalkyl group, a substituted or unsubstituted cycloalkyl group, a substituted or unsubstituted alkoxy group, a substituted or unsubstituted amino group, a substituted or unsubstituted heterocyclic group, a cyano group, a halogen atom, a substituted or unsubstituted alkylthio group, a substituted or unsubstituted arylthio group, a nitro group, a formyl group, a hydroxycarbonyl group, a substituted or unsubstituted alkylcarbonyl group, a substituted or unsubstituted alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, a substituted or unsubstituted alkoxyalkylthio group, a haloalkylthio group, a substituted or unsubstituted cycloalkylthio group, a substituted or unsubstituted silyl group, a substituted or unsubstituted oxysilyl group, the group represented by the formula (2a), a group represented by formula (X), or the group represented by the formula (X3),
when b is 2 to 3, a plurality of R⁷ are identical to or different from one another,
when c is 2 to 4, a plurality of R⁸ are identical to or different from one another,
when b is 2 to 3, and adjacent R⁷ are present, two adjacent R⁷ are optionally taken together with the carbon atoms bonded to R⁷ to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to the aliphatic ring, the aliphatic heterocyclic ring, the aromatic ring, or the aromatic heterocyclic ring, and
when c is 2 to 4, and adjacent R⁸ are present, two adjacent R⁸ are optionally taken together with the carbon atoms bonded to R⁸ to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to the aliphatic ring, the aliphatic heterocyclic ring, the aromatic ring, or the aromatic heterocyclic ring, 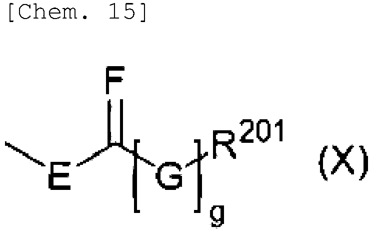
wherein in the formula (X),
E is an oxygen atom or NR¹⁰¹, and R¹⁰¹ is a hydrogen atom or an alkyl group,
F is an oxygen atom or a sulfur atom,
G is an oxygen atom, a sulfur atom, or NR²⁰², and R²⁰² is a hydrogen atom, an alkyl group, a cycloalkyl group, an aryl group, or a heteroaryl group,
g is 0 or 1,
R²⁰¹ is a hydrogen atom, an alkyl group, a cycloalkyl group, an aryl group, or a heteroaryl group, and
when G is an oxygen atom or a sulfur atom, R²⁰¹ is a group other than a hydrogen atom.
